(19) Europäisches Patentamt
European Patent Office
Office européen des brevets

(11) Veröffentlichungsnummer: **0 168 683**
**B1**

(12) # EUROPÄISCHE PATENTSCHRIFT

(45) Veröffentlichungstag der Patentschrift: **28.11.90**

(21) Anmeldenummer: **85107789.1**

(22) Anmeldetag: **24.06.85**

(51) Int. Cl.$^5$: **C 07 C 69/75,** C 07 C 69/773, C 07 C 43/215, C 07 D 319/04, C 07 C 13/19, C 09 K 19/30, C 09 K 19/34

(54) **Flüssigkristalle mit Alkenyl- oder Alkenyloxygruppen.**

(30) Priorität: **16.07.84 CH 3457/84**
**08.05.85 CH 1950/85**

(43) Veröffentlichungstag der Anmeldung:
**22.01.86 Patentblatt 86/04**

(45) Bekanntmachung des Hinweises auf die Patenterteilung:
**28.11.90 Patentblatt 90/48**

(84) Benannte Vertragsstaaten:
**CH DE FR GB IT LI SE**

(56) Entgegenhaltungen:
**EP-A-1 022 389**

**PATENT ABSTRACTS OF JAPAN, Band 9, Nr. 66 (C-271)1789r, 26. März 1985; & JP - A - 59 199 649 (TEIICHI TANIGAKI) 12.11.1984**

**PATENT ABSTRACTS OF JAPAN, Band 6, Nr. 143 (C-117)1021r, 3. August 1982; & JP - A - 57 67538 (ASAHI GLASS) 24.04.1982**
**Mol. Cryst.Liq. Cryst, 1987, Vol. 148, pp123-143**
**Mol. Cryst. Liq. Cryst.1986, Vol. 139, pp 1-25**
**Mol.Cryst.Liq.Cryst. 1985,Vol.131,pp. 109-123**

(73) Patentinhaber: **F. HOFFMANN-LA ROCHE AG**
**Postfach 3255**
**CH-4002 Basel (CH)**

(72) Erfinder: **Petrzilka, Martin, Dr.**
**Schwarzackerstrasse 54**
**CH-4303 Kaiseraugst (CH)**
Erfinder: **Schadt, Martin, Dr.**
**Liestalerstrasse 77**
**CH-4411 Seltisberg (CH)**

(74) Vertreter: **Zimmermann, Hans, Dr. et al**
**Grenzacherstrasse 124 Postfach 3255**
**CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 168 683 B1

**Beschreibung**

Die vorliegende Erfindung betrifft neue Verbindungen mit Alkenyl- oder Alkenyloxygruppen, flüssigkristalline Gemische, die solche Verbindungen enthalten, sowie die Verwendung für elektro-optische Zwecke.

Flüssige Kristalle haben in den letzten Jahren vor allem als Dielektrika in Anzeigevorrichtungen stark an Bedeutung gewonnen, da die optischen Eigenschaften solcher Substanzen durch eine angelegte Spannung beeinflusst werden können. Elektro-optische Vorrichtungen auf der Basis von Flüssigkristallen sind dem Fachmann gut bekannt und können auf verschiedenen Effekten beruhen, wie beispielsweise der dynamischen Streuung, der Deformation aufgerichteter Phasen (DAP-Zelle), dem Schadt-Helfrich-Effekt (Drehzelle), dem Gast/Wirt-Effekt (Guest/Host-Zelle) oder einem cholesterisch-nematischen Phasenübergang (Phase-Change-Zelle).

Die verwendeten Flüssigkristalle müssen eine gute Stabilität gegenüber Wärme, Feuchtigkeit, Luft, elektromagnetischer Strahlung, elektrischen Feldern und dergleichen besitzen. Ferner sollten sie farblos sein, kurze Ansprechzeiten und niedere Viskosität aufweisen, einen guten Kontrast ergeben und im ganzen Temperaturbereich, in welchem die Flüssigkristallzelle betrieben werden soll, eine nematische bzw. cholesterische Mesophase besitzen. Da Flüssigkristalle üblicherweise als Mischungen zur Anwendung gelangen, ist es zudem wichtig, dass die Komponenten untereinander gut mischbar sind und dabei eine nematische bzw. cholesterische Mesophase ausbilden. Weitere Eigenschaften, wie beispielsweise die elektrische Leitfähigkeit, die Schwellenspannung, die Multiplexierbarkeit und die dielektrische Anisotropie, müssen je nach verwendetem Zellentyp unterschiedliche Bedingungen erfüllen.

Gegenstand der vorliegenden Erfindung sind die Verbindungen der allgemeinen Formel

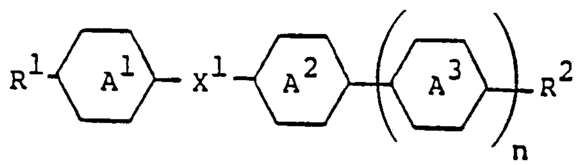

I

worin n für die Zahl 0 oder 1 steht; die Ringe $A^1$, $A^2$ und $A^3$ 1,4-Phenylen, 2-Fluor-1,4-phenylen oder trans-1,4-Cyclohexylen darstellen oder einer dieser Ringe auch einen 2,5-di- substituierten Pyrimidinring oder einen trans-2,5-disubstituierten m-Dioxanring darstellt; $X^1$ eine einfache Kovalenzbindung, —COO—, —OOC—, $—CH_2CH_2—$, p-$C_6H_4$—, $—CH_2CH_2$-p-$C_6H_4$—, $—CH_2CH_2$-p-$C_6H_4—CH_2CH_2—$ oder, sofern die Ringe $A^1$ und $A^2$ 1,4-Phenylen darstellen, auch eine Azoxygruppe —N=N(O)— oder —N(O)=N— bezeichnet; $R^2$ 1E-Alkenyl, 2Z-Alkenyl, 3E-Alkenyl, 4-Alkenyl oder Alkenyloxy darstellt, mit der Massgabe, dass das Sauerstoffatom in Alkenyloxy mit einem gesättigten Kohlenstoffatom verknüpft ist; und $R^1$ 1E-Alkenyl, 2Z-Alkenyl, 3E-Alkenyl, 4-Alkenyl oder, sofern $R^2$ Alkenyloxy darstellt, auch Alkyl bedeutet; mit der Massgabe, dass an einen Benzol- oder Pyrimidinring gebundene Reste $R^1$ und $R^2$ 3E-Alkenyl, 4-Alkenyl oder im Falle von $R^2$ auch Alkenyloxy oder im Falle von $R^1$ auch Alkyl bedeutet.

Gewisse unter Formel I fallende Verbindungen, nämlich solche, worin $R^1$ $C_1$—$C_{10}$-Alkyl, die Ringe $A^1$ und $A^2$ jeweils 1,4-Phenylen oder trans-1,4-Cyclohexylen, $X^1$ eine einfache Kovalenzbindung, n die Zahl 0 und $R^2$ eine Alkenyloxygruppe bedeuten, sind in EP—A—0136501 (Benannte Vertragsstaaten: CH, DE, FR, GB, LI) als Zwischenprodukte für Trialkanoyloxysilane beschrieben. Die vorliegende Erfindung betrifft daher bezüglich des Verbindungsaspektes in diesen Staaten nur Verbindungen der Formel I, worin nicht zugleich $R^1$ Alkyl, die Ringe $A^1$ und $A^2$ jeweils 1,4-Phenylen oder trans-1,4-Cyclohexylen, $X^1$ eine einfache Kovalenzbindung, n die Zahl 0 und $R^2$ Alkenyloxy bedeuten.

Die erfindungsgemässen Verbindungen besitzen die oben erwähnten erforderlichen Eigenschaften und zeigen zudem bezüglich verschiedener Parameter verbesserte Werte gegenüber bekannten Flüssigkristallkomponenten. Beispielsweise besitzen die Verbindungen mit Seitenketten wie 1E-Alkenyl, 3E-Alkenyl, (2E-Alkenyl)oxy, (4-Alkenyl)oxy etc. im allgemeinen verbesserte Mesophasebereiche und Klärpunkte und meist auch kürzere Ansprechzeiten im Vergleich zu den entsprechenden Verbindungen mit gesättigter Seitenkette. Anderseits ergeben die Verbindungen mit Seitenketten wie 2Z-Alkenyl, 4-Alkenyl, (3-Alkenyl)oxy, (5-Alkenyl)oxy etc. im allgemeinen eine niedere Viskosität (insbesondere eine verbesserte Rotationsviskosität $\gamma_1$), neidere Schwellenspannungen, ein günstiges Verhältnis der elastischen Konstanten $k_{33}$ (bend) und $k_{11}$ (splay) und somit steile Transmissionskurven und eine gute Multiplexierbarkeit.

Die Verbindungen der Formel I besitzen einen relativ kleinen Absolutbrag der dielektrischen Anisotropie ($\Delta\varepsilon = \varepsilon_\parallel - \varepsilon_\perp$, wobei $\varepsilon_\parallel$ die Dielektrizitätskonstante entlang der Moleküllängsachse und $\varepsilon_\perp$ die Dielektrizitätskonstante senkrecht dazu bedeuten) und eine geringe Leitfähigkeit. Sie sind daher für beliebige Mischungen, d.h. für Mischungen mit positiver, negativer oder kleiner absoluter dielektrischer Anisotropie, und für alle üblichen Flüssigkristallzellen, insbesondere für Drehzellen und Guest/Host-Zellen geeignet.

Im Rahmen der vorliegenden Erfindung umfasst der Ausdruck "1E-Alkenyl" Reste wie Vinyl, 1E-Propenyl, 1E-Butenyl, 1E-Pentenyl, 1E-Hexenyl, 1E-Heptenyl, 1E-Octenyl, 1E-Nonenyl und 1E-Decenyl. Der

Ausdruck "2Z-Alkenyl" umfasst Reste wie Allyl, 2Z-Butenyl, 2Z-Pentenyl, 2Z-Hexenyl, 2Z-Heptenyl, 2Z-Octenyl, 2Z-Nonenyl und 2Z-Decenyl. Der Ausdruck "3E-Alkenyl" umfasst Reste wie 3-Butenyl, 3E-Pentenyl, 3E-Hexenyl, 3E-Heptenyl, 3E-Octenyl, 3E-Nonenyl und 3E-Decenyl. Der Ausdruck "4-Alkenyl" umfasst Reste wie 4-Pentenyl und die E- und/oder Z-Form von 3-Hexenyl, 4-Heptenyl, 4-Octenyl, 4-Nonenyl und 4-Decenyl.

Der Ausdruck "Alkenyloxy" in $R^2$ bezeichnet Alkenyloxygruppen, in denen der Sauerstoff mit einem gesättigten Kohlenstoffatom direkt verknüpft ist (d.h. Gruppen welche ein oder mehrere Kohlenstoffatome zwischen der Doppelbindung und dem Sauerstoffatom aufweisen), wie (2E-Alkenyl)oxy, (3-Alkenyl)oxy, (4-Alkenyl)oxy, (5-Alkenyl)oxy und dergleichen. Der Ausdruck "(2E-Alkenyl)oxy" umfasst hierbei Reste wie Allyloxy, (2E-Butenyl)oxy, (2E-Pentenyl)oxy, (2E-Hexenyl)oxy, (2E-Heptenyl)oxy, (2E-Octenyl)oxy, (2E-Nonenyl)oxy und (2E-Decenyl)oxy. Der Ausdruck "(3-Alkenyl)oxy" umfasst Reste wie (3-Butenyl)oxy und die E- und/oder Z-Form von (3-Pentenyl)oxy, (3-Hexenyl)oxy, (3-Heptenyl)oxy, (3-Octenyl)oxy, (3-Nonenyl)oxy und (3-Decenyl)oxy. Der Ausdruck "(4-Alkenyl)oxy" umfasst Reste wie (4-Pentenyl)oxy und die E- und/oder Z-form von (4-Hexenyl)oxy, (4-Heptenyl)oxy, (4-Octenyl)oxy, (4-Nonenyl)oxy und (4-Decenyl)oxy. Der Ausdruck "(5-Alkenyl)oxy" umfasst Reste wie (5-Hexenyl)oxy und die E- und/oder Z-Form von (5-Heptenyl)oxy, (5-Octenyl)oxy, (5-Nonenyl)oxy und (5-Decenyl)oxy.

Der Ausdruck "Alkyl" bedeutet im Rahmen der vorliegenden Erfindung geradkettiges oder verzweigtes Alkyl, wie Methyl, Aethyl, Propyl, Isopropyl, Butyl, 2-Methylbutyl, Pentyl, Hexyl, Heptyl, Octyl, Nonyl und Decyl. Die obige Beziechnung $p\text{-}C_6H_4$— steht für 1,4-Phenylen. Der Ausdruck "Halogen" beziechnet im Rahmen der vorliegenden Erfindung Chlor, Brom oder Jod. Der Ausdruck "Alkalimetall" umfasst Lithium, Natrium und Kalium.

Die obige Formel I umfasst insbesondere die folgenden Teilformeln

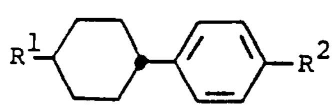

Ia

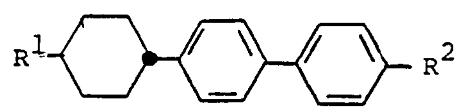

Ib

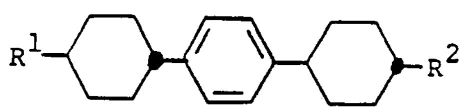

Ic

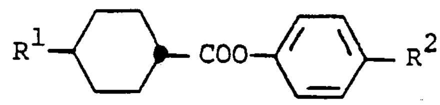

Id

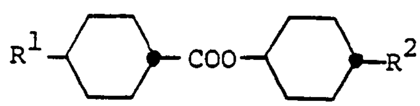

Ie

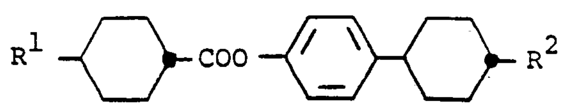

If

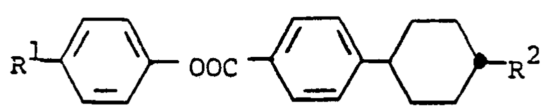

Ig

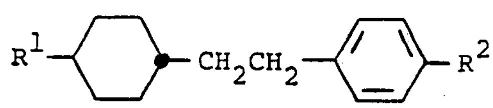

Ih

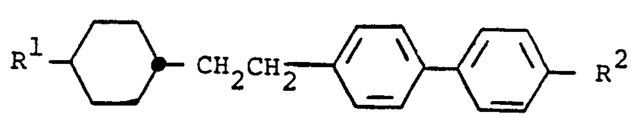

Ii

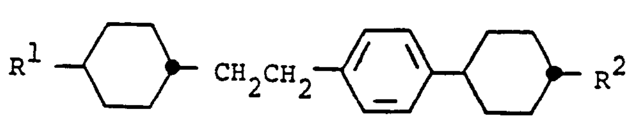

Ij

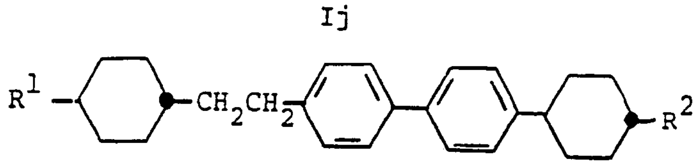

Ik

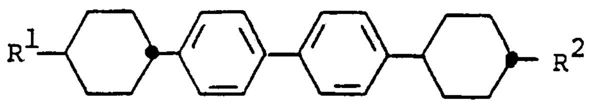

Il

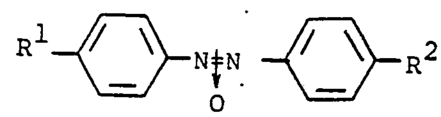

Im

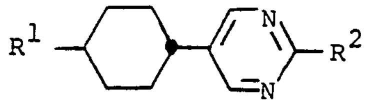

In

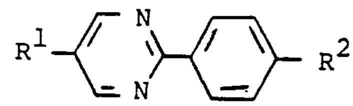

Io

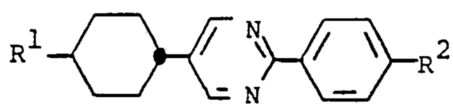

Ip

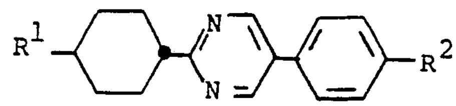

Iq

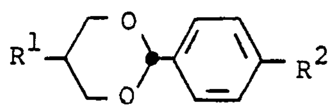

Ir

worin $R^1$ und $R^2$ die obigen Bedeutungen haben.

$R^1$ und $R^2$ stehen vorzugsweise für geradkettige Reste mit höchstens 12 Kohlenstoffatomen, d.h. für geradkettiges 1E-Alkenyl mit 2 bis 12 Kohlenstoffatomen, geradkettiges 2Z-Alkenyl mit 3 bis 12 Kohlenstoffatomen, geradkettiges 3E-Alkenyl mit 4 bis 12 Kohlenstoffatomen, geradkettiges 4-Alkenyl mit 5 bis 12 Kohlenstoffatomen, geradkettiges Alkyl mit 1 bis 12 Kohlenstoffatomen bzw. geradkettiges Alkenyloxy mit höchstens 12 Kohlenstoffatomen, wie geradkettiges (2E-Alkenyl)oxy mit 3 bis 12 Kohlenstoffatomen, geradkettiges (3-Alkenyl)oxy mit 4 bis 12 Kohlenstoffatomen, geradkettiges (4-Alkenyl)oxy mit 5 bis 12 Kohlenstoffatomen, geradkettiges (5-Alkenyl)oxy mit 6 bis 12 Kohlenstoffatomen und dergleichen. Besonders bevorzugt sind Reste $R^1$ und $R^2$ mit höchstens 7 Kohlenstoffatomen.

Bevorzugte Alkenyloxyreste $R^2$ sind (5-Alkenyl)oxy, (4-Alkenyl)oxy und insbesondere (3-Alkenyl)oxy und (2E-Alkenyl)oxy. Bevorzugte Alkenylreste $R^1$ bzw. $R^2$ sind 1E-Alkenyl, 3E-Alkenyl und 4-Alkenyl. Falls $R^2$ für Alkenyloxy steht, kann $R^1$ vorzugsweise auch Alkyl bedeuten. Besonders bevorzugte Verbindungen der Formel I sind diejenigen, worin $R^2$ (2E-Alkenyl)oxy bedeutet oder mindestens einer der Reste $R^1$ und $R^2$ 1E-Alkenyl oder 3E-Alkenyl bedeutet. Vorzugsweise steht 4-Alkenyl für 4Z-Alkenyl, (3-Alkenyl)oxy für (3Z-Alkenyl)oxy, (4-Alkenyl)oxy für (4E-Alkenyl)oxy und (5-Alkenyl)oxy für (5Z-Alkenyl)oxy.

Reste $R^1$ und $R^2$, welche an einen aromatischen Ring, d.h. an einen Benzol- oder Pyrimidinring gebunden sind, bedeuten 3E-Alkenyl, 4-Alkenyl oder im Falle von $R^2$ auch Alkenyloxy oder im Falle von $R^1$ auch Alkyl.

Laterale Fluorsubstituenten ergeben meist verbesserte nematische Tendenzen. Im allgemeinen sind jedoch Verbindungen ohne lateralen Fluorsubstituenten (d.h. ohne 2-Fluor-1,4-phenylengruppe) bevorzugt. Vorzugsweise bezeichnet $X^1$ eine einfache Kovalenzbindung, —COO—, —OOC—, $—CH_2CH_2—$ oder p-$C_6H_4$—. Vorzugsweise bezeichnet n die Zahl 0.

Ring $A^1$ steht vorzugsweise für 1,4-Phenylen, wenn $X^1$ —OOC, —N=N(O)— oder —N(O)=N— bezeichnet, und vorzugsweise für trans-1,4-Cyclohexylen in den übrigen Fällen. Bezeichnet $X^1$ eine einfache Kovalenzbindung, kann Ring $A^1$ vorzugsweise auch für einen 2,5-disubstituierten Pyrimidinring oder einen trans-2,5-disubstituierten m-Dioxanring stehen; Ring $A^2$ bezeichnet in diesem Fall trans-1,4-Cyclohexylen oder vorzugsweise 1,4-Phenylen. Ring $A^3$ steht vorzugsweise für 1,4-Phenylen oder trans-1,4-Cyclohexylen. Ring $A^2$ steht vorzugsweise für 1,4-Phenylen, einen 2,5-disubstituierten Pyrimidring oder, falls n die Zahl 0 bedeutet, auch für trans-1,4-Cyclohexylen.

Eine besonders bevorzugte Gruppe von Verbindungen der Formel I sind diejenigen, worin n für die Zahl 0 steht, Ring $A^1$ trans-1,4-Cyclohexylen darstellt, Ring $A^2$ 1,4-Phenylen darstellt und $X^1$ eine einfache Kovalenzbindung, —COO—, $—CH_2CH_2—$ oder p-$C_6H_4$— bezeichnet.

Beispiel bevorzugter erfindungsgemässer Verbindungen sind die Verbindungen der Formeln Ia, Ib, Ic, Id, Ie, If, Ig, Ih, Ii, Ij, Ik, Il, Im, In, Io, Ip, Iq und Ir, worin $R^1$ und $R^2$ im Rahmen der Formeln I gemäß deu Ausspruchsfassungen für die betreffenden Vertragsstaaten jeweils die folgenden Bedeutungen haben:

| $R^1$ | $R^2$ |
|---|---|
| Propyl | Allyloxy |
| Propyl | (2E-Butenyl)oxy |
| Propyl | (3-Butenyl)oxy |
| Pentyl | Allyloxy |
| Pentyl | (2E-Butenyl)oxy |
| Pentyl | (3-Butenyl)oxy |
| 1E-Propenyl | 1E-Propenyl |
| 1E-Propenyl | 1E-Butenyl |
| 1E-Propenyl | 1E-Pentenyl |
| 1E-Propenyl | 3-Butenyl |
| 1E-Propenyl | 3E-Pentenyl |
| 1E-Propenyl | 4-Pentenyl |
| 1E-Propenyl | 4Z-Hexenyl |
| 1E-Propenyl | Allyloxy |
| 1E-Propenyl | (2E-Butenyl)oxy |
| 1E-Propenyl | (3-Butenyl)oxy |
| 1E-Butenyl | 1E-Propenyl |
| 1E-Butenyl | 1E-Butenyl |
| 1E-Butenyl | 1E-Pentenyl |
| 1E-Butenyl | 3-Butenyl |
| 1E-Butenyl | 3E-Pentenyl |
| 1E-Butenyl | 4-Pentenyl |
| 1E-Butenyl | 4Z-Hexenyl |
| 1E-Butenyl | Allyloxy |
| 1E-Butenyl | (2E-Butenyl)oxy |
| 1E-Butenyl | (3-Butenyl)oxy |
| 1E-Pentenyl | 1E-Propenyl |
| 1E-Pentenyl | 1E-Butenyl |
| 1E-Pentenyl | 1E-Pentenyl |
| 1E-Pentenyl | 3-Butenyl |
| 1E-Pentenyl | 3E-Pentenyl |
| 1E-Pentenyl | 4-Pentenyl |
| 1E-Pentenyl | Allyloxy |
| 1E-Pentenyl | (2E-Butenyl)oxy |
| 1E-Pentenyl | (3-Butenyl)oxy |
| 3-Butenyl | 1E-Propenyl |
| 3-Butenyl | 1E-Butenyl |
| 3-Butenyl | 1E-Pentenyl |
| 3-Butenyl | 3-Butenyl |
| 3-Butenyl | 3E-Pentenyl |
| 3-Butenyl | 3E-Hexenyl |
| 3-Butenyl | 4-Pentenyl |
| 3-Butenyl | Allyloxy |
| 3-Butenyl | (2E-Butenyl)oxy |
| 3-Butenyl | (3-Butenyl)oxy |
| 3-Butenyl | (3Z-Pentenyl)oxy |
| 3E-Pentenyl | 1E-Propenyl |
| 3E-Pentenyl | 1E-Butenyl |
| 3E-Pentenyl | 1E-Pentenyl |
| 3E-Pentenyl | 3-Butenyl |
| 3E-Pentenyl | 3E-Pentenyl |
| 3E-Pentenyl | 4-Pentenyl |
| 3E-Pentenyl | Allyloxy |
| 3E-Pentenyl | (2E-Butenyl)oxy |
| 3E-Pentenyl | (3-Butenyl)oxy |
| 3E-Hexenyl | 1E-Propenyl |
| 3E-Hexenyl | 1E-Butenyl |
| 3E-Hexenyl | 3-Butenyl |
| 3E-Hexenyl | 4-Pentenyl |
| 3E-Hexenyl | Allyloxy |
| 3E-Hexenyl | (2E-Butenyl)oxy |
| 3E-Hexenyl | (3-Butenyl)oxy |
| 3E-Heptenyl | 1E-Propenyl |

| $R^1$ | $R^2$ |
|---|---|
| 3E-Heptenyl | 1E-Butenyl |
| 3E-Heptenyl | 3-Butenyl |
| 3E-Heptenyl | 4-Pentenyl |
| 3E-Heptenyl | Allyloxy |
| 3E-Heptenyl | (2E-Butenyl)oxy |
| 3E-Heptenyl | (3-Butenyl)oxy |
| 4-Pentenyl | 1E-Propenyl |
| 4-Pentenyl | 1E-Butenyl |
| 4-Pentenyl | 1E-Pentenyl |
| 4-Pentenyl | 3-Butenyl |
| 4-Pentenyl | 3E-Pentenyl |
| 4-Pentenyl | Allyloxy |
| 4-Pentenyl | (2E-Butenyl)oxy |
| 4Z-Hexenyl | 1E-Propenyl |
| 4Z-Hexenyl | 1E-Butenyl |
| 4Z-Hexenyl | 3-Butenyl |
| 4Z-Hexenyl | Allyloxy |

sowie die weiteren in den Synthesebeispielen genannten Verbindungen der Formel I.

Die Verbindungen der Formel I können dadurch hergestellt werden, dass man

a) zur Herstellung der Verbindungen der Formel I, worin $X^1$ —COO— oder —OOC— bezeichnet, eine Verbindung der allgemeinen Formel

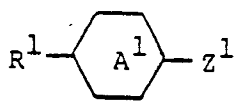
II

oder ein reaktionsfähiges Derivat hiervon mit einer Verbindung der allgemeinen Formel

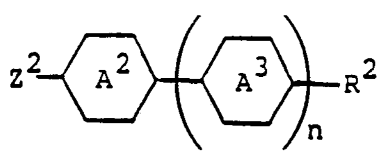
III

worin eine der Gruppen $Z^1$ und $Z^2$ —COOH und die andere —OH darstellt; und $R^1$, $R^2$, n und die Ringe $A^1$, $A^2$ und $A^3$ die obige Bedeutung haben, oder ein Reaktionsfähiges Derivat hiervon verestert, oder

b) zur Herstellung der Verbindungen der Formel I, worin einer der Ringe $A^1$, $A^2$ und $A^3$ einen trans-2,5-disubstituierten m-Dioxanring darstellt und $X^1$ eine einfache Kovalenzbindung, $—CH_2CH_2—$, p-$C_6H_4$—, —$CH_2CH_2$-p-$C_6H_4$— oder —$CH_2CH_2$-p-$C_6H_4$—$CH_2CH_2$— bezeichnet, eine Verbindung der allgemeinen Formel

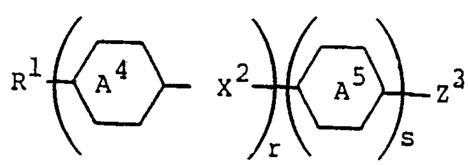
IV

mit einer Verbindung der allgemeinen Formel

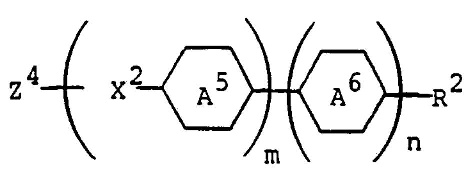
V

worin eine der Gruppen $Z^3$ und $Z^4$ —$CH(CH_2OH)_2$ und die andere —CHO darstellt; $X^2$ eine einfache Kovalenzbindung, $—CH_2CH_2—$, p-$C_6H_4$—, —$CH_2CH_2$-p-$C_6H_4$— oder —$CH_2CH_2$-p-$C_6H_4$—$CH_2CH_2$— bezeichnet; die Ringe $A^4$, $A^5$ und $A^6$ 1,4-Phenylen, 2-Fluor-1,4-phenylen oder trans-1,4-Cyclohexylen

darstellen; m, n, r und s die Zahlen 0 oder 1 bedeuten und die Summe (m + n + r + s) dieser Indices 1 oder 2 beträgt, wobei s nur dann die Zahl 1 bedeuten kann, wenn r für die Zahl 1 steht, und m nur dann die Zahl 1 bedeuten kann, wenn r für die Zahl 0 steht; und $R^1$ und $R^2$ die obigen Bedeutungen haben, umsetzt, oder

c) zur Herstellung der Verbindungen der Formel I, worin einer der Ringe $A^1$, $A^2$ und $A^3$ einen 2,5-disubstituierten Pyrimidinring darstellt und $X^1$ eine einfache Kovalenzbindung, $—CH_2CH_2—$, $p\text{-}C_6H_4—$, $—CH_2CH_2\text{-}p\text{-}C_6H_4—$ oder $—CH_2CH_2\text{-}p\text{-}C_6H_4—CH_2CH_2—$ bezeichnet, eine Verbindung der Formel IV mit einer Verbindung der Formel V, worin eine der Gruppen

$Z^3$ und $Z^4$ $H_2N—\overset{|}{C}=NH \cdot HCl$ und die andere $OHC—\overset{|}{C}=CHOR^3$ darstellt,

$R^3$ Alkyl bezeichnet und $X^2$, $R^1$, $R^2$, m, n, r, s und die Ringe $A^4$, $A^5$ und $A^6$ die obigen Bedeutungen haben, in Gegenwart einer Base umsetzt, oder

d) zur Herstellung der Verbindungen der Formel I, worin $R^1$ eine Alkenylgruppe und $R^2$ eine Alkenylgruppe oder Alkenyloxygruppe bedeuten und $X^1$ eine einfache Kovalenzbindung, $—CH_2CH_2—$, $p\text{-}C_6H_4—$, $—CH_2CH_2\text{-}p\text{-}C_6H_4—$ oder $—CH_2CH_2\text{-}p\text{-}C_6H_4—CH_2CH_2—$ bezeichnet, eine Verbindung der allgemeinen Formel

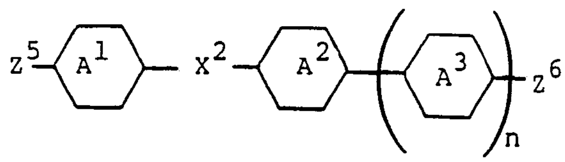

VI

worin $Z^5$ $OHC(CH_2)_p$ und $Z^6$ 1E-Alkenyl, 2Z-Alkenyl, 3E-Alkenyl, 4-Alkenyl oder Alkenyloxy darstellen, oder $Z^5$ 1E-Alkenyl, 2Z-Alkenyl, 3E-Alkenyl oder 4-Alkenyl und $Z^6$ $(CH_2)_qCHO$ darstellen, oder $Z^5$ $OHC(CH_2)_p$ und $Z^6$ $(CH_2)_qCHO$ darstellen; p und q für die Zahlen 0, 1, 2 oder 3 stehen; $X^2$ eine einfache Kovalenzbindung, $—CH_2CH_2—$, $p\text{-}C_6H_4—$, $—CH_2CH_2\text{-}p\text{-}C_6H_4—$ oder $—CH_2CH_2\text{-}p\text{-}C_6H_4—CH_2CH_2—$ bezeichnet; und n und die Ringe $A^1$, $A^2$ und $A^3$ die obigen Bedeutungen haben, in Gegenwart einer Base mit Alkyltriphenylphosphoniumhalogenid umsetzt oder

e) zur Herstellung der Verbindungen der Formel I, worin $R^2$ Alkenyloxy darstellt, eine Verbindung der allgemeinen Formel

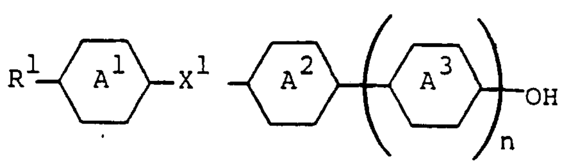

VII

worin $R^1$ 1E-Alkenyl, 2Z-Alkenyl, 3E-Alkenyl, 4E-Alkenyl oder Alkyl bedeutet und $X^1$, n und die Ringe $A^1$, $A^2$ und $A^3$ die obigen Bedeutungen haben, mit einer Verbindung der allgemeinen Formel

$$Z^7{-}(CH_2)_k{-}CH{=}CH{-}R \qquad \text{VIII}$$

worin k für eine ganze Zahl steht und mindestens 1 beträgt, R Wasserstoff oder Alkyl bezeichnet und $Z^7$ Halogen bedeutet, veräthert, oder

f) zur Herstellung der Verbindungen der Formel I, worin $X^1$ —NON— bezeichnet, eine Verbindung der allgemeinen Formel

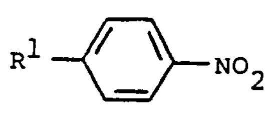

IX

mit einer Verbindung der allgemeinen Formel

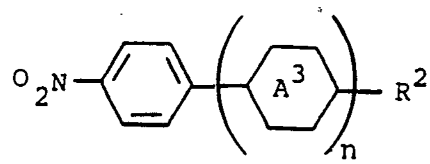

X

worin $R^1$, $R^2$, n und Ring $A^3$ die obigen Bedeutungen haben, in Gegenwart eines Reduktionsmittels umsetzt, oder

g) zur Herstellung der Verbindungen der Formel I, worin einer der Reste $R^1$ und $R^2$ 3E-Alkenyl oder 4-Alkenyl und der andere 1E-Alkenyl, 2Z-Alkenyl, 3E-Alkenyl oder 4-Alkenyl bedeutet, die Ringe $A^1$, $A^2$ und $A^3$ 1,4-Phenylen, 2-Fluor-1,4-phenylen oder trans-1,4-Cyclohexylen darstellen und $X^1$ eine einfache Kovalenzbindung, $—CH_2CH_2—$, $p\text{-}C_6H_4—$, $—CH_2CH_2\text{-}p\text{-}C_6H_4—$ oder $—CH_2CH_2\text{-}p\text{-}C_6H_4—CH_2CH_2—$ bezeichnet, eine Verbindung der allgemeinen Formel

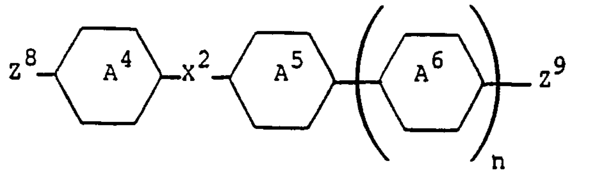

XXXIXa

worin n für die Zahl 0 oder 1 steht; die Ringe $A^4$, $A^5$ und $A^6$ 1,4-Phenylen, 2-Fluor-1,4-phenylen oder trans-1,4-Cyclohexylen darstellen; $X^2$ eine einfache Kovalenzbindung, $—CH_2CH_2—$, $p\text{-}C_6H_4—$, $—CH_2CH_2\text{-}p\text{-}C_6H_4—$ oder $—CH_2CH_2\text{-}p\text{-}C_6H_4—CH_2CH_2—$ bezeichnet; und einer der Reste $Z^8$ und $Z^9$ 3E-Alkenoyl oder 4-Alkenoyl und der andere 3E-Alkenyl, 4-Alkenyl, 3E-Alkenoyl, 3-Alkenoyl oder, sofern er nicht ann einen Benzol- oder Pyrimidinring gebunden ist, auch 1E-Alkenyl oder 2Z-Alkenyl bedeutet, reduziert, oder

h) zur Herstellung der Verbindungen der Formel I, worin einer der Reste $R^1$ und $R^2$ 3E-Alkenyl oder 4-Alkenyl und der andere 1E-Alkenyl, 2Z-Alkenyl, 3E-Alkenyl oder 4-Alkenyl bedeutet und $X^1$ eine einfache Kovalenzbindung, $—CH_2CH_2—$, $p\text{-}C_6H_4—$, $—CH_2CH_2\text{-}p\text{-}C_6H_4—$ oder $—CH_2CH_2\text{-}p\text{-}C_6H_4—CH_2CH_2—$ bezeichnet, eine Verbindung der allgemeinen Formel

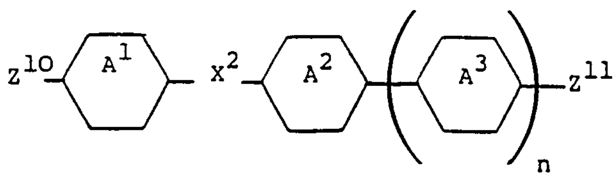

LV

worin einer der Reste $Z^{10}$ und $Z^{11}$ Halogenmethyl und der andere 3E-Alkenyl, 4-Alkenyl, Halogenmethyl oder, sofern er nicht an einen Benzol- oder Pyrimidinring gebunden ist, auch 1E-Alkenyl oder 2Z-Alkenyl bedeutet; $X^2$ eine einfache Kovalenzbindung, $—CH_2CH_2—$, $p\text{-}C_6H_4—$, $—CH_2CH_2\text{-}p\text{-}C_6H_4—$ oder $—CH_2CH_2\text{-}p\text{-}C_6H_4—CH_2CH_2—$ bezeichnet; und n und die Ringe $A^1$, $A^2$ und $A^3$ die obigen Bedeutungen haben, in Gegenwart von Kupfer-(I)-jodid mit (2E-Alkenyl)magnesiumhalogenid oder (3-Alkenyl)magnesiumhalogenid umsetzt.

Die Veresterung der Verbindungen der Formeln II und III oder von reaktionsfähigen Derivaten hiervon (z.B. Säurechlorid, Alkalimetallalkoholat oder -phenolat) gemäss Verfahrensvariante a) kann in an sich bekannter Weise erfolgen. Die Veresterung der Säurechloride kann beispielsweise in Diäthyläther, Tetrahydrofuran, Dimethylformamid, Benzol, Toluol, Tetrachlorkohlenstoff, Pyridin und dergleichen erfolgen. Die Veresterung einer Verbindung der Formel II mit einer Verbindung der Formel III erfolgt vorzugsweise in Gegenwart von 4-(Dimethylamino)pyridin und Dicyclohexylcarbodiimid oder in Gegenwart von Oxalylchlorid und Dimethylformamid. Temperatur und Druck dieser Veresterungsreaktionen sind nicht kritisch. Im allgemeinen werden Atmosphärendruck und eine Temperatur zwischen etwa −30°C und der Siedetemperatur des Reaktionsgemisches angewendet.

Die Umsetzung einer Verbindung der Formel IV mit einer Verbindung der Formel V gemäss Verfahrensvariante b) kann in an sich bekannter Weise erfolgen. Anstelle des Aldehyds kann auch ein geeignetes Acetal, z.B. das Dimethylacetal, eingesetzt werden. Zweckmässigerweise erfolgt die Umsetzung in einem inerten organischen Lösungsmittel (beispielsweise einem aromatischen Kohlenwasserstoff, wie Benzol, Toluol oder Xylol) in Gegenwart einer katalytischen Menge einer organischen oder anorganischen Säure, wie p-Toluolsulfonsäure oder trockener Chlorwasserstoff. Temperatur und Druck sind nicht kritisch, vorzugsweise wird jedoch bei Rückflusstemperatur und Atmosphärendruck gearbeitet.

Die Umsetzung einer Verbindung der Formel IV mit einer Verbindung der Formel V gemäss Verfahrensvariante c) kann ebenfalls in an sich bekannter Weise erfolgen. Zweckmässigerweise wird die Umsetzung in Wasser oder einem organischen Lösungsmittel (vorzugsweise einem Alkohol, wie Methanol, Aethanol, Aethylenglykol und dergleichen) in Gegenwart einer Base, vorzugsweise einem Alkalimetallalkoholat, wie Natriummethylat oder Natriumäthylat, durchgeführt. $R^3$ umfasst zweckmässigerweise Alkylreste mit 1 bis 5 Kohlenstoffatomen, wie Methyl, Aethyl, Propyl, Isopropyl und dergleichen. Temperatur und Druck sind keine kritischen Aspekte in dieser Reaktion. Im allgemeinen werden jedoch Atmosphärendruck und eine Temperatur zwischen Raumtemperatur und Rückflusstemperatur angewendet.

Die Umsetzung einer Verbindung der Formel VI mit Alkyl-triphenylphosphoniumhalogenid (vorzugsweise Alkyltriphenylphosphoniumbromid) in Gegenwart einer Base (gemäss Verfahrensvariante d) kann in an sich bekannter Weise erfolgen. Geeignete Basen sind Kalium-t-butylat, Natriummethylat, Kaliumcarbonat, Natriumhydrid, Natriumamid und dergleichen. Die Reaktion wird zweckmässigerweise in einem inerten organischen Lösungsmittel, beispielsweise einem Aether, wie Diäthyläther, Tetrahydrofuran

oder Dioxan, durchgeführt. Temperatur und Druck sind nicht kritisch; im allgemeinen wird jedoch bei Atmosphärendruck und einer Temperatur von Raumtemperatur bis Rückflusstemperatur gearbeitet.

Die Verätherung einer Verbindung der Formel VII mit einer Verbindung der Formel VIII (gemäss Verfahrensvariante e) kann in an sich bekannter Weise erfolgen. Zweckmässigerweise wird die Reaktion in Gegenwart einer Base (beispielsweise einem Alkalimetall, Alkalimetallhydrid oder Alkalimetallcarbonat, wie Natrium, Natriumhydrid, Kaliumhydrid oder Kaliumcarbonat) in einem inerten organischen Lösungsmittel, beispielsweise einem Kohlenwasserstoff, Aether, Keton oder Amid, wie Benzol, Tetrahydrofuran, Aceton oder Dimethylformamid, durchgeführt. Vorzugsweise steht $Z^7$ für Brom oder Jod. Die Verbindungen der Formel VII, worin die Hydroxygruppe an einen gesättigten Ring (Cyclohexan, m-Dioxan) gebunden ist, werden bevorzugt in Gegenwart von Natrium- oder Kaliumhydrid in einem Tetrahydrofuran/Dimethylformamid-Gemisch bei etwa 0°C bis Raumtemperatur umgesetzt. Die Verbindungen der Formel VII, worin die Hydroxygruppe an einem aromatischen Ring (Benzol, Pyrimidin) gebunden ist, werden bevorzugt in Gegenwart eines Alkalimetallcarbonats in Aceton bei einer Temperatur von Raumtemperatur bis Rückflusstemperatur, vorzugsweise bei Rückflusstemperatur, veräthert. Temperatur und Druck dieser Verätherungsreaktionen sind jedoch nicht kritisch und im allgemeinen können Atmosphärendruck und eine Temperatur von etwa 0°C bis Rückflusstemperatur angewendet werden.

Die Umsetzung einer Verbindung der Formel IX mit einer Verbindung der Formel X gemäss Verfahrensvariante f) kann in an sich bekannter Weise erfolgen. Als Reduktionsmittel eignen sich übliche, zur Reduktionn von Nitroverbindungen zu Nitrosoverbindungen und Hydroxylaminen geeignete Reduktionsmittel, wie Glucose, Alkohole (z.b. Methanol, Aethanol), Aldehyde (z.B. Formaldehyd), Hydrazin, Magnesium, Zink, Zinn-(II)-Verbindungen und dergleichen. Die Reaktion kann unter den üblichen, in der Literatur beschriebenen Bedingungen durchgeführt werden [z.B. K. H. Schündehütte in Houben-Weyl: Methoden der organischen Chemie, Band X-3, Seite 745 ff. (1965), Georg Thieme Verlag, Stuttgart]. Falls in den Formeln IX und X $R^1$ und $R^2$ verschiedene Bedeutungen haben, oder n für die Zahl 1 steht, werden bei dieser Reaktion im allgemeinen statistische Gemische mehrere Azoxyverbindungen erhalten. Diese Gemische können als solche verwendet oder, gewünschtenfalls, in an sich bekannter Weise, z.B. durch Chromatographie, getrennt werden.

Die Reduktion einer Verbindung der Formel XXXIXa gemäss Verfahrensvariante g) kann in an sich bekannter Weise erfolgen. Beispielsweise kann eine Verbindung der Formel XXXIXa mit Hydrazin in Gegenwart einer Base (z.B. Kaliumhydroxid, Natriumäthylat, Kalium-t-butylat) in einem inerten organischen Lösungsmittel, wie Dimethylsulfoxid, Aethanol, Diäthylenglykol oder Triäthylenglykol, umgesetzt und anschliessend das gebildete Hydrazon bei erhöhter Temperatur zersetzt werden. Eine bevorzugte Variante ist die Umsetzung nach dem Huang-Minolon-Verfahren, d.h. Erhitzen der Verbindung der Formel XXXIXa unter Rückfluss in einem hochsiedenden mit Wasser mischbaren Lösungsmittel (z.B. Diäthylenglykol oder Triäthylenglykol), zusammen mit Hydrazinhydrat und Kaliumhydroxid, anschliessendes Abdestillieren des Wassers bis zur Zersetzung des Hydrazons, und weiteres Kochen unter Rückfluss, bis die Reduktion beendet ist. Ferner können die Verbindungen der Formel XXXIXa durch Erwärmen mit amalgamiertem Zink und Salzsäure reduziert werden; gewünschtenfalls kann dem Reaktionsgemisch ein organisches Lösungsmittel, wie Aethanol, Essigsäure, Dioxan oder Toluol, zugesetzt werden.

Die Umsetzung der Verbindungen der Formel LV mit Grignard-Reagenzien gemäss Verfahrensvariante h) kann in an sich bekannter Weise erfolgen. Halogenmethyl steht vorzugsweise für Jodmethyl. Bevorzugte Alkenylmagnesiumhalogenide sind die Alkenylmagnesiumbromide. Die Reaktion wird zweckmässig in einem inerten organischen Lösungsmittel, beispielsweise einem Aether, wie Diäthyläther, Tetrahydrofuran oder Dioxan, in Gegenwart einer Base, wie Methylltihium, Butyllithium und dergleichen, durchgeführt. Temperatur und Druck sind nicht kritisch; vorzugsweise wird jedoch bei Atmosphärendruck und einer Temperatur von etwa −80°C bis Raumtemperatur gearbeitet. Die Hydrolyse des Reaktionsgemisches kann ebenfalls nach bekannten Methoden erfolgen, beispielsweise mit Wasser oder Ammoniumchlorid-Lösung.

Die Verbindungen der Formeln II, IV, VII und IX, worin $R^1$ Alkyl bedeutet, und die Verbindungen der Formel VIII sind bekannte oder Analoge bekannter Verbindungen.

Die Verbindungen der Formel XXXIXa sind neu und ebenfalls Gegenstand der vorliegenden Erfindung. Sie können nach an sich bekannten Methoden hergestellt werden, beispielsweise durch Umsetzung der entsprechenden Verbindungen, welche anstelle der Alkenoylgruppe eine Cyanogruppe aufweisen, mit (2E-Alkenyl)magnesiumbromid oder (3-Alkenyl)magnesiumbromid.

Die Verbindungen der Formeln II, IV, VII und IX, worin $R^1$ von Alkyl verschieden ist, und die Verbindungen der Formeln III, V, VI, X und LV sind ebenfalls neu. Die Herstellung dieser Verbindungen wird anhand repräsentativer Beispiele in den folgenden Reaktionsschemata 1—7 veranschaulicht, worin R Wasserstoff oder Alkyl, $R^3$ Alkyl und $R^4$ 1E-Alkenyl, 2Z-Alkenyl, 3E-Alkenyl oder 4-Alkenyl bedeuten.

Schema 1

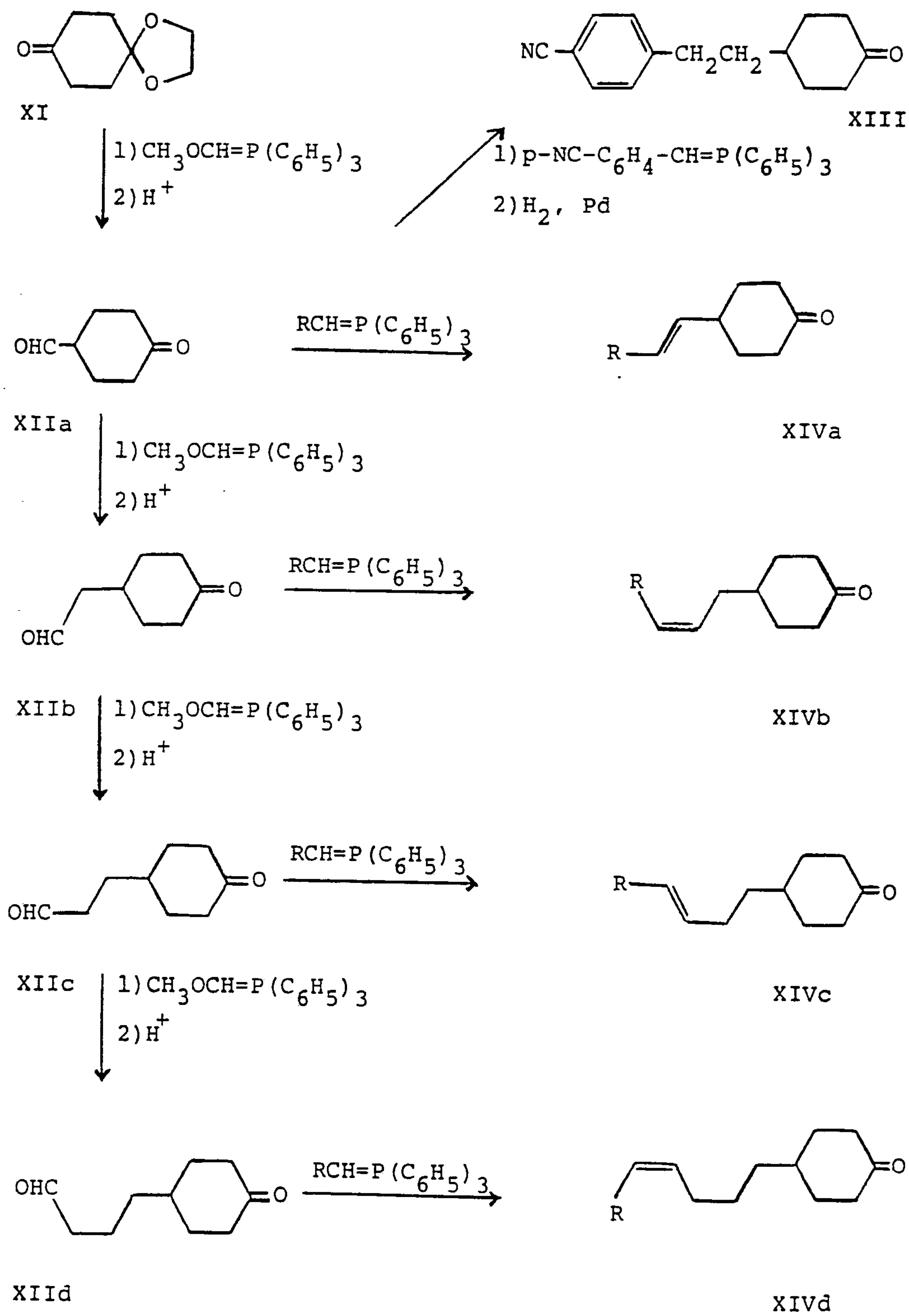


XI XIII XIIa XIVa XIIb XIVb XIIc XIVc XIId XIVd

Schema 2

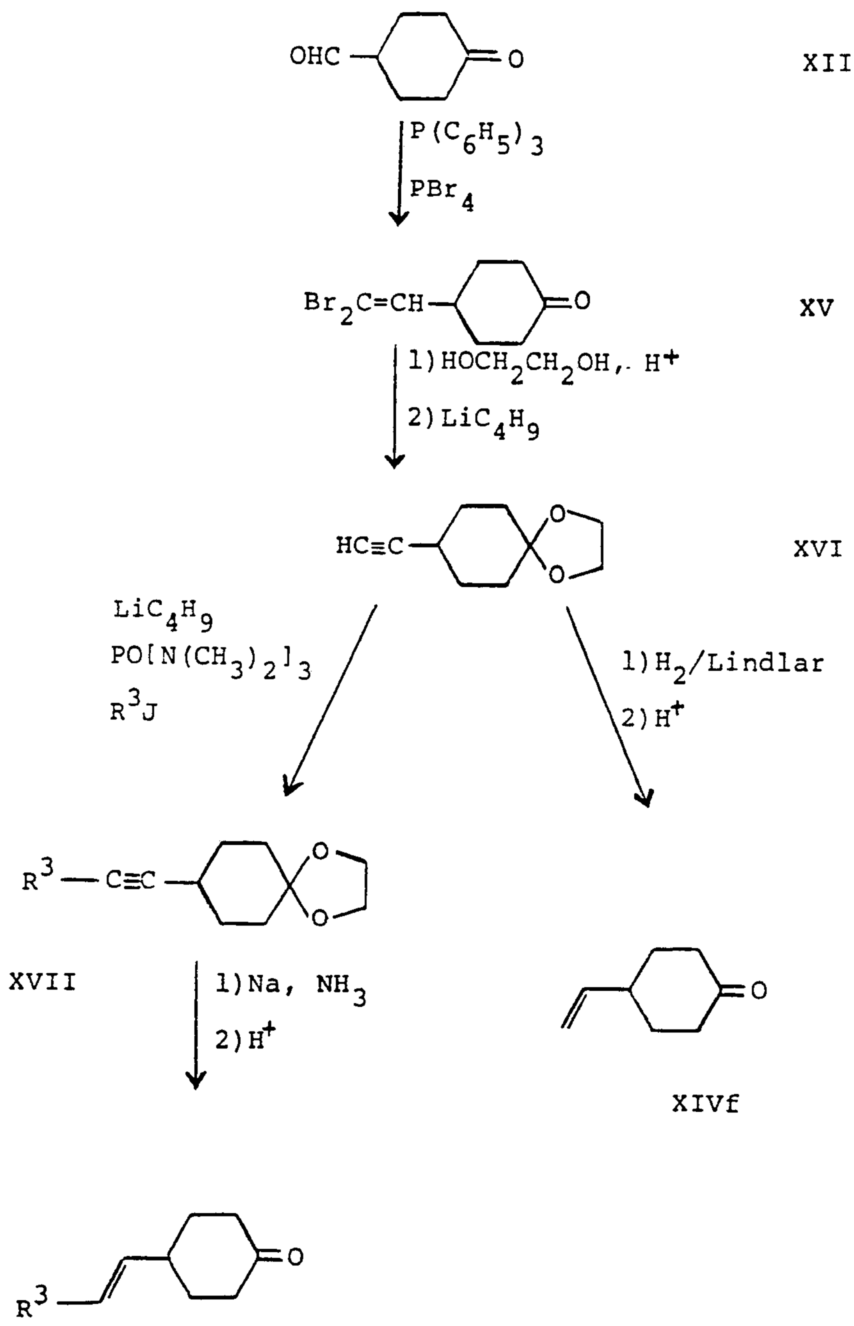


XIIa

XV

XVI

XVII

XIVf

XIVe

Schema 3

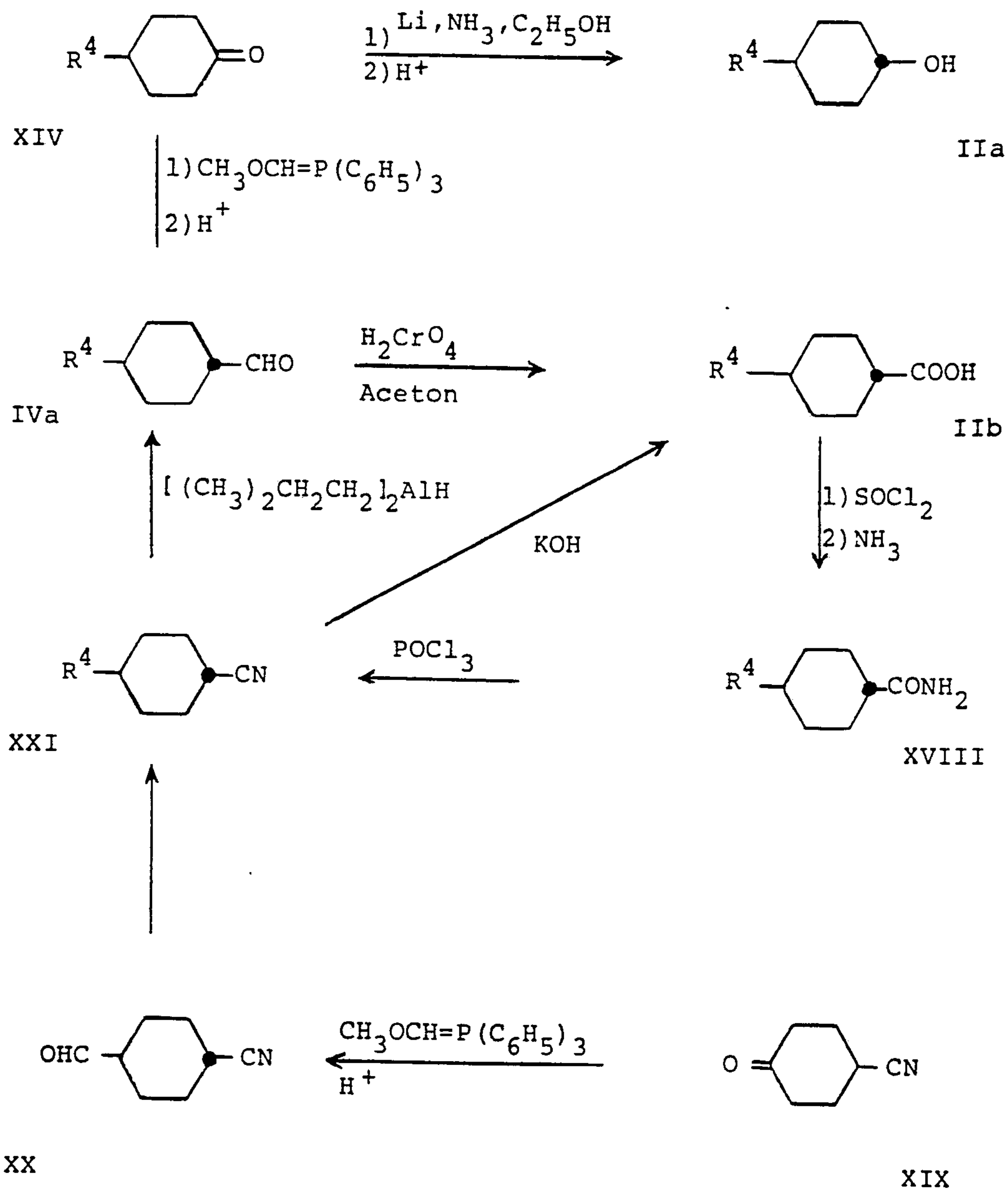


XIV

IIa

IVa

IIb

XXI

XVIII

XX

XIX

Schema 4

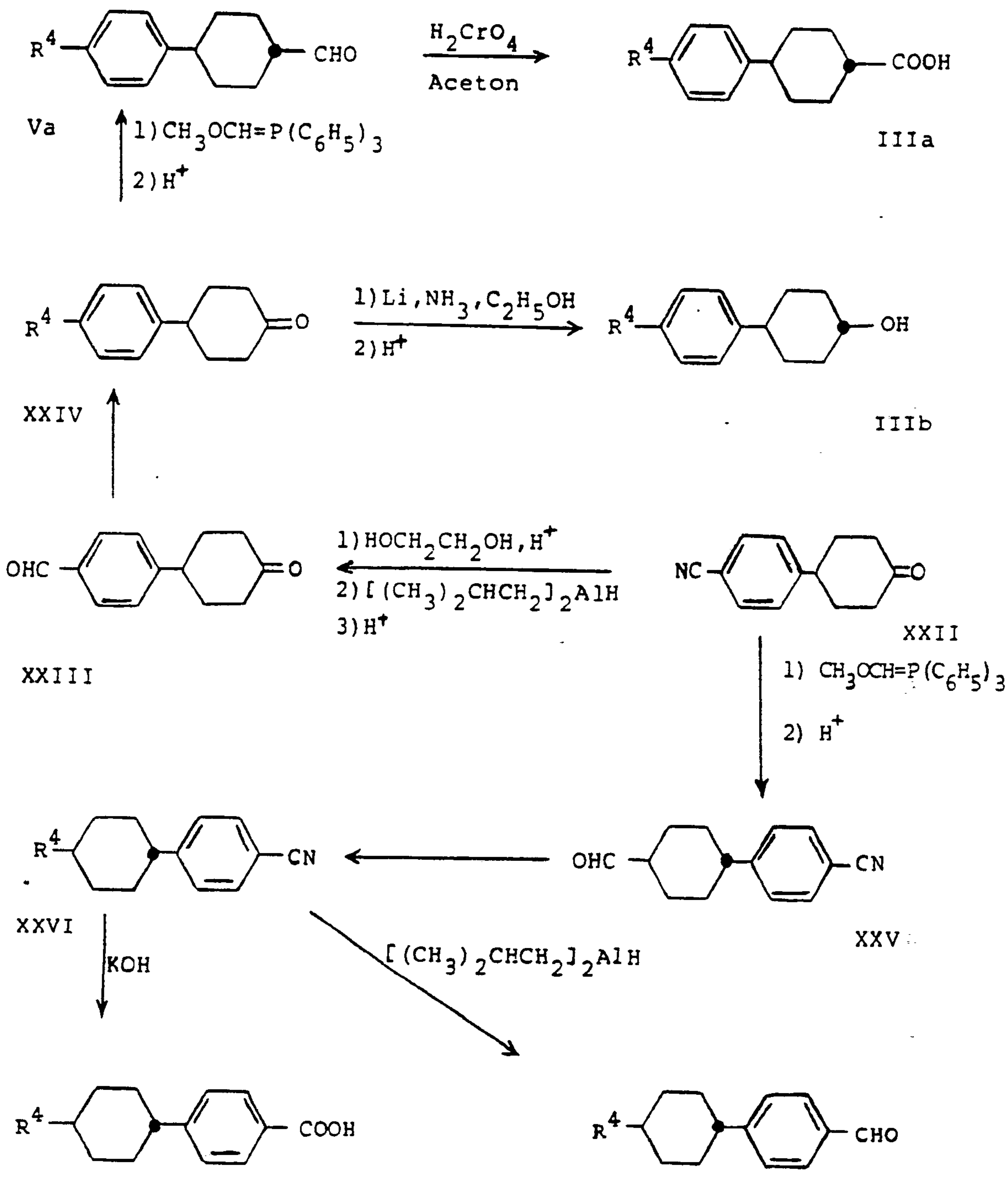


Va, IIIa, XXIV, IIIb, XXIII, XXII, XXVI, XXV, IIIc, Vb

Schema 5

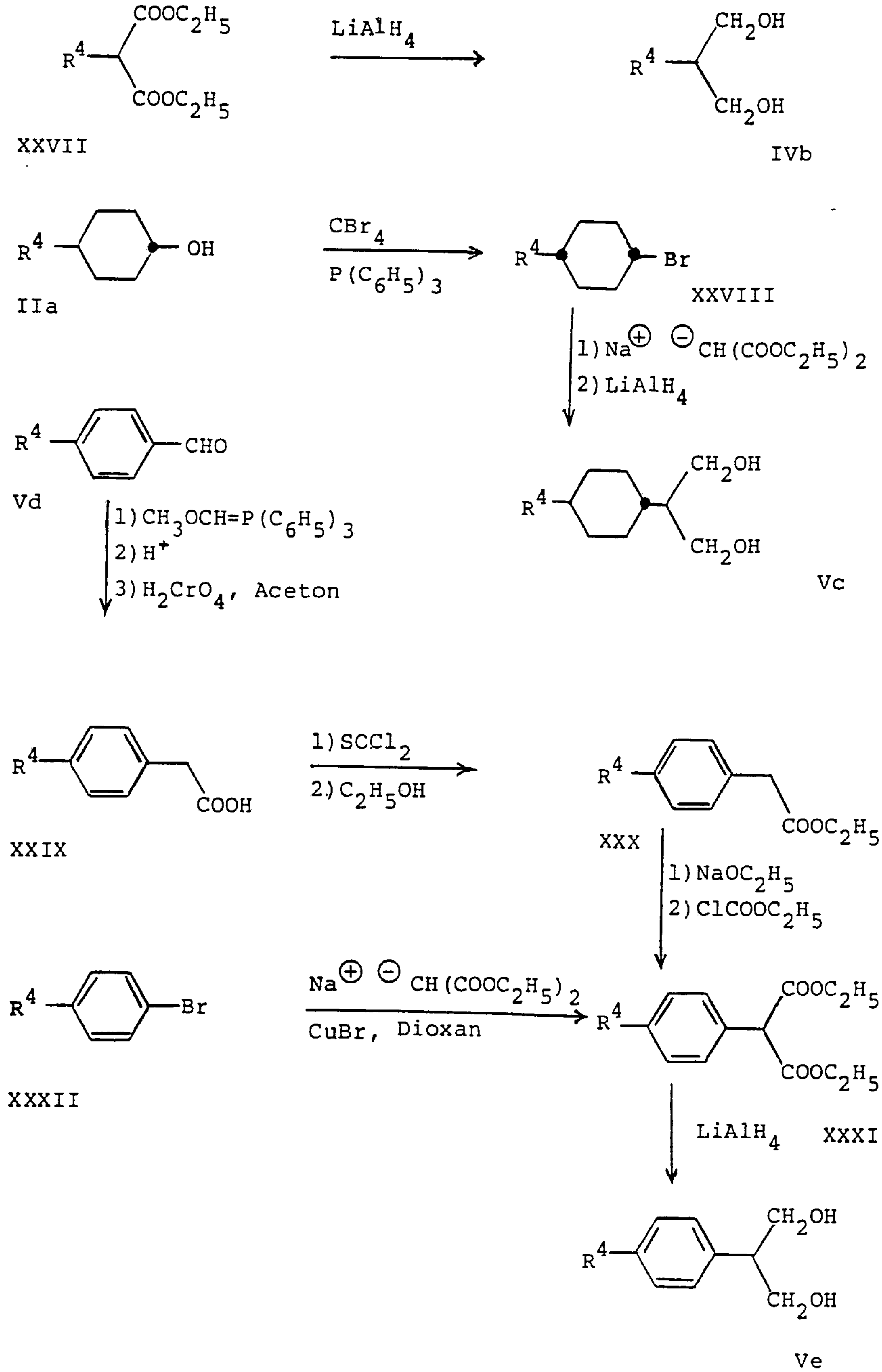


XXVII IVb IIa XXVIII Vd Vc XXIX XXX XXXII XXXI Ve

Schema 6

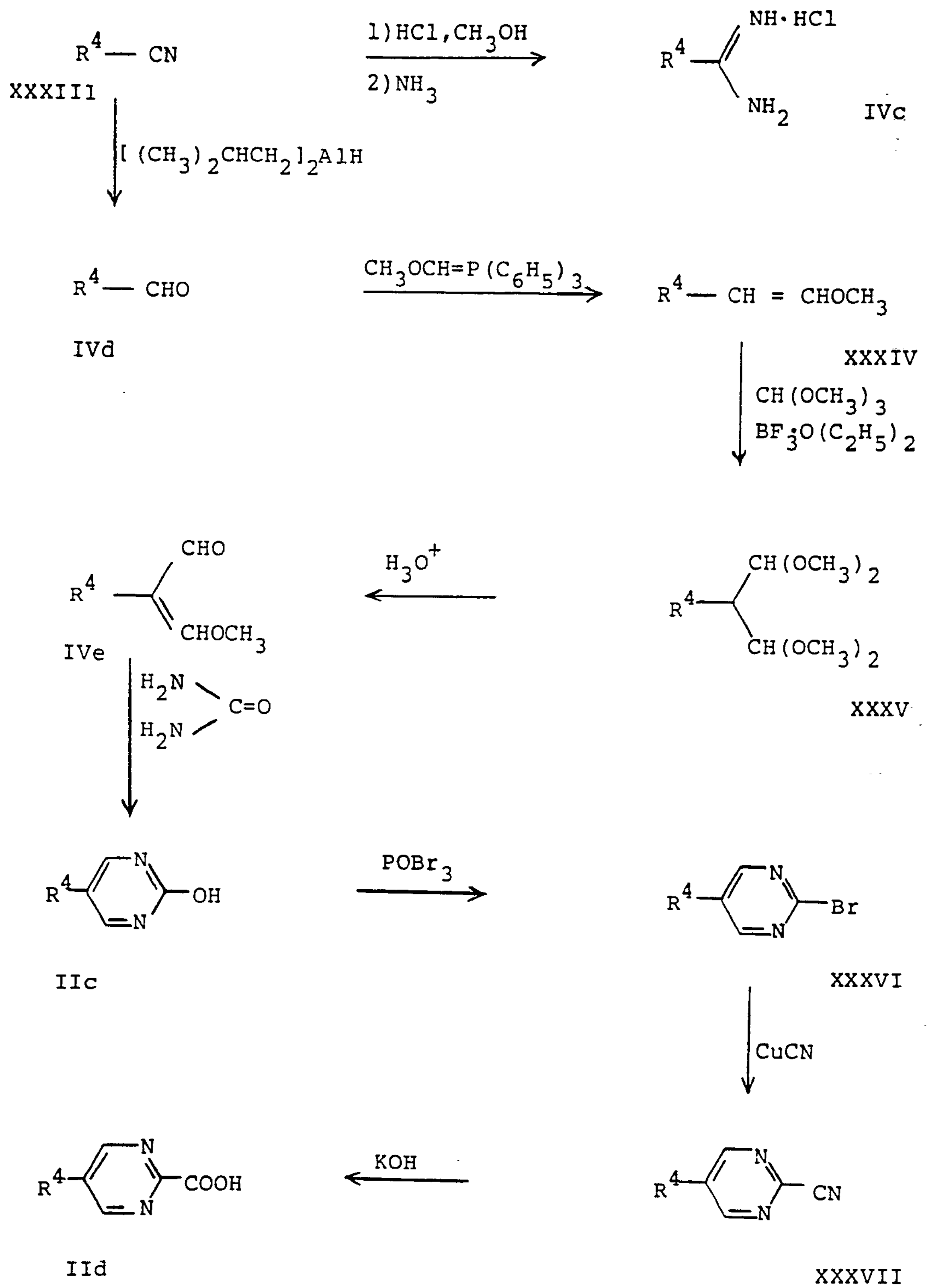
XXXIII
1) HCl, CH3OH
2) NH3
IVc
[(CH3)2CHCH2]2AlH
CH3OCH=P(C6H5)3
IVd
XXXIV
CH(OCH3)3
BF3·O(C2H5)2
H3O+
IVe
XXXV
POBr3
IIc
XXXVI
CuCN
KOH
IId
XXXVII

Schema 7

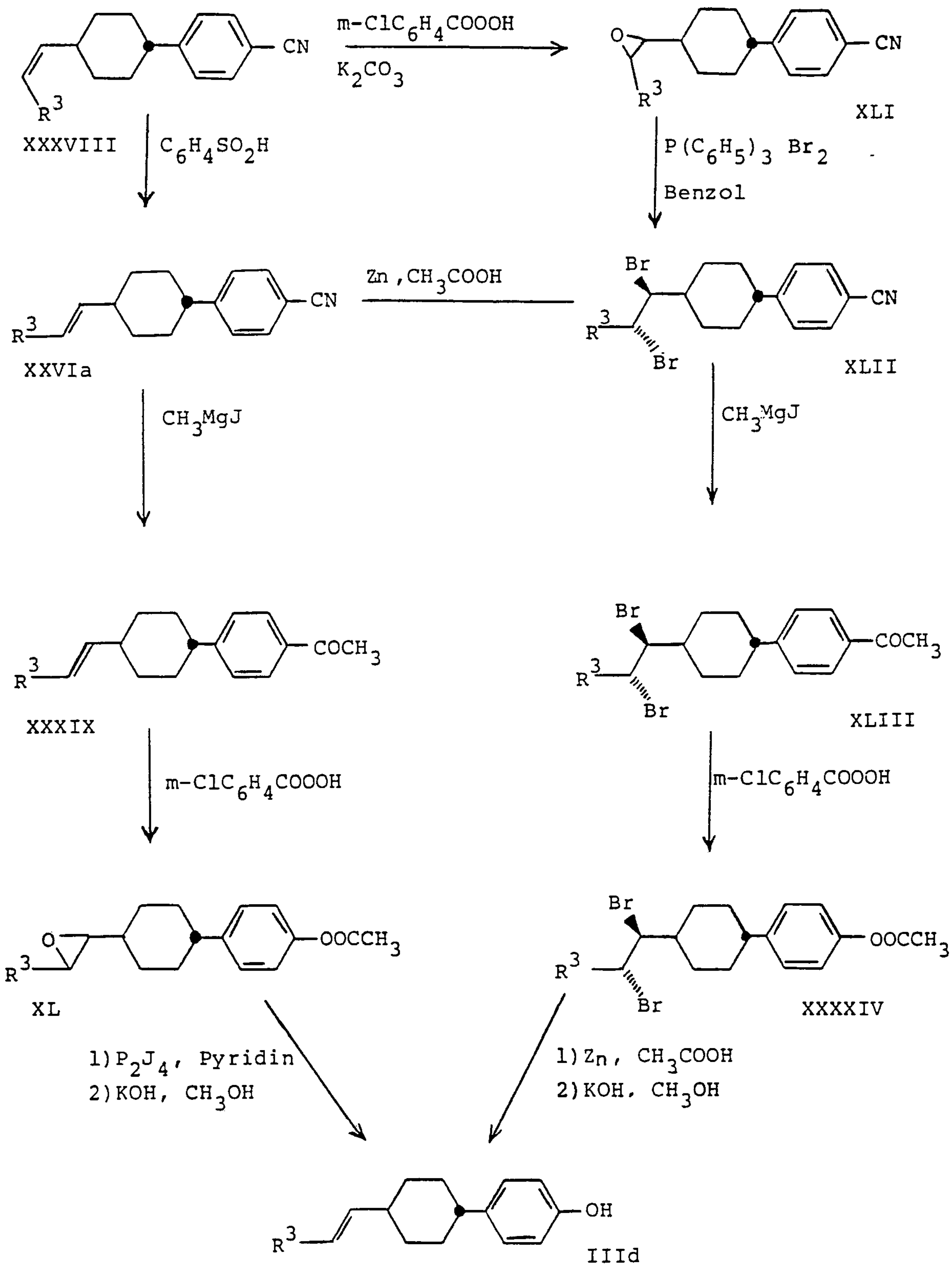


XXXVIII XLI

XXVIa XLII

XXXIX XLIII

XL XXXXIV

IIId

Die Verbindungen der Formel XIII können in analoger Weise zu Schema 3 zu entsprechenden Alkenylverbindungen weiter umgesetzt werden.

Die Einführung der Alkenylgruppen gemäss XX→XXI, XXIII→XXIV und XXV→XXVI kann in analoger Weise zu der in Schema 1 beschriebenen Methode erfolgen. Weitere Alternativen zur Einführung von Alkenylgruppen sind in den nachstehenden Synthesebeispielen 19—22 illustriert.

Alkenylphenole können beispielsweise durch Umsetzung von p-Hydroxybenzaldehyd mit (2-Methoxyäthoxy)methylchlorid (Schutz der Hydroxygruppe), anschliessende Einführung der Alkenylgruppe nach der in Schema 1 beschriebenen Methode und Abspaltung der Schutzgruppe mittels Zinkbromid erhalten werden.

4'-Cyano-4-biphenylcarboxaldehyd kann beispielsweise dadurch erhalten werden, dass man 4-Brombiphenyl zuerst mit $Cl_2CHOCH_3$ und Titantetrachlorid und dann mit Wasser umsetzt und den erhaltenen Brom-aldehyd mit Kupfer-(I)-cyanid in Dimethylformamid zum Cyano-aldehyd umsetzt. Dieser kann dann beispielsweise in analoger Weise zu Schema 3 weiter umgesetzt werden.

Die Halogenmethyl-Derivate der Formel LV können beispielsweise aus den entsprechenden Aldehyden erhalten werden durch Reduktion mit Natriumborhydrid, Umsetzung des Hydroxymethyl-Derivates mit p-Tosylchlorid und Ueberführung des Tosylates in das Halogenid, z.B. mit Natriumjodid in Aceton.

In obigen Reaktionen werden in einigen Fällen Gemische von cis/trans-isomeren Cyclohexanderivaten erhalten, welche jedoch in an sich bekannter Weise, z.B. durch Chromatographie und/oder Kristallisation, getrennt werden können.

Ferner werden in einigen Fällen E/Z-Gemische von Alkenylverbindungen erhalten, insbesondere bei der Einführung der Alkenylgruppen durch Wittig-Reaktion gemäss Schemata 1, 3 und 4 und gemäss Verfahrensvariante d). Derartige Gemische können ebenfalls nach an sich bekannten Methoden getrennt werden, z.B. durch Chromatographie. Hierbei hat sich insbesondere mit Silbernitrat imprägniertes Kieselgel als vorteilhaft erwiesen. Z-Isomere können gewünschtenfalls durch Aequilibrierung mit Sulfinsäuren, z.B. Benzolsulfinsäure oder p-Toluolsulfinsäure, in ein Gemisch mit mehrheitlich E-form übergeführt werden. Ferner kann beispielsweise die Z- bzw. E-Form durch Umsetzung mit Persäure (z.B. m-Chlorperbenzoesäure) in Gegenwart von Kaliumcarbonat, Halogenierung des Epoxids mit Triphenylphosphin-Brom in Benzol und Reduktion des Bromids mit Zink in Eisessig in die E- bzw. Z-Form übergeführt werden. Anderseits bleibt die Konfiguration beispielsweise erhalten, wenn eine Alkenylverbindung mit Persäure (z.B. m-Chlorperbenzosäure) oxidiert und das Epoxid mit Diphosphortetrajodid in Pyridin reduziert wird oder eine Alkenylverbindung mit Brom in Methylenchlorid halogeniert und das Bromid mit Zink in Eisessig reduziert wird. Derartige Reaktionen sind in obigem Schema 7 veranschaulicht und z.B. durch P. E. Sonnet in Tetrahedron *36*, 557—604 (1980) ausführlich beschrieben. Sie lassen sich vorteilhaften zur Optimierung der Ausbeute bezüglich des gewünschten Isomeren einsetzen.

Bei den Verfahrensvarianten a, b, c, e, f, g und h werden zweckmässigerweise Ausgangsmaterialien eingesetzt, bei welchen die Alkenylgruppe bereits in der gewünschten Konfiguration vorliegt.

Die Wittig-Reaktion gemäss Verfahrensvariante d ergibt im allgemeinen ein Gemisch, in welchem vorwiegend die entsprechende Z-Alkenylverbindung vorliegt. Zur Herstellung von Verbindungen der Formel I, worin $R^1$ und $R^2$ Z-Alkenylreste bedeuten kann daher eine Isomerisierung im allgemeinen unterbleiben. Zur Herstellung der Verbindungen der Formel I, worin einer der Reste $R^1$ und $R^2$ E-Alkenyl und der andere Z-Alkenyl bedeutet, wird vorzugsweise zuerst der E-Alkenylrest eingeführt (gegebenenfalls via Isomerisierung des Z-Alkenylrestes) und dann die Umsetzung gemäss Verfahrensvariante d) durchgeführt. Es kann aber auch zuerst der Z-Alkenylrest eingeführt und dieser während einer allfälligen Isomerisierung des andern Alkenylrestes beispielsweise als Dibromid (durch Umsetzung mit Brom) geschützt werden. Zur Herstellung der Verbindungen der Formel I, worin $R^1$ und $R^2$ E-Alkenylreste bedeuten, kann vorzugsweise die Isomerisierung beider Alkenylreste am Schluss erfolgen. Ein bereits vorhandener E-Alkenylrest kann aber gewünschtenfalls während der Isomerisierung des andern Restes als Dibromid geschützt werden. Selbstverständlich kann in allen Fällen auch lediglich eine chromatographische Trennung des erhaltenen Isomerengemisches erfolgen.

Bei der Umsetzung der Verbindungen der Formel XL (Schema 7) kann es von Vorteil sein, zuerst die Estergruppe zu verseifen, dann das erhaltene Epoxy-phenol in analoger Weise zur Verfahrensvariante e) mit einer Verbindung der Formel VIII zu veräthern und schliesslich die Epoxygruppe mit Diphosphortetrajodid in Pyridin zu reduzieren.

Die Verbindungen der Formeln IX und X können in analoger Weise zu den oben beschriebenen Methoden erhalten werden, beispielsweise aus Nitro-aldehyden oder Nitro-ketonen (z.B. p-Nitrobenzaldehyd, 4-(p-Nitrophenyl)cyclohexanon) durch allfällige Kettenverlängerung an der Aldehyd- oder Ketongruppe und Alkenylierung in analoger Weise zu den Schemata 1, 3 und 4. Die Isomerisierung eine Z-Alkenylgruppe kann beispielsweise durch Umsetzung mit N-Bromsuccinimid in Trifluoressigsäure und anschliessendes Erhitzen mit Natriumjodid in Dimethylformamid erfolgen. Ferner eignet sich auch die Aequilibrierung mit Benzolsulfinsäure oder die Umsetzung mit Diphenyldisulfid unter Lichteinfluss.

Die erfindungsgemässen Verbindungen können in Form von Gemischen untereinander und/oder mit anderen Flüssigkristallkomponenten verwendet werden, wie z.B. mit Substanzen aus den Klassen der Schiffschen Basen, Azobenzole, Azoxybenzole, Phenylbenzoate, Cyclohexancarbonsäurephenylester, Cyclohexancarbonsäurecyclohexylester, Biphenyle, Terphenyle, Phenylcyclohexane, Cyclohexylbiphenyle,

Phenylpyrimidine, Diphenylpyrimidine, Cyclohexylphenylpyrimidine, Phenyldioxane, 2-Cyclohexyl-1-phenyläthane und dergleichen. Derartige Substanzen sind dem Fachmann bekannt und viele davon sind zudem im Handel erhältlich.

Aufgrund der günstigen Eingenschaften der erfindungsgemässen Verbindungen kann ihr Anteil in flüssigkristallinen Gemischen im breiten Grenzen variieren und bis zu 100% betragen. Zweckmässigerweise enthalten die erfindungsgemässen Mischungen mindestens etwa 1 Gew.-% und vorzugsweise etwa 10—90 Gew.-% an Verbindungen der Formel I.

Bevorzugte Komponenten, welche im Gemisch mit einer oder mehreren Verbindungen der Formel I verwendet werden können, sind die Verbindungen der folgenden allgemeinen Formeln

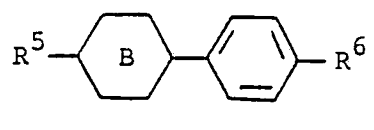

VL

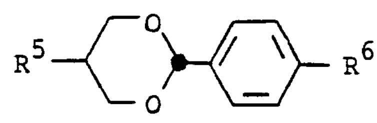

VLI

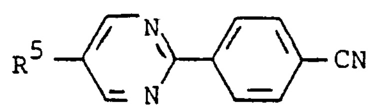

VLII

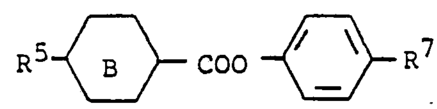

VLIII

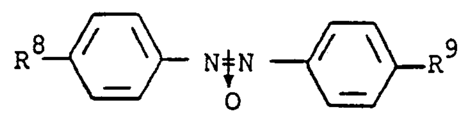

IL

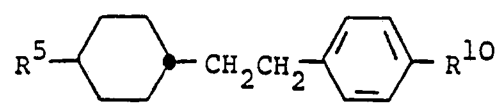

L

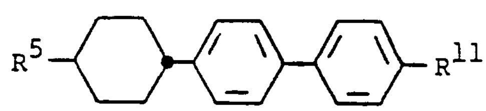

LI

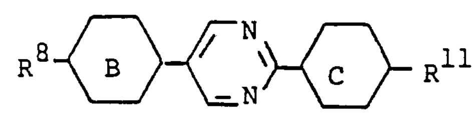

LII

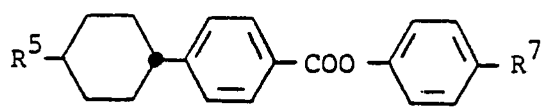

LIII

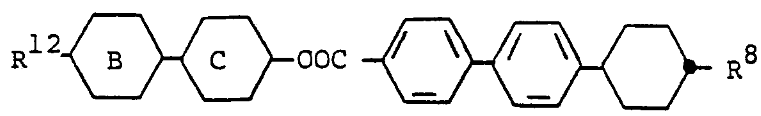

LIV

worin die Ringe B und C 1,4-Phenylen oder trans-1,4-Cyclohexylen darstellen; $R^5$ Alkyl, 3E-Alkenyl, 4-Alkenyl oder an einem Cyclohexanring auch 2Z-Alkenyl oder an einem Cyclohexan- oder m-Dioxanring auch 1E-Alkenyl bedeutet; $R^6$ Alkyl, Alkoxy, —CN oder —NCS bezeichnet; $R^7$ Alkyl oder Alkoxy darstellt; $R^8$ und $R^9$ Alkyl bedeuten; $R^{10}$ Cyano, Alkyl, Alkoxy, p-Alkylphenyl, p-Alkoxyphenyl, trans-4-Alkylcyclohexyl, 4'-Alkyl-4-biphenylyl, p-(trans-4-Alkylcyclohexyl)phenyl, 2-(trans-4-Alkylcyclohexyl)äthyl, p-[2-(trans-4-Alkylcyclohexyl)äthyl]phenyl oder 2-[p-(trans-4-Alkylcyclohexyl)phenyl]äthyl darstellt; $R^{11}$ Cyano oder Alkyl bezeichnet; $R^{12}$ Alkyl, 3E-Alkenyl oder an einem Cyclohexanring auch 1E-Alkenyl bedeutet; und die Alkyl-, Alkoxy- und Alkenylreste in $R^5$, $R^6$, $R^7$, $R^8$, $R^9$, $R^{10}$, $R^{11}$ und $R^{12}$ geradkettige Reste mit höchstens 7 Kohlenswasserstoffatomen sind.

Die erfindungsgemässen Mischungen können ebenfalls optisch aktive Verbindungen (beispielsweise optische aktive Biphenyle) und/oder dichroitische Farbstoffe (beispielsweise Azo-, Azoxy- oder Anthrachinonfarbstoffe) enthälten. Der Anteil solcher Verbindungen wird durch die Löslichkeit, die gewünschte Ganghöhe, Farbe, Extinktion und dergleichen bestimmt. Vorzugsweise beträgt der Anteil optisch aktiver Verbindungen höchstens etwa 4 Gew.-% und der Anteil dichroitischer Farbstoffe höchstens etwa 10 Gew.-% im Gesamtgemisch.

Die Herstellung der erfindungsgemässen Mischungen kann in an sich bekannter Weise erfolgen, z.B.

durch Erhitzen einer Mischung der Bestandteile auf eine Temperatur knapp oberhalb des Klärpunktes und anschliessendes Abkühlen. Die Herstellung einer elektro-optischen Vorrichtung kann ebenfalls in an sich bekannter Weise erfolgen, z.B. durch Evakuieren einer geeigneten Zelle und Einbringen der Mischung in die evakuierte Zelle.

Die Verbindungen der obigen Formeln VL—VLIII, L, LI, LIII und LIV, worin $R^5$ bzw. $R^{12}$ Alkenyl bedeutet, sind neu. Sie können nach den oben beschriebenen Methoden hergestellt werden.

Die Herstellung der erfindungsgemässen Verbindungen wird durch die folgende Beispiele weiter veranschaulicht. C bedeutet eine kritstalline, S eine smektische, $S_B$ eine smektische B, N eine nematische und I die isotrope Phase.

Beispiel 1

a) 261,2 g Methoxymethyl-triphenylphosphoniumchlorid wurden in 550 ml t-Butylmethyläther aufgeschlämmt und bei −10°C mit 90,53 g Kalium-t-butylat versetzt. Das Kühlbad wurde entfernt und das Gemisch noch 1 Stunde bei Raumtemperatur gerührt. Anschliessend wurde die Suspension bei −10°C langsam mit einer Lösung von 70 g 4,4-Aethylendioxycyclohexanon in 350 ml Tetrahydrofuran versetzt, noch 1 Stunde bei Raumtemperatur gerührt, dann mit Wasser versetzt und dreimal mit Diäthyläther extrahiert. Die organischen Phasen wurden zweimal mit Wasser gewaschen und die wässrigen Phasen mit Diäthyläther nachextrahiert. Die vereinigten organischen Phasen wurden über Magnesiumsulfat getrocknet und eingeengt. Der erhaltene, krisalline Rückstand wurde in Aethylacetat gelöst, mit Petroläther verdünnt, filtriert und vom Lösungsmittel befreit. Destillation des erhaltenen, gelben Oeles (100 g) ergab im Hauptlauf (71°C/0,20—0,17 Torr) 75,28 g (91,17%) 1,1-Aethylendioxy-4-(methoxymethylen)cyclohexan als klare, farblose Flüssigkeit.

b) Ein Gemisch von 10,55 g 1,1-Aethyendioxy-4-(methoxymethylen)cyclohexan, 130 ml Wasser und 200 ml Eisessig wurde 1 Stunde zum Rückfluss erhitzt. Anschliessend wurde am Rotationsverdampfer das Lösungsmittel abdestilliert und das Destillat zweimal mit Methylenchlorid extrahiert. Der Destillationsrückstand (gelbes Oel) wurde mit 200 ml Wasser verdünnt, mit Natriumcarbonat-Lösung neutralisiert und dreimal mit Methylenchlorid extrahiert. Die organischen Phasen wurden mit gesättigter Natriumcarbonat-Lösung gewaschen und die wässrigen Phasen mit Methylenchlorid nachextrahiert. Die organischen Phasen wurden über Magnesiumsulfat getrocknet und eingedampft. Destillation des zurückbleibenden, gelben Oeles ergab bei 70°C/0,15 Torr 6,7 g (93%) 4-Formylcyclohexanon.

Beispiel 2

a) Eine Lösung von 36,72 g Triphenylphosphin in 200 ml Methylenchlorid wurde bei −20°C langsam mit 23,22 g Tetrabromkohlenstoff versetzt und noch 10 Minuten gerührt. Anschliessend wurde das Gemisch mittels einer Kanüle zu einer auf −60°C gekühlten Lösung von 6,30 g 4-Formylcyclohexanon in 100 ml Methylenchlorid zugetropft. Das Reaktionsgemisch wurde noch 15 Minuten −60°C gerührt und dann in Wasser/Methylenchlorid verteilt. Die wässrigen Phasen wurden noch zweimal mit Methylenchlorid extrahiert. Die organischen Phasen wurden zweimal mit Wasser gewaschen, über Magnesiumsulfat getrocknet und eingedampft. Niederdruckchromatographie des erhaltenen hellgelben Oeles (16 g) an Kieselgel mit Aethylacetat/Petroläther (Vol. 10:90) ergab 12,08 g (85,8%) 4-(2,2-Dibromvinyl)cyclohexanon als hellgelbe Flüssigkeit.

b) Ein Gemisch von 2 g 4-(2,2-Dibromvinyl)cyclohexanon, 3,43 g Aethylenglykol, 0,202 g p-Toluolsulfonsäure und 240 ml Benzol wurde unter Wasserabscheidung 5 Stunden am Rückfluss gekocht. Anschliessend wurde das Reaktionsgemisch mit Kaliumcarbonat versetzt, kurz gerührt und über Nacht stehen gelassen. Danach wurde das Gemisch filtriert und das Filtrat am Rotationsverdampfer von Lösungsmittel befreit. Der Rückstand wurde in 200 ml Methylenchlorid aufgenommen und zweimal mit verdünnter Natronlauge und einmal mit Wasser gewaschen. Die wässrigen Phasen wurden mit Methylenchlorid nachextrahiert. Die organischen Phasen wurden über Magnesiumsulfat getrocknet, filtriert und eingedampft. Hierbei wurden 14 g 1,1-Aethylendioxy-4-(2,2-dibromvinyl)cyclohexan als leicht gelbe, kristallisierende Flüssigkeit erhalten.

c) Eine Lösung von 14 g 1,1-Aethylendioxy-4-(2,2-dibromvinyl)cyclohexan in 70 ml Tetrahydrofuran wurde auf −20°C gekühlt und bei dieser Temperatur langsam mit 76,62 ml einer 1,4M Lösung von Butyllithium in Hexan versetzt (exotherme Reaktion). Das Kühlbad wurde entfernt und das Gemisch innert etwa 20 Minuten auf 20°C erwärmen gelassen. Anschliessend wurde das Reaktionsgemisch mit 150 ml Wasser versetzt und dreimal mit Diäthyläther extrahiert. Die organischen Phasen wurden zweimal mit Wasser gewaschen und die Waschwasser mit Diäthyläther nachextrahiert. Die organischen Phasen wurden über Magnesiumsulfat getrocknet, filtriert und eingedampft. Niederdruckchromatographie der zurückbleibenden, gelben Flüssigkeit (8 g) an Kieselgel mit Aethylacetat/Petroläther (Vol. 7:93) ergab 6,5 g (91%) 4-Aethinyl-1,1-äthylendioxy-cyclohexan als klare Flüssigkeit.

d) Eine Lösung von 6,5 g 4-Aethinyl-1,1-äthylendioxy-cyclohexan in 40 ml Tetrahydrofuran wurde bei −20°C mit 39,1 ml einer 1,4M Lösung von Butyllithium in Hexan versetzt. Anschliessend wurde das Gemisch bei 0°C mit 60 ml Hexamethylphosphorsäuretriamid (kurzer Temperaturanstieg auf 26°C) und dann tropfenweise mit 6,5 ml Propyljodid versetzt. Das Kühlbad wurde entfernt und das Gemisch auf Raumtemperatur erwärmen gelassen. Hierbei bildete sich ein weisser Niederschlag. Nach 30 Minuten wurde das Reaktionsgemisch mit 150 ml Wasser versetzt und dreimal mit Hexan extrahiert. Die

organischen Phasen wurden dreimal mit Wasser gewaschen und die Waschwasser mit Hexan nachextrahiert. Die organischen Phasen wurden über Magnesiumsulfat getrocknet, filtriert und am Rotationsverdampfer vom Lösungsmittel befreit. Niederdruckchromatographie der erhaltenen gelben Flüssigkeit (9 g) an Kieselgel mit Aethylacetat/Petroläther (Vol. 10:90) und Behandlung mit Aktivkohle ergab 6,23 g (76,5%) 1,1-Aethylendioxy-4-(1-pentinyl)cyclohexan als leicht hellgelbe Flüssigkeit.

e) In einem Sulfierkolben mit Magnetrührer wurde eine Lösung von 4,5 g 1,1-Aethylendioxy-4-(1-pentinyl)cyclohexan in 54 ml Tetrahydrofuran tropfenweise mit ca. 50 ml vorkondensiertem Ammoniak versetzt. Anschliessend wurde das Gemisch bei −78°C innert 7 Stunden portionenweise mit 1,3 g Natrium versetzt. 1,5 Stunden nach der letzten Zugabe wurde das Ammoniak abgedampft und das Reaktionsgemisch mit 25-proz. Salzsäure neutralisiert und über Nacht stehen gelassen. Danach wurde das Reaktionsgemisch dreimal in Wasser/Diäthyläther verteilt. Die wässrigen Phasen wurden mit Diäthyläther nachextrahiert. Die organischen Phasen wurden über Magnesiumsulfat getrocknet und am Rotationsverdampfer vom Lösungsmittel befreit. Die erhaltene, hellgelbe Flüssigkeit (3,8 g) wurde mit 200 ml Aceton und 0,1 ml konzentrierter Schwefelsäure versetzt. Das Gemisch wurde 10 Minuten zum Rückfluss erhitzt, dann mit Wasser versetzt und am Rotationsverdampfer vom Aceton befreit. Der Rückstand wurde dreimal in Methylenchlorid/Wasser verteilt. Die wässrigen Phasen wurden mit Methylenchlorid nachextrahiert. Die organischen Phasen wurden über Magnesiumsulfat getrocknet und eingedampft. Chromatographische Trennung der zurückbleibenden, gelben Flüssigkeit (3,5 g) an Kieselgel mit Aethylacetat/Petroläther (Vol. 7:93) ergab 3,0 g (83,5%) 4-(1E-Pentenyl)cyclohexanon als hellgelbe Flüssigkeit.

## Beispiel 3

1,63 g 4-(1E-Pentenyl)cyclohexanon wurden in 8 ml Diäthyläther und 14 ml Aethanol gelöst Anschliessend wurde die Lösung tropfenweise mit ca. 70 ml Ammoniak versetzt und portionsweise solange mit Lithiumdraht versetzt, bis die Farbe des Reaktionsgemisches 1,5 Stunden lang konstant blieb (ca. 1,3 g Lithium). Danach wurde das Ammoniak abgedampft und das Gemisch mit Ammoniumchlorid und Salzsäure sauer gestellt und 3 Tage stehengelassen. Dann wurde das Reaktionsgemisch in Diäthyläther/Wasser verteilt und die wässrige Phase mit Diäthyläther nachextrahiert. Die organischen Phasen wurden über Magnesiumsulfat getrocknet, filtriert und am Rotationsverdampfer von Lösungsmittel befreit. Niederdruckchromatographie des zurückbleibenden, gelben Oeles an Kieselgel mit Aethylacetat/Petroläther (Vol. 10:90) ergab 1,47 g (89,1%) trans-4-(1E-Pentenyl)cyclohexanol als leicht gelbes, viskoses Oel.

In analoger Weise wurde folgende Verbindung hergestellt: trans-4-(1E-Butenyl)cyclohexanol.

## Beispiel 4

a) 7 g Methoxymethyl-triphenylphosphoniumchlorid wurden in 35 ml t-Butylmethyläther aufgeschlämmt und bei −20°C mit 2,43 g Kalium-t-butylat versetzt. Das Gemisch wurde noch 1 Stunde bei Raumtemperatur gerührt, dann bei −20°C tropfenweise mit einer Lösung von 2 g 4-(1E-Pentenyl)cyclohexanon in 18 ml Tetrahydrofuran versetzt und nochmals 1 Stunde bei Raumtemperatur gerührt. Anschliessend wurde das Reaktionsgemisch mit Wasser versetzt und dreimal mit je 50 ml Diäthyläther extrahiert. Die Extrakte wurden mit Wasser gewaschen und die Waschwasser mit Diäthyläther nachextrahiert. Die organischen Phasen wurden über Magnesiumsulfat getrocknet und vom Lösungsmittel befreit. Der Rückstand wurde in Aethylacetat gelöst und die Lösung mit Petroläther verdünnt, durch Filtration vom ausgefallenen Triphenylphosphinoxid befreit und erneut eingeengt. Dieser Vorgang zur Abtrennung von Triphenylphosphinoxid wurde noch zweimal wiederholt und das erhaltene Rohprodukt von 1-(Methoxymethylen)-4-(1E-pentyl)cyclohexan ohne zusätzliche Reinigung weiterverwendet.

b) 2,75 g 1-(Methoxymethylen)-4-(1E-pentenyl)cyclohexan (Rohprodukt aus Absatz a) wurden mit 100 ml Tetrahydrofuran/2N Salzsäure (Vol. 4:1) 30 Minuten zum Rückfluss erhitzt und dann über Nacht bei Raumtemperatur gerührt. Anschliessend wurde das Gemisch mit 100 ml Wasser versetzt und dreimal mit je 100 ml Diäthyläther extrahiert. Die Extrackte wurden mit verdünnter Natriumhydrogencarbonat-Lösung und Wasser gewaschen und die wässrigen Phasen mit Diäthyläther nachextrahiert. Die organischen Phasen wurden über Magnesiumsulfat getrocknet und eingedampft. Niederdruckchromatographie der zurückbleibenden gelblichen Flüssigkeit (2,5 g) an Kieselgel mit Petroläther und Aethylacetat/Petroläther (Vol. 3:97) ergab 1,65 g (76%) 4-(1E-Pentenyl)cyclohexancarboxaldehyd.

c) Eine Lösung von 1,6 g 4-(1E-Pentenyl)cyclohexancarboxaldehyd in 120 ml Aceton wurde auf −10°C gekühlt, tropfenweise mit 8N Chromsäure (ca. 10 ml) versetzt, bis die Farbe des Reaktionsgemisches braunorange blieb, und noch 1 Stunde gerührt. Ueberschüssige Chromsäure wurde durch Zugabe von Isopropanol reduziert. Anschliessend wurde die grüne Lösung dreimal in Wasser/Methylenchlorid verteilt. Die organischen Extrakte wurden zweimal mit Wasser gewaschen und die Waschwasser mit Methylenchlorid nachextrahiert. Die organischen Phasen wurden über Magnesiumsulfat getrocknet und eingedampft. Der hellbraune kristalline Rückstand (2,01 g) wurde in 20 ml Petroläther teilweise gelöst. Ungelöster Rückstand wurde abfiltriert und das Filtrat eingedampft. Umkristallisation des erhaltenen Rückstandes aus 60 ml Petroläther bei −78°C ergab 866 mg (49,5%) 4-(1E-Pentenyl)cyclohexancarbonsäure als weisse Kristalle.

d) Ein Gemisch von 1,29 g 4-(1E-Pentenyl)cyclohexancarbonsäure und 20 ml einer 11-proz. (Gew./Vol.)

Lösung von Kaliumhydroxid in Diäthylenglykol wurde unter Argonbegasung 20 Stunden am Rückfluss gekocht. Anschliessend wurde das Reaktionsgemisch mit 25-proz. Salzsäure schwach sauer gestellt und dreimal in Wasser/Methylenchlorid verteilt. Die organischen Extrakte wurden zweimal mit Wasser gewaschen und die Waschwasser mit Methylenchlorid nachextrahiert. Die organischen Phasen wurden über Magnesiumsulfat getrocknet und an Rotationsverdampfer von Lösungsmittel befreit. Umkristallisation des erhaltenen, dunkelbraunen, kristallisierenden Oeles (1,17 g) aus 50 ml Petroläther bei −78°C ergab 0,44 trans-4-(1E-Pentenyl)cyclohexancarbonsäure als hellbraune Kristalle. Die Mutterlauge enthaltend 0,695 g rohe cis/trans-4-(1E-Pentenyl)cyclohexancarbonsäure wurde nicht aufgearbeitet.

In analoger Weise wurde folgende Verbindung hergestellt: trans-4-(1E-Butenyl)cyclohexancarbonsäure.

Beispiel 5

a) In einem Sulfierkolben mit Thermometer, mechanischem Rührer, Tropftrichter und Festsubstanzzugaberohr wurden unter Argonbegasung 10,4 g Methoxymethyl-triphenylphosphoniumchlorid in 60 ml t-Butylmethyläther aufgeschlämmt und bei −10°C innert 10 Minuten mit 3,6 g festem Kalium-t-butylat versetzt. Nach beendeter Zugabe wurde noch während 30 Minuten bei −10°C bis 0°C gerührt und dann das tieforange, heterogene Reaktionsgemisch bei 0°C tropfenweise mit einer Lösung von 4,2 g 4-(p-Cyanophenyl)cyclohexanon in 50 ml absolutem Tetrahydrofuran versetzt. Anschliessend wurde das Reaktionsgemisch noch 2 stunden bei Raumtemperatur gerührt, dann auf 500 ml Hexan gegossen und filtriert. Niederdruckchromatographie (0,5 bar) des eingeengten Rückstandes (7,1 g) an Kieselgel mit Aethylacetat/Petroläther (Vol. 5:95) ergab 4,5 g (94%) p-(4-(Methoxymethylen)cyclohexyl]benzonitril als farbloses Oel; Reinheit 95%, Rf-Wert (Aethylacetat/Petroläther Vol. 1:9) 0,30.

b) In einem Rundkolben wurde ein Gemisch von 4,2 g p-(4-(Methoxymethylen)cyclohexyl]benzonitril und 100 ml Tetrahydrofuran/2N Salzsäure (Vol. 4:1) während 30 Minuten zum Rückfluss erhitzt. Anschliessend wurde das Reaktionsgemisch auf 100 ml Wasser gegossen und dreimal mit je 100 ml Diäthyläther extrahiert. Die orgnaischen Phasen wurden noch einmal mit 100 ml Wasser gewaschen, über Magnesiumsulfat getrocknet und eingeengt. Es resultierten 3,9 g (100%) 4-(p-Cyanophenyl)cyclohexancarboxaldehyd als farbloses Oel, welches ohne weitere Reinigung in der nächsten Stufe eingesetzt wurde; trans/cis-Verhältnis ca. 3:1, Rf-Wert (Aethylacetat/Petroläther Vol. 3:7) 0,41. Durch Kristallisation aus Hexan konnte reiner trans-4-(p-Cyanophenyl)cyclohexancarboxaldehyd erhalten werden; Smp. 57,1°C.

c) Eine Suspension von 29,0 g Methoxymethyl-triphenylphosphoniumchlorid in 200 ml t-Butylmethyläther wurde unter Argonbegasung bei −10°C innert 10 Minuten mit 9,7 g Kalium-t-butylat versetzt und noch 1 Stunde gerührt. Dann wurde das Gemisch bei −10°C innert 15 Minuten tropfenweise mit einer Lösung von 12,0 g trans-4-(p-Cyanophenyl)cyclohexancarboxaldehyd im 90 ml t-Butylmethyläther versetzt und noch 1 Stunde bei 0°C gerührt. Anschliessend wurde das Reaktionsgemisch auf 150 ml Wasser gegossen und dreimal mit je 150 ml Diäthyläther extrahiert. Die organischen Phasen wurden zweimal mit je 100 ml Wasser gewaschen, über Magnesiumsulfat getrocknet und eingeengt. Das resultierende, zähflüssige Oel wurde in 20 ml Aethylacetat gelöst und die klare Lösung mit 300 ml Petroläther verdünnt und 10 Minuten bei −20°C stehengelassen. Anschliessend wurde das ausgefallene Triphenylphosphinoxid abfiltriert und das Filtrat zur Trockene eingeengt. Niederdruckchromatographie (0,5 bar) des Rückstandes (16,3 g) an Kieselgel mit Aethylacetat/Petroläther (Vol. 5:95) ergab 10,1 g (74%) p-[trans-4-(2-Methoxyvinyl)cyclohexyl]benzonitril als farblose Kristalle; Rf-Wert (Aethylacetat/Petroläther Vol. 10:90) 0,32.

d) Eine Lösung von 10,1 g p-[trans-4-(2-Methoxyvinyl)cyclohexyl]benzonitril in 200 ml Tetrahydrofuran/2N Salzsäure (Vol. 4:1) wurde 1 Stunde zum Rückfluss erhitzt. Anschliessend wurde das Reaktionsgemisch auf 200 ml Wasser gegossen und dreimal mit je 150 ml Diäthyläther extrahiert. Die organischen Phasen wurden zweimal mit je 100 ml Wasser gewaschen, über Magnesiumsulfat getrocknet und eingeengt, wobei 9,8 g [trans-4-(p-Cyanophenyl)cyclohexyl]acetaldehyd als leicht gelbliche Kristalle (Reinheit 98,7%) erhalten wurden. Umkristallisation einer Probe aus Hexan/t-Butylmethyläther (Vol. 1:1) ergab reinen Aldehyd mit Smp. 43,4°C.

e) Eine Suspension von 4,5 g Methoxymethyl-triphenylphosphoniumchlorid in 40 ml t-Butylmethyläther wurde unter Argonbegasung bei −10°C innert 3 Minuten mit 1,5 g Kalium-t-butylat versetzt und noch 1 Stunde bei 0—5°C gerührt. Dann wurde die Suspension bei 0°C innert 5 Minuten tropfenweise mit einer Lösung von 2,0 g [trans-4-(p-Cyanophenyl)cyclohexyl]acetaldehyd in 20 ml t-Butylmethyläther versetzt und noch 2 Stunden bei Raumtemperatur gerührt. Anschliessend wurde das Reaktionsgemisch auf 100 ml Wasser gegossen und dreimal mit je 100 ml Diäthyläther extrahiert. Die organischen Phasen wurden zweimal mit je 100 ml Wasser gewaschen, über Magnesiumsulfat getrocknet und eingeengt. Das resultierende, zähflüssige Oel wurde in 15 ml Aethylacetat gelöst und die klare Lösung mit 250 ml Petroläther versetzt. Nach 10 Minuten Stehenlassen bei −20°C wurde das ausgefallene Triphenylphosphinoxid abfiltriert und das Filtrat zur Trockene eingeengt. Niederdruckchromatographie (0,5 bar) des Rückstandes (3,3 g) an Kieselgel mit Aethylacetat/Petroläther (Vol. 5:95) ergab 2,04 g (91%) p-[trans-4-(3-Methoxy-2-propenyl)cyclohexyl]benzonitril als farbloses Oel; Rf-Wert (Aethylacetat/Petroläther Vol. 10:90) 0,33.

f) Eine Lösung von 2,04 g p-[trans-4-(3-Methoxy-2-propenyl)cyclohexyl]benzonitril in 100 ml Tetrahydrofuran/2N-Salzsäure (Vol. 4:1) wurde 1 Stunde zum Rückflüss erhitzt. Anschliessend wurde das

Reaktionsgemisch auf 100 ml Wasser gegossen und dreimal mit je 100 ml Diäthyläther extrahiert. Die organischen Phasen wurden zweimal mit je 100 ml Wasser gewaschen, über Magnesiumsulfat getrocknet und eingeengt, wobei 1,9 g (99%) 3-[trans-4-(p-Cyanophenyl)cyclohexyl]propionaldehyd als farblose Kristalle erhalten wurden; Rf-Wert (Aethylacetat/Petroläther Vol. 10:90) 0,15.

g) Eine Suspension von 6,4 g Methoxymethyl-triphenylphosphoniumchlorid in 80 ml t-Butylmethyläther wurde unter Argonbegasung bei 0°C innert 3 Minuten mit 2,1 g Kalium-t-butylat versetzt und noch 1 Stunden bei 0—5°C gerührt. Dann wurde das Gemisch bei 0°C innert 5 Minuten tropfenweise mit einer Lösung von 3,0 g 3-[trans-4-(p-Cyanophenyl)cyclohexyl]propionaldehyd in 20 ml t-Butylmethyläther versetzt und noch 2 Stunden bei Raumtemperatur gerührt. Anschliessend wurde das Reaktionsgemisch auf 100 ml Wasser gegossen und dreimal mit je 100 ml Diäthyläther extrahiert. Die organischen Phasen wurden zweimal mit je 100 ml Wasser gewaschen, über Magnesiumsulfat getrocknet und eingeengt. Der Rückstand wurde in 15 ml Aethylacetat gelöst und die klare Lösung mit 250 ml Petroläther versetzt. Nach 10 Minuten Stehenlassen bei −20°C wurde das ausgefallene Triphenylphosphinoxid abfiltriert und das Filtrat zur Trockene eingeengt. Niederdruckchromatographie (0,5 bar) des Rückstandes (5,5 g) an Kieselgel mit Aethylacetat/Petroläther (Vol. 5:95) ergab 2,95 (88%) p-[trans-4-(4-Methoxy-3-butenyl)cyclohexyl]benzonitril als farblose Kristalle.

h) Eine Lösung von 2,95 g p-[trans-4-(4-Methoxy-3-butenyl)cyclohexyl]benzonitril in 100 ml Tetrahydrofuran/2N Salzsäure (Vol. 4:1) wurde 15 Minuten zum Rückfluss erhitzt. Anschliessend wurde das Reaktionsgemisch auf 100 ml Wasser gegossen und dreimal mit je 100 ml Diäthyläther extrahiert. Die organische Phasen wurden zweimal mit je 100 ml Wasser gewaschen, über Magnesiumsulfat getrocknet und eingeengt, wobei 2,6 g (93%) 4-[trans-4-(p-Cyanophenyl)cyclohexyl]butyraldehyd als leicht gelbliches Oel erhalten wurde; Rf-Wert (Toluol/Aethylacetat Vol. 95:5) 0,23.

In analoger Weise wurden folgende Verbindungen hergestellt:

4-Cyanocyclohexancarboxaldehyd,
(4-Cyanocyclohexyl)acetaldehyd,
3-(4-Cyanocyclohexyl)propionaldehyd,
4-(4-Cyanocyclohexyl)butyraldehyd,
trans-4-(4'-Cyano-4-biphenyl)cyclohexancarboxaldehyd,
[trans-4-(4'-Cyano-4-biphenylyl)cyclohexyl]acetaldehyd,
3-[trans-4-(4'-Cyano-4-biphenylyl)cyclohexyl]propionaldehyd,
4-[trans-4-(4'-Cyano-4-biphenylyl)cyclohexyl]butyraldehyd.

Beispiel 6

a) 10,0 g 4'-Brom-4-biphenylcarboxaldehyd und 5,31 g Kupfer-(I)-cyanid wurden unter Argonbegasung in 80 ml Dimethylformamid gelöst und die Lösung bei 180°C Badtemperatur 15 Stunden zum Rückfluss erhitzt. Danach wurde das Reaktionsgemisch vorsichtig aus 200 ml 25-proz. Ammoniak gegossen und dreimal mit je 200 ml Methylenchlorid extrahiert. Die organischen Phasen wurden einmal mit 200 ml 25-proz. Ammoniak und zweimal mit je 200 ml Wasser gewaschen, über Magnesiumsulfat getrocknet und eingeengt. Niederdruckchromatographie (0,5 bar) des gelblichen, kristallinen Rückstandes (8,7 g) an Kieselgel ergab 5,85 g (74%) 4'-Cyano-4-biphenylcarboxaldehyd als leicht gelbliche Kristalle; Rf-Wert (Toluol/Aethylacetat Vol. 90:10) 0,27.

b) Eine Suspension von 12,4 g Methoxymethyl-triphenylphosphoniumchlorid in 120 ml t-Butylmethyläther wurde unter Argonbegasung bei 0°C innert 3 Minuten mit 4,1 g Kalium-t-butylat versetzt und noch 1 Stunde bei 0°C gerührt. Danach wurde das Gemisch innert 10 Minuten mit einer Lösung von 5,0 g 4'-Cyano-4-biphenylcarboxaldehyd in 40 ml Tetrahydrofuran versetzt und noch 1,5 Stunden bei Raumtemperatur gerührt. Anschliessend wurde das Reaktionsgemisch auf 200 ml Wasser gegossen und dreimal mit je 150 ml Diäthyläther extrahiert. Die organischen Phasen wurden zweimal mit je 150 ml Wasser gewaschen, über Magnesiumsulfat getrocknet und eingeengt. Der erhaltene Rückstand wurde in 25 ml Aethylacetat gelöst und mit 350 ml Petroläther versetzt. Nach 10 Minuten Stehenlassen bei −20°C wurde das ausgefallene Triphenylphosphinoxid abfiltriert und das Filtrat zur Trockene eingeengt. Niederdruckchromatographie (0,5 bar) des dunkelbraunen Oels (9,5 g) an Kieselgel mit Aethylacetat/Petroläther (Vol. 10:90) ergab 5,2 g (91%) 4'-(2-Methoxyvinyl)-4-biphenylcarbonitril als gelbliche Kristalle; Rf-Wert (Aethylacetat/Petroläther Vol. 10:90) 0,17.

c) Ein Gemisch von 4,9 g 4'-(2-Methoxyvinyl)-4-biphenylcarbonitril und 80 ml Eisessig/Wasser (Vol. 2:1) wurde bei 100°C Badtemperatur 1,5 Stunden zum Rückfluss erhitzt. Anschliessend wurde das Reaktionsgemisch auf 150 ml Wasser gegossen und dreimal mit je 100 ml Methylenchlorid extrahiert. Die organischen Phasen wurden mit 100 ml Wasser und mit 100 ml gesättigter Natriumhydrogencarbonat-Lösung gewaschen, über Magnesiumsulfat getrocknet und eingeengt. Es resultierten 4,8 g (4'-Cyano-4-biphenylyl)acetaldehyd als gelblicher, kristalliner Rückstand, welcher ohne zusätzliche Reinigung weiterverwendet wurde; Rf-Wert (Toluol/Aethylacetat Vol. 90:10) 0,37.

d) (4'-Cyano-4-biphenylyl)acetaldehyd wurde in analoger Weise zu Absatz b) zu 4'-(3-Methoxy-2-propenyl)-4-biphenylcarbonitril umgesetzt; Rf-Wert (Aethylacetat/Petroläther Vol. 10:90) 0,20.

e) 4'-(3-Methoxy-2-propenyl)-4-biphenylcarbonitril wurde in analoger Weise zu Absatz c) zum 3-(4'-Cyano-4-biphenylyl)propionaldehyd umgesetzt; Rf-Wert (Toluol/Aethylacetat Vol. 95:5) 0,34.

Beispiel 7

Eine Suspension von 7,34 g Methyl-triphenylphosphoniumbromid in 90 ml t-Butylmethyläther wurde unter Argonbegasung bei 0°C innert 2 Minuten mit 2,3 g Kalium-t-butylat versetzt und noch 1 Stunde bei Raumtemperatur gerührt. Danach wurde das Gemisch innert 5 Minuten tropfenweise mit einer Lösung von 3,5 g 4-[trans-4-(p-Cyanophenyl)cyclohexyl]butyraldehyd in 20 ml t-Butylmethyläther versetzt und noch 20 Stunden bei Raumtemperatur gerührt. Anschliessend wurde das Reaktionsgemisch auf 100 ml Wasser gegossen und dreimal mit je 100 ml Diäthyläther extrahiert. Die organischen Phasen wurden zweimal mit je 100 ml Wasser gewaschen, über Magnesiumsulfat getrocknet und eingeengt. Der Rückstand wurde in 15 ml Aethylacetat gelöst und die klare Lösung mit 250 ml Petroläther versetzt. Nach 10 Minuten Stehenlassen bei −20°C wurde das ausgefallene Triphenylphosphinoxid abfiltriert und das Filtrat zur Trockene eingeengt. Niederdruckchromatographie (0,5 bar) des öligen Rückstandes (4,8 g) an Kieselgel mit Aethylacetat/Petroläther (Vol. 5:95) ergab 3,1 g (89%) gelbliches Oel. Umkristallisation bei −20°C aus Methanol ergab schliesslich 2,76 g (80%) p-[trans-4-(4-Pentenyl)cyclohexyl)benzonitril als farblose Kristalle; Smp. (C—I) 29,8°C, Klp. (N—I) 10,2°C.

In analoger Weise wurden folgende Verbindungen hergestellt:

p-(trans-4-Vinylcyclohexyl)benzonitril; Smp. (C—I) 56,4°C, Klp. (N—I) 28,5°C,
p-(trans-4-Allylcyclohexyl)benzonitril; Smp. (C—I) 29,2°C,
p-[trans-4-(3-Butenyl)cyclohexyl]benzonitril; Smp. (C—N) 49,5°C, Klp. (N—I) 52,5°C,
4′-[trans-4-(3-Butenyl)cyclohexyl]-4-biphenylcarbonitril; Smp. (C—N) 119,2°C, Klp. (N—I) 232,7°C,
4′-[trans-4-(4-Pentenyl)cyclohexyl]-4-biphenylcarbonitril; Smp. (C—N) 77,42°C, Klp. (N—I) 200,8°C,
4′-(3-Butenyl)-4-biphenylcarbonitril; Smp. (C—I) 69,3°C, Klp. (N—I) 45°C,
4-(3-Butenyl)cyclohexancarbonitril.

Beispiel 8

a) In einem Sulfierkolben mit Thermometer, mechanischem Rührer, Tropftrichter und Festsubstanzzugaberohr wurde unter Argonbegasung eine Suspension von 3,6 g Butyltriphenylphosphoniumbromid in 40 ml t-Butylmethyläther bei Raumtemperatur vorgelegt, mit 1,01 g Kalium-t-butylat versetzt und noch 1 Stunde bei Raumtemperatur gerührt. Anschliessend wurde das teiforange, heterogene Reaktionsgemisch auf −60°C abgekühlt und innert 15 Minuten mit einer Lösung von 1,28 g trans-4-(p-Cyanophenyl)cyclohexancarboxaldehyd in 10 ml t-Butylmethyläther versetzt. Das Reaktionsgemisch wurde noch 60 Minuten unter langsamen Erwärmen auf −30°C gerührt, dann auf 100 ml Wasser gegossen und dreimal mit je 50 ml Diäthyläther extrahiert. Die organischen Phasen wurden einmal mit 50 ml Wasser gewaschen, über Magnesiumsulfat getrocknet und eingeengt. Niederdruckchromatographie (0,5 bar) des Rückstandes (3,45 g) an Kieselgel mit Aethylacetat/Petroläther (Vol. 3:97) ergab 1,52 g (99%) p-[trans-4-(1-Pentenyl)cyclohexyl]benzonitril (1E-Pentenyl/1Z-Pentenyl-Verhältnis ca. 5:95) als farbloses Oel; Rf-Wert (Aethylacetat/Petroläther Vol. 3:97) 0.19.

b) In einem Sulfierkolben mit Thermometer, Tropftrichter und mechanischem Rührer wurde unter Argonbegasung ein Gemisch von 1,51 g 90-proz. m-Chlorperbenzoesäure und 3,0 g gemahlenem Kaliumcarbonat in 60 ml Methylenchlorid bei 0°C vorgelegt und innert 15 Minuten mit einer Lösung von 2,0 g p-[trans-4-(1-Pentenyl)cyclohexyl]benzonitril (1E-Pentenyl/1Z-Pentenyl-Verhältnis ca. 5:95) in 20 ml Methylenchlorid versetzt. Anschliessend wurde das Kühlbad entfernt und das Reaktionsgemisch nach insgesamt 75 Minuten und 105 Minuten nochmals mit je 0,75 g 90-proz. m-Chlorperbenzoesäure versetzt. Das Reaktionsgemisch wurde noch 60 Minuten bei Raumtemperatur gerührt, dann auf 50 ml 10-proz. (Gew./Vol.) Natriumthiosulfat-Lösung gegossen und dreimal mit je 100 ml Methylenchlorid extrahiert. Die organischen Phasen wurden mit 50 ml gesättigter Natriumhydrogencarbonat-Lösung gewaschen, über Magnesiumsulfat getrocknet und eingeengt. Hierbei wurden 2,1 g (98%) p-[trans-4-(1,2-Epoxypentyl)-cyclohexyl]benzonitril (trans-1,2-Epoxypentyl/cis-1,2-Epoxypentyl-Verhältnis ca. 5:95) als farbloses Oel erhalten; Rf-Werte (Aethylacetat/Petroläther Vol. 10:90) trans-1,2-Epoxypentyl-Isomer 0,17, cis-1,2-Epoxypentyl-Isomer 0,14.

c) In einem Rundkolben mit Tropftrichter wurde unter Argonbegasung eine Lösung von 2,46 g Triphenylphosphin in 30 ml Methylenchlorid bei 0°C vorgelegt und tropfenweise solange mit einer ca. 1M Lösung von Brom in Methylenchlorid versetzt, bis eine schwach gelbe Farbe bestehen blieb. Anschliessend wurde die Lösung am Rotationsverdampfer vorsichtig eingeengt und am Hochvakuum nachgetrocknet. Der resultierende, kristalline Rückstand wurde in 30 ml Benzol aufgeschlämmt, mit einer Lösung von 2,1 g p-[trans-4-(1,2-Epoxypentyl)cyclohexyl]benzonitril in 10 ml Benzol versetzt und 3 Stunden zum Rückfluss erhitzt. Filtration der warmen Reaktionslösung an Kieselgel mit Toluol ergab 3,0 g kristallines Rohprodukt, welches nach Niederdruckchromatographie (0,5 bar) an Kieselgel mit Hexan/Toluol (Vol. 1:1) 2,61% g (81%) fast reines p-[trans-4-(erythro-1,2-Dibrompentyl)cyclohexyl]benzonitril als farblose Kristalle liefert. Durch Umkristallisation aus 90 ml Petroläther/Aethylacetat (Vol. 2:1) wurden schliesslich 2,09 g (65%) sehr reines erythro-Dibromid erhalten; Smp. 140,9°C.

d) In einem Sulfierkolben mit mechanischem Rührer und Thermometer wurde unter Argonbegasung eine Gemisch von 2,75 g p-[trans-4-(erythro-1,2-Dibrompentyl)cyclohexyl]benzonitril und 20 ml Eisessig bei Raumtemperatur mit 2,42 g Zinkpulver versetzt und dann 2 Stunden gerührt, wobei sich das Reaktionsgemisch auf 33°C erwärmte und das Edukt allmählich in Lösung ging. Anschliessend wurde das Reaktionsgemisch auf 100 ml Wasser gegossen und dreimal mit je 100 ml Petroläther extrahiert. Die

organischen Phasen wurden zweimal mit je 100 ml Wasser und einmal mit 50 ml gesättigter Natriumhydrogencarbonat-Lösung gewaschen, über Magnesiumsulfat getrocknet und eingeengt. Hierbei wurden 1,43 g (99%) p-[trans-4-(1E-Pentenyl)cyclohexyl]benzonitril in einer Reinheit von 99,5% erhalten; Smp. (C—N) 15,6°C, Klp. (N—I) 58,5°C.

In analoger Weise wurden folgende Verbindungen hergestellt:
p-[trans-4-(1E-Propenyl)cyclohexyl]benzonitril; Smp. (C—N) 66,3°C, Klp. (N—I) 73,0°C,
p-[trans-4-(1E-Butenyl)cyclohexyl]benzonitril; Smp. (C—N) 45,1°C, Klp. (N—I) 51,8°C,
p-[trans-4-(1E-Hexenyl)cyclohexyl]benzonitril; Smp. (C—N) 14,4°C, Klp. (N—I) 39,2°C,
p-[trans-4-(1E-Heptenyl)cyclohexyl]benzonitril; Smp. (C—N) 17,9°C, Klp. (N—I) 49,2°C,
p-[trans-4-(3E-Pentenyl)cyclohexyl]benzonitril; Smp. (C—N) 59,8°C, Klp. 73,7°C,
p-[trans-4-(3E-Hexenyl)cyclohexyl]benzonitril; Smp. (C—N) 31,1°C, Klp. (N—I) 50,2°C,
p-[trans-4-(3E-Heptenyl)cyclohexyl]benzonitril; Smp. (C—N) 15,4°C, Klp. (N—I) 48,3°C,
4'-(3E-Propenyl)-4-biphenylcarbonitril; Smp. (C—I) 76,8°C, Klp. (N—I) 72,6°C,
4'-[trans-4-(1E-Pentenyl)cyclohexyl]-4-biphenylcarbonitril; Smp. (C—N) 125,9°C, Klp. (N—I) 253,5°C,
4'-[trans-4-(3E-Pentenyl)cyclohexyl]-4-biphenylcarbonitril; Smp. (C—N) 124,1°C, Klp. 242,6°C,
4-(3E-Pentenyl)cyclohexancarbonitril.

Beispiel 9

In einem Rundkolben mit Magnetrührer und Rückflusskühler wurde unter Argonbegasung ein Gemisch von 3,79 g p-[trans-4-(1-Hexenyl)cyclohexyl)benzonitril (hergestellt gemäss Beispiel 8a; 1E-Hexenyl/1Z-Hexenyl-Verhältnis ca. 5:95) und 758 mg Benzolsulfinsäure in 50 ml 1,4-Dioxan 15 Stunden unter Rückfluss gekocht. Anschliessend wurde nochmals 379 mg Benzolsulfinsäure zugegeben und das Gemisch weitere 4 Stunden zum Rückfluss erhitzt. Dann wurde das abgekühlte Reaktionsgemisch auf 50 ml 1N Natronlauge gegossen und dreimal mit je 100 ml Hexan extrahiert. Die organischen Phasen wurden zweimal mit je 50 ml Wasser gewaschen, über Magnesiumsulfat getrocknet und eingeengt. Dreimalige Kristillisation des quantitativ zurückerhaltenen, äquilibrierten Olefingemisches (1E-Hexenyl(1Z-Verhältnis 80,4:19,6) aus Methanol ergab schliesslich 1,74 g (46%) p-[trans-4-(1E-Hexenyl)cyclohexyl]benzonitril (enthaltend 0,3% 1Z-Hexenyl-Isomer) mit Smp. (C—N) 14,3°C und Klp. (N—I) 39,5°C. die Mutterlaugen wurden nicht aufgearbeitet. Gewünschtenfalls können diese aber wiederum äquilibriert und kristallisiert werden.

In analoger Weise können alle in Beispiel 8 hergestellten Nitrile erhalten werden.

Beispiel 10

a) Eine Suspension von 5,1 g Propyl-triphenylphosphoniumbromid in 80 ml t-Butylmethyläther wurde unter Argonbegasung bei −10°C innert 5 Minuten mit 1,48 g Kalium-t-butylat versetzt und noch 60 Minuten bei Raumtemperatur gerührt. Danach wurde das Gemisch bei 0°C innert 5 Minuten mit einer Lösung von 2,0 g [trans-4-(p-Cyanophenyl)cyclohexyl]acetaldehyd in 25 ml t-Butylmethyläther versetzt und noch 45 Minuten bei Raumtemperatur gerührt. Anschliessend wurde das Reaktionsgemisch auf 100 ml Wasser gegossen und dreimal mit je 100 ml Diäthyläther extrahiert. Die organischen Phasen wurden zweimal mit je 100 ml Wasser gewaschen, über Magnesiumsulfat getrocknet und eingeengt. Der Rückstand wurde in 20 ml Aethylacetat gelöst und mit 250 ml Petroläther versetzt. Nach 10 Minuten Stehenlassen bei −20°C wurde das ausgefallene Triphenylphosphinoxid abfiltriert und das Filtrat zur Trockene eingeengt. Niederdruckchromatographie (0,5 bar) des gelblichen, öligen Rückstandes (3,33 g) an Kieselgel mit Aethylacetat/Petroläther (Vol. 3:97) ergab 2,1 g p-[trans-4-(2-Pentenyl)cyclohexyl]benzonitril als farbloses Oel, enthaltend 92,7% p-[trans-4-(2Z-Pentenyl)cyclohexyl]benzonitril und 6,6% p-[trans-4-(2E-Pentenyl)cyclohexyl]benzonitril. Dieses Material wurde ohne zusätzliche Reinigung weiter umgesetzt. Gewünschtenfalls kann aber dieses Isomerengemisch durch Chromatographie an mit Silbernitrat beschichtem Kieselgel getrennt werden (wie in Absatz d illustriert).

b) Eine Lösung von 1,59 g 90-proz. m-Chlorperbenzoesäure in 60 ml Methylenchlorid wurde mit 4,0 g gemahlenem Kaliumcarbonat versetzt. Die erhaltene Suspension wurde bei 0°C innert 5 Minuten tropfenweise mit einer Lösung von 2,1 g p-[trans-4-(2-Pentenyl)cyclohexyl]benzonitril (enthaltend 92.7% 2Z-Isomer und 6,6% 2E-Isomer) in 20 ml Methylenchlorid versetzt und dann noch 3 Stunden bei Raumtemperatur gerührt, wobei nach 1 Stunde und nach 2 Stunden nochmals je 0,8 g m-Chlorperbenzoesäure zugegeben wurden. Anschliessend wurde das Reaktionsgemisch auf 150 ml 10-proz. Natriumthiosulfat-Lösung gegossen und dreimal mit je 100 ml Methylenchlorid extrahiert. Die organischen Phasen wurden mit 100 ml 10-proz. Natriumthiosulfat-Lösung und mit 100 ml gesättigter Natriumcarbonat-Lösung gewaschen, über Magnesiumsulfat getrocknet und eingeengt. Dies ergab 2,2 g (99%) p-[trans-4-(2,3-Epoxypentyl)cyclohexyl]benzonitril als farblose Kristalle; Rf-Wert (Aethylacetat/Petroläther Vol. 10:90) 0,15. Dieses Material wurde ohne zusätzliche Reinigung weiterverwendet.

c) Eine Lösung von 2,6 g Triphenylphosphin in 30 ml Methylenchlorid wurde bei 0°C tropfenweise mit einer Lösung von 0,519 ml Brom in 20 ml Methylenchlorid versetzt, bis eine leichte Gelbfärbung bestehen blieb. Die erhaltene gelbe Suspension wurde am Rotationsverdampfer vorsichtig zur Trockene eingeengt und der gelbliche, kristalline Rückstand am Hochvakuum (0,5 Torr) bei Raumtemperatur 1 Stunde getrocknet. Das erhaltene Triphenylphosphin-Brom wurde in 50 ml Toluol aufgeschlämmt und am Rotationsverdampfer nochmals zur Trockene eingeengt. Danach wurde der Rückstand in 30 ml Toluol

aufgeschlämmt, mit einer Lösung von 2,2 g p-[trans-4-(2,3-Epoxypentyl)cyclohexyl]benzonitril in 10 ml Toluol versetzt und das Gemisch 2 Stunden bei 80°C Badtemperatur gerührt. Niederdruckchromatographie (0,5 bar) des abgekühlten Reaktionsgemisches an Kieselgel mit Toluol ergab 2,83 g (84%) p-[trans-4-(2,3-Dibrompentyl)cyclohexyl]benzonitril als gelbliches Oel (enthaltend 66.6% erythro-Form und 32,8% threo-Form); Rf-Wert (Aethylacetat/Petroläther Vol. 10:90) 0,33. Dieses Material wurde ohne zusätzliche Reinigung weiterverwendet.

d) Eine Lösung von 1,98 g p-[trans-4-(2,3-Dibromopentyl)cyclohexyl]benzonitril (erythro/threo 66,6:32,8) in 30 ml Eisessig wurde bei Raumtemperatur unter Argonbegasung mit 2.0 g Zinkpulver versetzt und die Suspension 1 Stunde bei Raumtemperatur gerührt. Anschliessend wurde das Reaktionsgemisch auf 100 ml Wasser gegossen und dreimal mit je 100 ml Petroläther extrahiert. Die organischen Phasen wurden zweimal mit je 100 ml Wasser gewaschen, über Magnesiumsulfat getrocknet und eingeengt. Niederdruckchromatographie (0,5 bar) des gelblichen Rückstandes (1,16 g) an mit Silbernitrat belegtem Kieselgel mit Hexan/Diäthyläther (Vol. 90:10) ergab 586 mg (48%) p-[trans-4-(2E-Pentenyl)cyclohexyl]benzonitril und 171 mg (14%) p-[trans-4-(2Z-Pentenyl)cyclohexyl]benzonitril als farblose Oele. Das 2E-Isomer hat einen Schmelzpunkt von 9,6°C bzw. 16,1°C (2 Modifikationen) und einen virtuellen Klp. von −67°C; das 2Z-Isomer hat einen Schmelzpunkt von −7,7°C und einen virtuellen Klp. von −54°C.

Die Beschichtung von Kieselgel bzw. Dünnschichtplatten mit Silbernitrat wurde wie folgt durchgeführt:

34 g Silbernitrat wurde in 1000 ml Acetonitril gelöst. Die Dünnschichtplatten wurden einmal kurz in die Silbernitrat-Lösung eingetaucht und danach 2 Stunden bei 80°C im Vakuum (12 Torr) getrocknet. Dann wurden 300 g Kieselgel in die restliche Silbernitrat-Lösung gegeben, gut vermengt, am Rotationsverdampfer vorsichtig zur Trockene eingeengt und bei Raumtemperatur am Hochvakuum (0,5 Torr) 2 Stunden getrocknet.

## Beispiel 11

a) In einem Sulfierkolben wurden unter Rühren und Stickstoffbegasung 149 g Methoxymethyl-triphenylphosphoniumchlorid und 860 ml t-Butylmethyläther bei Raumtemperatur vorgeleft, die Suspension auf −10°C gekühlt und innert 10 Minuten mit 51,6 g Kalium-t-butylat versetzt. Die Suspension wurde noch 30 Minuten bei −10°C bis 0°C gerührt und dann innert 45 Minuten bei 0°C tropfenweise mit einer Lösung von 47,3 g 4,4-Aethylendioxycyclohexanon in 720 ml Tetrahydrofuran versetzt. Die orange Suspension wurde noch 2 Stunden bei Raumtemperatur weiter gerührt, dann auf 5 l Hexan gegossen, 10 Minuten gerührt und genutscht. Das Filtrat wurde im Vakuum eingeengt und das erhaltene gelb-bräunliche Oel (104,1 g) mit 500 ml Hexan versetzt und genutscht. Das Filtrat wurde im Vakuum eingeengt, wobei 61,7 g gelbbraünliches Oel erhalten wurden. Chromatographische Trennung dieses Rohproduktes an Kieselgel mit Methylenchlorid/Aceton (Vol. 98:2 und 95:5) ergab schliesslich 53,5 g 1,1-Aethylendioxy-4-(methoxymethylen)cyclohexan als farbloses Oel.

b) In einem Rundkolben wurde unter Stickstoffbegasung ein Gemisch von 28,2 g 1,1-Aethylendioxy-4-(methoxymethylen)cyclohexan, 770 ml Eisessig und 385 ml Wasser 1 Stunde zum Rückfluss erhitzt. Danach wurde die gelbliche klare Lösung auf Raumtemperatur gekühlt, mit 800 ml Wasser verdünnt und dreimal mit je 700 ml Methylenchlorid extrahiert. Die organischen Phasen wurden zweimal mit je 500 ml 10-proz. (Gew./Vol.) Natriumcarbonat-Lösung gewaschen, über Natriumsulfat getrocknet, filtriert und eingeengt. Chromatographische Trennung der erhaltenen bräunlichen Flüssigkeit (18,5 g) an Kieselgel mit Methylenchlorid als Eluens ergab schliesslich 16,7 g 4-Formylcyclohexanon als bräunliche Flüssigkeit.

c) In einem Sulfierkolben wurden unter Rühren und Stickstoffbegasung 63,3 g p-Cyanobenzyltriphenylphosphoniumchlorid, 17,2 g Kalium-t-butylat und 195 ml Aethylenglykoldimethyläther vorgelegt, wobei die Innentemperatur bis auf 44°C anstieg. Die braune Suspension wurde auf 0°C gekühlt und innert 2 Minuten mit einer Lösung von 16,7 g 4-Formylcyclohexanon in 100 ml Aethylglykoldimethyläther versetzt. Danach wurde das Kühlbad entfernt und das Reaktionsgemisch noch 3,5 Stunden bei Raumtemperatur gerührt. Anschliessend wurde die Suspension auf 500 ml Wasser gegossen und dreimal mit je 600 ml Methylenchlorid extrahiert. Die organischen Phasen wurden zweimal mit je 500 ml 10-proz. (Gew./Vol.) Kochsalzlösung gewaschen, über Natriumsulfat getrocknet, filtriert und eingeengt, wobei 76,9 g einer bräunlichen Paste zurückblieben. Chromatographische Trennung dieses Rohproduktes an Kieselgel mit Methylenchlorid als Eluens ergab 33,0 g 4-[2-(p-Cyanophenyl)vinyl]cyclohexanon als gelbbräunliches Oel.

d) In einem Rundkolben mit Magnetrührer wurde ein Gemisch von 33,0 g 4-[2-(p-Cyanophenyl)vinyl)cyclohexanon, 520 ml Toluol, 260 ml Aethanol und 3,2 g 5-proz. Palladium/Kohle bei Raumtemperatur vorgelegt und das Gemisch bis zum Stillstand der Wasserstoffaufnahme hydriert. Anschliessend wurde die schwarze Suspension genutscht (Nachwaschen mit Toluol) und das Filtrat im Vakuum eingeengt. Das erhaltene, leicht trübe, gelbliche Oel (34,1 g) wurde an Kieselgel chromatographisch gereinigt. Methylenchlorid/Hexan (Vol. 1:1), Methylenchlorid/Hexan (Vol. 8:2) und Methylenchlorid eluierten 25,6 g gelbliches Oel, welches aus t-Butylmethyläther kristallisiert wurde. Hierbei wurden 22,6 g 4-[2-(p-Cyanophenyl)äthyl]cyclohexanon als farblose Kristalle mit Schmelzpunkt 62.5—64.3°C erhalten.

e) In analoger Weise zu den Beispielen 5 und 8 wurde 4-[2-(p-Cyanophenyl)äthyl]cyclohexanon zu den folgenden Verbindungen weiter umgesetzt:

p-[2-(trans-4-(1E-Propenyl)cyclohexyl)äthyl]benzonitril: Smp. (C—I) 61,3°C, Klp. (N—I) 54,2°C,

p-[2-(trans-4-(1E-Butenyl)cyclohexyl)äthyl]benzonitril: Smp. (C—I) 42,6°C, Klp. (N—I) 39,7°C,

p-[2-(trans-4-(1E-Pentenyl)cyclohexyl)äthyl]benzonitril: Smp. (C—N) 25,1°C, Klp. (N—I) 47,5°C,

p-[2-(trans-4-(1E-Hexenyl)cyclohexyl)äthyl]benzonitril: Smp. (C—N) 16,8°C, 19,7°C (2 Modifikationen), Klp. (N—I) 34,6°C,

p-[2-(trans-4-(1E-Heptenyl)cyclohexyl)äthyl]benzonitril: Smp. (C—N) 31,6°C, Klp. (N—I) 43,6°C.

## Beispiel 12

1,1 g p-[trans-4-(1E-Pentenyl)cyclohexyl]benzonitril wurden unter Argonbegasung in 17,5 ml einer 10-proz. Lösung von Kaliumhydroxid in Diäthyläther 16 Stunden bei 180°C gekocht. Nach Abkühlen auf Raumtemperatur wurde des dunkelbraune Reaktionsgemisch mit 20 ml Wasser verdünnt, mit 25-proz. Salzsäure sauer gestellt und viermal mit je 100 ml Methylenchlorid extrahiert. Die organischen Phasen wurden zweimal mit je 80 ml Wasser gewaschen, über Magnesiumsulfat und Aktivkohle getrocknet und eingeengt. Es resultierten 1,16 g (98%) p-[trans-4-(1E-Pentenyl)cyclohexyl]benzoesäure als beige Kristalle; Rf-Wert (Toluol/Aethylacetat Vol. 3:1) 0,45.

In analoger Weise wurden folgende Verbindungen hergestellt:

trans-4-(3-Butenyl)cyclohexancarbonsäure,

trans-4-(3E-Pentenyl)cyclohexancarbonsäure,

p-[trans-4-(1E-Hexenyl)cyclohexyl]benzoesäure,

p-[trans-4-(3-Butenyl)cyclohexyl]benzoesäure,

p-[trans-4-(3E-Pentenyl)cyclohexyl]benzoesäure.

## Beispiel 13

a) 20 ml einer 0,462M Lösung von Methylmagnesiumjodid in Diäthyläther wurden unter Argonbegasung bei 40°C innert 5 Minuten mit einer Lösung von 1,7 g p-[trans-4-(erythro-1,2-Dibromopentyl)cyclohexyl]benzonitril in 20 ml Toluol versetzt. Danach wurde durch Erwärmen unter starken Argonstrom soviel Diäthyläther abdestilliert, dass die Innentemperatur auf 50°C anstieg, und dann unter Verwendung eines Rückflusskühlers des Gemisch noch 15 Stunden bei dieser Temperatur gerührt. Anschliessend wurde das Reaktionsgemisch auf 100 ml Eiswasser gegossen und dreimal mit je 100 ml Diäthyläther extrahiert. Die organischen Phasen wurden zweimal mit je 100 ml Wasser gewaschen, über Magnesiumsulfat getrocknet und eingeengt. Niederdruckchromatographie (0,5 bar) des gelblichen, kristallinen Rückstandes (1,73 g) an Kieselgel mit Aethylacetat/Petroläther (Vol. 10:90) ergab 1,31 g (74%) p-[trans-4-(erythro-1,2-Dibrompentyl)cyclohexyl]acetophenon als farblose Kristalle, welche ohne zusätzliche Reinigung weiter umgesetzt wurden; Rf-Wert (Aethylacetat/Petroläther Vol. 10:90) 0,26.

b) Eine Lösung von 817 mg p-[trans-4-(erythro-1,2-Dibrompentyl)cyclohexyl]acetophenon in 40 ml Methylenchlorid wurde bei Raumtemperatur unter Argonbegasung mit 233 mg Natriumhydrogencarbonat und 392 mg 90-proz. m-Chlorperbenzoesäure versetzt und gerührt, wobei jeweils im Abstand von 2,5 Stunden weitere 233 mg Natriumhydrogencarbonat und 392 mg 90-proz. m-Chlorperbenzoesäure zugegeben wurden. Nach insgesamt 70 Stunden Rühren und Zugabe wurde das Reaktionsgemisch auf 80 ml 10-proz. Natriumthiosulfat-Lösung gegossen und dreimal mit je 60 ml Methylenchlorid extrahiert. Die organischen Phasen wurden zweimal mit je 60 ml 10-proz. Natriumthiosulfat-Lösung gewaschen, über Magnesiumsulfat getrocknet und eingeengt. Niederdruckchromatographie (0,5 bar) des gelblichen, kristallinen Rückstandes (0,98 g) an Kieselgel mit Aethylacetat/Petroläther (Vol. 5:95) ergab 0,71 g (84%) p-[trans-4-(erythro-1,2-Dibrompentyl)cyclohexyl]phenylacetat als gelbliche Kristalle, welche ohne zusätzliche Reinigung weiter umgesetzt wurden: Rf-Wert (Aethylacetat/Petroläther Vol. 10:90) 0,28.

c) Eine Lösung von 710 mg p-[trans-4-(erythro-1,2-Dibrompentyl)cyclohexyl]phenylacetat in 40 ml Eisessig wurde bei Raumtemperatur unter Argonbegasung mit 676 mg Zinkpulver versetzt. Die graue Suspension wurde 2 Stunden bei Raumtemperatur gerührt, dann auf 100 ml Wasser gegossen und dreimal mit je 80 ml Petroläther extrahiert. Die organischen Phasen wurden zweimal mit je 80 ml Wasser gewaschen, über Magnesiumsulfat getrocknet und eingeengt. Es resultierten 385 mg (85%) p-[trans-4-(1E-Pentenyl)cyclohexyl]phenylacetat als farblose Kristalle; Rf-Wert (Aethylacetat/Petroläther Vol. 10:90) 0,35.

d) 385 mg p-[trans-4-(1E-Pentenyl)cyclohexyl]phenylacetat wurden unter Argonbegasung in 10 ml einer 1N Lösung von Kaliumhydroxid in Methanol gelöst und das Gemisch bei Raumtemperatur 30 Minuten gerührt. Anschliessend wurde das Reaktionsgemisch auf 80 ml 1N Salzsäure gegossen und dreimal mit je 60 ml Diäthyläther extrahiert. Die organischen Phasen wurden zweimal mit je 60 ml Wasser gewaschen, über Magnesiumsulfat getrocknet und eingeengt. Es resultierten 328 mg (100%) p-[trans-4-(1E-Pentenyl)cyclohexyl]phenol als farblose Kristalle; Rf-Wert (Aethylacetat/Petroläther Vol. 10:90) 0,17.

In analoger Weise wurden folgende Verbindungen hergestellt:

p-[trans-4-(1E-Propenyl)cyclohexyl]phenol,

p-[2-(trans-4-(1E-Pentenyl)cyclohexyl]äthyl]phenol.

## Beispiel 14

a) Eine Lösung von Methylmagnesiumjodid in Diäthyläther (hergestellt aus 384 mg Magnesium-Späne

und 0,984 ml Methyljodid in 30 ml Diäthyläther) wurde bei Raumtemperatur tropfenweise mit einer Lösung von 2,0 g p-[trans-4-(1E-Pentenyl)cyclohexyl]benzonitril versetzt. Das Gemisch wurde 15 Minuten zum Rückfluss erhitzt. Anschliessend wurden 30 ml Toluol zum Reaktionsgemisch zugegeben, der Diäthyläther abdestilliert und das Gemisch nochmals 1,5 Stunden zum Rückfluss erhitzt. Danach wurde das Reaktionsgemisch bei 0°C vorsichtig mit gesättigter Ammoniumchlorid-Lösung versetzt und dreimal in Diäthyläther/Wasser verteilt. Die organischen Extrakte wurden zweimal mit Wasser gewaschen, über Magnesiumsulfat getrocknet, filtriert und eingedampft. Chromatographische Trennung des gelben kristallinen Rückstandes (2,6 g) an Kieselgel mit Aethylacetat/Petroläther (Vol. 5:95) ergab 1,85 g (87%) p-[trans-4-(1E-Pentenyl)cyclohexyl]acetophenon als leicht gelbe Kristalle; Smp. 51,2°C.

b) Eine Lösung von 2,63 g p-[trans-4-(1E-Pentenyl)cyclohexyl]acetophenon in 90 ml Methylenchlorid wurde bei 0°C unter Argonbegasung nacheinander mit 7,46 g m-Chlorperbenzoesäure und 100 mg 2,6-Di-t-butyl-p-kresol versetzt. Das Gemisch wurde unter Lichtausschluss bei Raumtemperatur 40 Stunden gerührt. Anschliessend wurde das Reaktionsgemisch in Methylenchlorid/10-proz. Natriumthiosulfat-Lösung verteilt und die optische Phase einmal mit 10-proz. Natriumthiosulfat-Lösung und zweimal mit Natriumhydrogencarbonat-Lösung gewaschen. Die wässrigen Phasen wurden dreimal mit Methylenchlorid nachextrahiert. Die vereinigten organischen Phasen wurden über Magnesiumsulfat getrocknet, filtriert und eingedampft. Der gelbe, ölige Rückstand von 4-Acetoxy-1-[trans-4-(1,2-epoxypentyl)-cyclohexyl)benzol wurde in 100 ml 1N methanolischer Kaliumhydroxid-Lösung gelöst und 1 Stunde bei Raumtemperatur gerührt. Anschliessend wurde das Gemisch mit 10 ml 10-proz. Salzsäure auf ca. pH 8 gestellt und in Diäthyläther/Wasser verteilt. Die wässrige Phase wurde noch dreimal mit Diäthyläther extrahiert. Die organischen Phasen wurden über Magnesiumsulfat getrocknet, filtriert und eingedampft. Chromatographische Trennung des zurückbleibenden braunen Oeles (2,96 g) an Kieselgel mit Aethylacetat/Petroläther (Vol. 10:90) ergab 2,16 g p-[trans-4-(1,2-epoxypentyl)cyclohexyl]phenol als gelbliche Kristalle.

Beispiel 15

Ein Gemisch von 200 mg trans-4-(1E-Butenyl)cyclohexancarbonsäure, 272,4 mg Dicyclohexylcarbodiimid, 13,4 mg 4-(Dimethylamino)pyridin, 169,7 mg trans-4-(1E-Butenyl)cyclohexanol und 4 ml Methylenchlorid wurde unter Argonbegasung 21 Stunden bei Raumtemperatur gerührt. Anschliessend wurde das heterogene Reaktionsgemisch mit 10 ml Hexan verdünnt und filtriert (Nachwaschen mit Hexan). Das eingeengte Filtrat wurde in 30 ml Hexan aufgenommen und je einmal mit 30 ml 5-proz. Salzsäure, 30 ml gesättigter Natriumhydrogencarbonat-Lösung und 30 ml gesättigter Kochsalzlösung gewaschen, über Magnesiumsulfat getrocknet und eingeengt. Niederdruckchromatographie (0,5 bar) des farblosen Oeles (450 mg) an Kieselgel mit Aethylacetat/Petroläther (Vol. 1:99) ergab 280 mg (80.1%) trans-4-(1E-Butenyl)cyclohexancarbonsäure-trans-4-(1E-butenyl)cyclohexylester als farblose Kristalle. Umkristallisation aus 15 ml Methanol bei −20°C ergab 223 mg Ester als farblose Nadeln mit Schmelzpunkt (C—N) 46,3°C und Klp. (N—I) 58,7°C.

In analoger Weise wurden folgende Verbindungen hergestellt:

trans-4-(1E-Butenyl)cyclohexancarbonsäure-trans-4-(1E-pentenyl)cyclohexylester; Smp. (C—S) 28,8°C, Uebergang S—N 61,5°C, Klp. (N—I) 67,5°C,

trans-4-(1E-Pentenyl)cyclohexancarbonsäure-trans-4-(1E-butenyl)cyclohexylester; Smp. (C—S) 30,5°C, Uebergang S—N 55,2°C, Klp. (N—I) 68,0°C,

trans-4-(1E-Pentenyl)cyclohexancarbonsäure-trans-4-(1E-pentenyl)cyclohexylester; Smp. (C—S) 39,6°C, Uebergang S—N 75,4°C, Klp. (N—I) 77,1°C,

trans-4-(3-Butenyl)cyclohexancarbonsäure-trans-4-(1E-butenyl)cyclohexylester; Smp. (C—S) 26,4°C, Uebergang S—N 30,7°C, Klp. (N—I) 43,3°C,

trans-4-(3E-Pentenyl)cyclohexancarbonsäure-trans-4-(1E-butenyl)cyclohexylester; Smp. (C—S) 25,7°C, Uebergang S—N 31,8°C, Klp. (N—I) 70,0°C,

trans-4-(3E-Pentenyl)cyclohexancarbonsäure-p-[trans-4-(1E-pentenyl)cyclohexyl]phenylester; Smp. (C—S) 55,0°C, Uebergang S—N 166,5°C, Klp. (N—I) 221,5°C,

trans-4-(4-Pentenyl)cyclohexancarbonsäure-p-(4-pentenyl)phenylester,

trans-4-Pentylcyclohexancarbonsäure-p-allyloxyphenylester; Smp. (C—N) 33,7°C, Klp. (N—I) 77,9°C,

trans-4-(4-Pentenyl)cyclohexancarbonsäure-p-allyloxyphenylester; Smp. (C—N) 32,2°C, Klp. (N—I) 51,8°C,

p-[trans-4-(3E-Pentenyl)cyclohexyl]benzoesäure-p-(3E-pentenyl)phenylester; Smp. (C—S) 93,5°C, Uebergang S—S 95°C, Uebergang S—N 125°C, Klp. (N—I) 205°C.

Beispiel 16

Ein Gemisch von 400 mg p-(trans-4-Pentylcyclohexyl)phenol, 853 mg gemahlenem Kaliumcarbonat, 0,5 ml Allylbromid und 50 ml Aceton wurde über Nacht unter Argonbegasung bei Raumtemperatur gerührt. Anschliessend wurde das heterogene Reaktionsgemisch filtriert (Nachwaschen mit Hexan) und das Filtrat eingeengt. Der Rückstand wurde in 50 ml Hexan aufgenommen und die Lösung zweimal mit je 50 ml Wasser gewaschen, über Magnesiumsulfat getrocknet und eingeengt. Niederdruckchromatographie (0,5 bar) des öligen Rückstandes (490 mg) an Kieselgel mit Hexan/Diäthyläther (Vol. 90:10) ergab 435 mg (93,5%) Produkt als farblose Kristalle in einer Reinheit von 95%. Umkristallisation aus Methanol bei 0°C

ergab 155 mg 4-Allyloxy-1-(trans-4-pentylcyclohexyl)benzol als farblose Kristalle in einer Reinheit von 99,8%; Smp. (C—N) 31,9°C, Klp. (N—I) 40,9°C.

In analoger Weise können folgende Verbindungen hergestellt werden:

4-(3-Butenyl)oxy-1-(trans-4-pentylcyclohexyl)benzol; Smp. (C—I) 29,9°C, virtueller Klp. −5,0°C,

4-(2E-Butenyl)oxy-1-(trans-4-pentylcyclohexyl)benzol; Smp. (C—N) 32,0°C bzw. 38,4°C, Klp. (N—I) 66,8°C,

4-(2E-Butenyl)oxy-1-(trans-4-propylcyclohexyl)benzol; Smp. (C—N) 42,3°C, Klp. (N—I) 56,5°C,

4-(2E-Butenyl)oxy-1-[trans-4-(1E-propenyl)cyclohexyl)benzol; Smp. (C—N) 52,0°C, Klp. (N—I) 72,8°C,

4-Allyloxy-1-[2-(trans-4-pentenylcyclohexyl)äthyl]benzol; Smp. (C—$S_B$) 15,7°C, Uebergang $S_B$—N 23,8°C, Klp. (N—I) 40,7°C,

4-(2E-Butenyl)oxy-1-[2-(trans-4-(1E-propylcyclohexyl)äthyl]benzol; Smp. (C—N) 44,6°C, Klp. (N—I) 52,5°C,

4-(3-Butenyl)oxy-1-[2-(trans-4-propylcyclohexyl)äthyl]benzol; Smp. (C—S) 6,0°C, Uebergang S—N 16,3°C, Klp. (N—I) 16,5°C,

4-(3-Butenyl)oxy-1-[2-(trans-4-pentylcyclohexyl)äthyl]benzol; Smp. (C—$S_B$) 1,6°C bzw. 5,0°C (2 Modifikationen), Klp. ($S_B$—I) 33°C,

4-(4-Pentenyl)oxy-1-[2-(trans-4-propylcyclohexyl)äthyl]benzol; Smp. (C—I) 32,1°C, Klp. (N—I) 29,6°C,

4-(4-Pentenyl)oxy-1-[2-(trans-4-pentylcyclohexyl)äthyl]benzol; Smp. (C—$S_B$) 29,7°C, Klp. ($S_B$—I) 42,3°C,

4-Allyloxy-2-fluor-1-[2-(trans-4-pentylcyclohexyl)äthyl]benzol; Smp. (C—N) 17,2°C, Klp. 20,2°C,

4-(3-Butenyloxy)-2-fluor-1-[2-(trans-4-pentylcyclohexyl)äthyl]benzol; Smp. (C—I) 10,8°C, Klp. (N—I) 4,7°C.

4-(4-Pentenyl)oxy-2-fluor-1-[2-(trans-4-pentylcyclohexyl)äthyl]benzol; Smp. (C—I) 28,2°C, Klp. (N—I) 20,0°C,

4-(5-Hexenyl)oxy-2-fluor-1-[2-(trans-4-pentylcyclohexyl)äthyl]benzol; Smp. (C—N) 11,5°C, Klp. (N—I) 12,7°C,

2-(p-Allyloxyphenol)-5-butylpyrimidin; Smp. (C—I) 56°C,

2-[p-(3-Butenyl)oxyphenyl]-5-butylpyrimidin; Smp. (C—I), 37,3°C, Klp. (N—I) 12,5°C,

2-[p-(4-Pentenyl)oxyphenyl]-5-butylpyrimidin; Smp. (C—I), 36,5°C, Klp. (N—I) 35,9°C.

## Beispiel 17

Zu einer Suspension von 5,3 g Lithiumaluminiumhydrid in 200 ml trockenem Tetrahydrofuran wurde unter Rühren in Inertgasatmosphäre innert 1 Stunde bei 5°C eine Lösung von 16,1 g 2-(1E-Pentenyl)malonsäurediäthylester (Tetrahedron Lett. *1979*, 861) in 75 ml Tetrahydrofuran zugetropft. Das Gemisch wurde noch 3,5 Stunden bei Raumtemperatur gerührt und dann nacheinander tropfenweise mit 15 ml Aceton und 20 ml gesättigter Natriumhydrogencarbonat-Lösung versetzt. Das Reaktionsgemisch wurde filtriert, das Filtrat eingeengt und der Rückstand (8,2 g) im Kugelrohr bei 150°C/ca. 1 Torr destilliert. Hierbei wurden 6,5 g 2-(1E-Pentenyl)-1,3-propandiol als farbloses Oel erhalten.

In analoger Weise wurden folgende Verbindungen hergestellt:

2-(1E-Propenyl)-1,3-propandiol,

2-(1E-Butenyl)-1,3-propandiol,

2-(1E-Hexenyl)-1,3-propandiol,

2-(1E-Heptenyl)-1,3-propandiol.

## Beispiel 18

a) In einer Lösung von 49 mg Malonsäurediäthylester in 175 ml Aethanol wurden 7,05 g Natrium aufgelöst. Die noch warme Lösung (50°C) wurde innert 15 Minuten tropfenweise mit 45,6 g 5-Brom-1-penten versetzt und 2 Stunden zum Sieden erhitzt. Nach dem Erkalten wurde das Reaktionsgemisch auf 500 ml Diäthyläther und 300 ml halbgesättigte Kochsalzlösung gegossen. Die wässrige Phase wurde abgetrennt und zweimal mit je 200 ml Diäthyläther nachextrahiert. Die organischen Phasen wurden zweimal mit je 150 ml halbgesättigter Kochsalzlösung gewaschen, über Natriumsulfat getrocknet, filtriert und eingeengt. Chromatographische Trennung der erhaltenen, gelben Flüssigkeit (55,2 g) an Kieselgel mit Hexan/Aethylacetat (Vol. 95:5) ergab 33,2 g (4-Pentenyl)malonsäurediäthylester als farblose Flüssigkeit.

b) Eine Suspension von 13,8 g Lithiumaluminiumhydrid in 500 ml Tetrahydrofuran wurde unter Stickstoff bei 0—5°C innert 1 Stunde tropfenweise mit einer Lösung von 33,2 g (4-Pentenyl)malonsäurediäthylester in 125 ml Tetrahydrofuran versetzt. Das Gemisch wurde über Nacht bei Raumtemperatur gerührt und dann 3 Stunden zum Sieden erhitzt. Nach dem Erkalten wurde vorsichtig zuerst 25 ml Aceton und dann 25 ml gesättigte Natriumhydrogencarbonat-Lösung zum Reaktionsgemisch zugetropft. Der entstandene Brei wurde genutscht und der Rückstand auf der Nutsche viermal mit Tetrahydrofuran gewaschen. Einengen des Filtrates ergab 17,8 g 2-(4-Pentenyl)-1,3-propandiol (Reinheit 89%) als gelbe Flüssigkeit.

c) Ein Gemisch von 3,6 g 2-(4-Pentenyl)-1,3-propandiol, 4,4 g p-(3-Butenyl)oxybenzaldehyd, 75 ml Toluol und 3 Tropfen 10-proz. Schwefelsäure wird 2,5 Stunden zum Sieden erhitzt, wobei ca. 50 ml feuchtes Lösungsmittel abdestilliert und dieses durch Zutropfen von 50 ml frischem Toluol ersetzt wird. Danach wird das Gemisch mit 4 Tropfen Triäthylamin neutralisiert und nach dem Erkalten mit 5-proz. Natriumhydrogencarbonat-Lösung und Wasser gewaschen. Die organische Phase wird über Natriumsulfat getrocknet und

eingeengt. Chromatographische Trennung des Rückstandes an Kieselgel mit Hexan/Essigester und Umkristallisation aus Hexan ergibt trans-2-[p-(3-Butenyl)oxyphenyl]-5-(4-pentenyl)-m-dioxan mit Smp. (C—I) 17,8°C und Klp. (N—I) 7,3°C.

In analoger Weise können folgende Verbindungen hergestellt werden:
trans-2-(p-Allyloxyphenyl)-5-pentyl-m-dioxan; Smp. (C—N) 41,6°C, Klp. (N—I) 42,5°C,
trans-2-[p-(3-Butenyl)oxyphenyl]-5-pentyl-m-dioxan; Smp. (C—N) 31,4°C, Klp. (N—I) 35,2°C,

Beispiel 19

a) Eine Suspension von 829 mg Natriumborhydrid in 20 ml Methanol/Diäthyläther (Vol. 9:1) wurde bei 0°C innert 5 Minuten tropfenweise mit einer Lösung von 3,0 g trans-4-Cyanocyclohexancarboxaldehyd in 30 ml Methanol/Diäthyläther (Vol. 9:1) versetzt. Das Reaktionsgemisch wurde noch 2 Stunden bei 10°C gerührt, dann mit 10 ml verdünnter Salzsäure versetzt und in Methylenchlorid/Wasser verteilt. Die wässrige Phase wurde dreimal mit Methylenchlorid extrahiert. Die organischen Phasen wurden zweimal mit Wasser gewaschen, über Magnesiumsulfat getrocknet, filtriert und eingeengt. Es resultierten 3,0 g (98%) trans-4-(Hydroxymethyl)cyclohexancarbonitril als farbloses Oel.

b) Eine Lösung von 3,0 g trans-4-(Hydroxymethyl)cyclohexancarbonitril in 10 ml Pyridin wurde bei 0°C innert 3 Minuten tropfenweise mit einer Lösung von 6,81 g p-Tosylchlorid in 10 ml Pyridin versetzt. Das Reaktionsgemisch wurde 15 Stunden bei Raumtemperatur gerührt, dann mit 50 ml 25-proz. Salzsäure sauer gestellt (pH ca. 2) und in Chloroform/Wasser verteilt. Die wässrige Phase wurde dreimal mit Chloroform extrahiert. Die organischen Phasen wurden zweimal mit Wasser gewaschen, über Magnesiumsulfat getrocknet, filtriert und eingeengt. Es resultierten 6,3 g (99%) trans-4-(p-Tosyloxymethyl)cyclohexancarbonitril als farblose Kristalle.

c) Eine Lösung von 6,3 g trans-4-(p-Tosyloxymethyl)cyclohexancarbonitril in 80 ml Aceton wurde mit 3,87 g Natriumjodid versetzt und das Gemisch unter Rühren 15 Stunden zum Rückfluss erhitzt. Danach wurde die weisse Suspension filtriert und das Filtrat eingeengt. Der Rückstand wurde in Wasser/Chloroform verteilt. Die wässrige Phase wurde dreimal mit Chloroform extrahiert. Die organischen Phasen wurden zweimal mit Wasser gewaschen, über Magnesiumsulfat getrocknet, filtriert und eingeengt. Es resultierten 4,8 g (89%) trans-4-(Jodmethyl)cyclohexancarbonitril als gelbliches Oel.

d) Eine Suspension von 9,14 g Kupfer-(I)-jodid in 90 ml Tetrahydrofuran wurde bei −78°C innert 5 Minuten mittels einer Spritze mit 25,7 ml einer 1,5M Lösung von Methyllithium in Tetrahydrofuran versetzt. Die Suspension wurde noch 45 Minuten bei −78°C gerührt, dann auf 0°C erwärmen gelassen und noch 3 Minuten bei 0°C gerührt. Danach wurde die Suspension wieder auf −78°C abgekühlt und innert 5 Minuten mittels einer Stahlkanüle mit einer aus 4,56 ml 4-Brom-1-buten und 1,1 g Magnesium in 60 ml Tetrahydrofuran hergestellten Grignardlösung versetzt. Die Suspension wurde noch 20 Minuten bei −78°C gerührt, dann auf 15°C erwärmen gelassen und noch 5 Minuten bei 15°C gerührt. Danach wurde die Suspension wieder auf −78°C abgekühlt und innert 5 Minuten tropfenweise mit einer Lösung von 4,8 g trans-4-(Jodmethyl)cyclohexancarbonitril in 30 ml Tetrahydrofuran versetzt. Danach wurde das Reaktionsgemisch erwärmen gelassen und noch 30 Minuten bei 16°C gerührt. Anschliessend wurde das Reaktionsgemisch im Kühlbad vorsichtig mit ca. 50 ml Ammoniumchlorid-Lösung versetzt und in Methylenchlorid/Ammoniumchlorid-Lösung verteilt. Die wässrige Phase wurde dreimal mit Methylenchlorid extrahiert. Die organischen Phasen wurden einmal mit Ammoniumchlorid-Lösung und zweimal mit Wasser gewaschen, über Magnesiumsulfat getrocknet, filtriert und eingeengt. Chromatographische Trennung des gelben, öligen Rückstandes (3,4 g) an Kieselgel mit Aethylacetat/Petroläther (Vol. 5:95) ergab 2,9 g (85%) trans-4-(4-Pentenyl)cyclohexancarbonitril als leicht gelbes Oel.

e) Eine Lösung von 2,9 g trans-4-(4-Pentenyl)cyclohexancarbonitril in 80 ml Diäthylenglykol wurde mit 8,0 g Kaliumhydroxid versetzt und das Gemisch 3,5 Stunden bei einer Badtemperatur von 180°C gerührt. Anschliessend wurde das Reaktionsgemisch mit Eis versetzt, mit 25 ml 25-proz. Salzsäure sauer gestellt und in Methylenchlorid/Wasser verteilt. Die wässrige Phase wurde dreimal mit Methylenchlorid extrahiert. Die organischen Phasen wurden zweimal mit Wasser gewaschen, über Magnesiumsulfat getrocknet, filtriert und eingeengt. Chromatographische Trennung des braunen, öligen Rückstandes (3,5 g) an Kieselgel mit Aethylacetat/Petroläther (Vol. 10:90) ergab 2,9 g (90%) trans-4-(4-Pentenyl)cyclohexancarbonsäure als gelbliches Oel.

Beispiel 20

a) Eine Lösung von 400 mg p-(3-Hydroxypropyl)phenol und 0,421 ml Triäthylamin in 2,6 ml Methylenchlorid wurde bei 0°C portionenweise mit 539 mg p-Tosylchlorid versetzt und das Gemisch noch 5 Minuten bei 0°C und 15 Minuten bei −5°C gerührt. Anschliessend wurde das Reaktionsgemisch mit Wasser verdünnt, mit verdünnter Salzsäure leicht sauer gestellt und dreimal mit Methylenchlorid extrahiert. Die organischen Phasen wurden zweimal mit Wasser gewaschen, über Magnesiumsulfat getrocknet und vom Lösungsmittel befreit. Das zurückbleibende, farblose Oel (870 mg) wurde an Kieselgel mit Aethylacetat/Petroläther chromatographisch gereingt, wobei 693 mg (83%) p-(3-Hydroxypropyl)phenyl-p-tosylat als milchiges Oel isoliert wurden.

b) Eine Lösung von 12 ml Oxalylchlorid in 380 ml Methylenchlorid wurde bei −60°C tropfenweise mit einer Lösung von 19,8 ml Dimethylsulfoxid in 30 ml Methylenchlorid versetzt und noch 5 Minuten bei −60°C gerührt. Anschliessend wurde das Gemisch tropfenweise mit einer Lösung von 38,9 g p-(3-Hydroxypropyl)phenyl-p-tosylat in 100 ml Methylenchlorid versetzt, noch 20 Minuten bei −60°C gerührt und dann mit 88,5 ml Triäthylamin versetzt. Das Reaktionsgemisch wurde noch 5 Minuten bei −60°C gerührt, dann langsam auf Raumtemperatur erwärmen gelassen und noch 5 Minuten bei 24°C gerührt. Anschliessend wurde das Reaktionsgemisch mit Wasser versetzt. Die organische Phase wurde mit Wasser und Kochsalzlösung gewaschen (Nachextrahieren mit Methylenchlorid), über Magnesiumsulfat getrocknet und eingedampft. Chromatographische Trennung des zurückbleibenden, braungelben Oeles (38 g) an Kieselgel mit Aethylacetat/Petroläther (Vol. 40:60) ergab 33,8 g 3-[p-(p-Tosyloxy)phenyl]propionaldehyd als hellgelbes Oel.

c) Eine Suspension von 14,87 g Methoxymethyl-triphenylphosphoniumchlorid in 130 ml t-Butylmethyläther wurde bei −20°C mit 5,2 g Kalium-t-butylat versetzt und ohne Kühlung noch 1,2 Stunden gerührt. Anschliessend wurde das Gemisch bei −5°C tropfenweise mit einer Lösung von 8,8 g 3-[p-(p-Tosyloxy)-phenyl]propionaldehyd in 30 ml Tetrahydrofuran versetzt. Das Kühlbad wurde entfernt und das Gemisch noch 10 Minuten bei Raumtemperatur gerührt. Dann wurde das Reaktionsgemisch mit Wasser versetzt und zweimal in Wasser/Diäthyläther verteilt. Die wässrigen Phasen wurden mit Diäthyläther nachextrahiert. Die organischen Phasen wurden über Magnesiumsulfat getrocknet und eingedampft. Der ölige Rückstand wurde in Aethylacetat gelöst und die Lösung mit Petroläther versetzt und durch Filtration von ausgefallenen Triphenylphosphinoxid befreit. Nach Eindampfen des Filtrates und chromatographischer Trennung des Rückstandes an Kieselgel wurden 4,4 g (44%) p-(4-Methoxy-3-butenyl)phenyl-p-tosylat als leicht gelbliches Oel erhalten.

d) Ein Gemisch von 4,3 g p-(4-Methoxy-3-butenyl)phenyl-p-tosylat, 60 ml Eisessig und 30 ml Wasser wurde 40 Minuten bei 110°C gerührt, dann auf Raumtemperatur abgekühlt und mit 200 ml Wasser verdünnt. Die wässrige Phase wurde viermal mit Methylenchlorid extrahiert. Die organischen Phasen wurden zweimal mit verdünnter Natriumcarbonat-Lösung gewaschen, über Magnesiumsulfat getrocknet und vom Lösungsmittel befreit. Es resultierten 4,35 g 4-[p-(p-Tosyloxy)phenyl]butyraldehyd als gelbliches Oel.

e) Eine Suspension von 7,32 g Methyltriphenylphosphoniumbromid in 70 ml t-Butylmethyläther wurde bei −5°C mit 2,45 g Kalium-t-butylat versetzt und noch 40 Minuten bei Raumtemperatur gerührt. Anschliessend wurde das Gemisch bei 0°C langsam mit einer Lösung von 4,35 g 4-[p-(p-Tosyloxy)phenyl]-butyraldehyd in 30 ml t-Butylmethyläther versetzt. Das Reaktionsgemisch wurde noch 5 Minuten bei 0°C und 30 Minuten bei Raumtemperatur gerührt, dann mit Wasser versetzt und zweimal in Wasser/Diäthyläther verteilt. Die wässrigen Phasen wurden mit Diäthyläther nachextrahiert. Die organischen Phasen wurden über Magnesiumsulfat getrocknet und eingeengt. Der Rückstand wurde in Aethylacetat gelöst und die Lösung mit Petroläther versetzt und durch Filtration vom ausgefallenen Triphenylphosphinoxid befreit. Nach Einengen des Filtrates und chromatographischer Trennung des zurückbleibenden, gelben Oeles (4,3 g) an Kieselgel wurden 3,37 g (78%) p-(4-Pentenyl)phenyl-p-tosylat als farbloses Oel erhalten.

f) Ein Gemisch von 3,35 g p-(4-Pentenyl)phenyl-p-tosylat und 50 ml 10%-iger, äthanolischer Kaliumhydroxid-Lösung wurde 1 Stunde bei 100°C gerührt. Anschliessend wurde das Reaktionsgemisch auf Raumtemperatur abgekühlt und mit verdünnter Salzsäure sauer gestellt. Die wässrige Phase wurde viermal mit Diäthyläther extrahiert. Die organischen Phasen wurden mit Wasser gewaschen, über Magnesiumsulfat getrocknet und eingeengt. Das zurückbleibende braune Oel (1,92 g) wurde an Kieselgel mit Aethylacetat/Petroläther (Vol. 8:92) chromatographisch gereinigt, wobei 1,70 g (99%) p-(4-Pentenyl)phenol als leicht gelbes Oel erhalten wurden.

Beispiel 21

a) Eine Suspension von 32 g Aethyltriphenylphosphoniumbromid in 450 ml t-Butylmethyläther wurde bei 0°C langsam mit 10 g Kalium-t-butylat versetzt und noch 1 Stunde ohne Kühlung gerührt. Anschliessend wurde das Gemisch bei 0°C langsam mit einer Lösung von 15 g 3-[p-(p-Tosyloxy)phenyl]-propionaldehyd in 50 ml Tetrahydrofuran versetzt und dann langsam auf Raumtemperatur erwärmt. Danach wurde das Reaktionsgemisch mit Wasser versetzt und zweimal in Diäthyläther/Wasser verteilt. Die wässrigen Phasen wurden mit Diäthyläther nachextrahiert. Die organischen Phasen wurden über Magnesiumsulfat getrocknet und eingeengt. Das erhaltene Oel wurde durch wiederholtes Lösen in Aethylacetat, Versetzen mit Petroläther, Filtrieren und Einengen des Filtrates von Triphenylphosphinoxid befreit. Das erhaltene, gelbe ölige Rohprodukt (18 g) wurde an Kieselgel mit Petroläther und Aethylacetat/Petroläther chromatographisch gereinigt. Es resultierten 7,1 g (46%) p-(3-Pentenyl)phenyl-p-tosylat als farbloses Oel (cis/trans-Gemisch).

b) p-(3-Pentenyl)phenyl-p-tosylat wurde in analoger Weise zu Beispiel 8b) mit m-Chlorperbenzoesäure zu p-(3,4-Epoxypentyl)phenyl-p-tosylat oxidiert, dann das Epoxid in analoger Weise zu Beispiel 8c) mit Triphenylphosphin-Brom zu p-(erythro-3,4-Dibrompentyl)phenyl-p-tosylat umgesetzt und schliesslich das Dibromid in analoger Weise zu Beispiel 8d) mit Zink in Eisessig zu p-(3E-Pentenyl)phenyl-p-tosylat reduziert.

c) Ein Gemisch von 7,9 g p-(3E-Pentenyl)phenyl-p-tosylat und 120 ml 10%-iger, äthanolischer Kaliumhydroxid-Lösung wurde 45 Minuten zum Rückfluss erhitzt, dann auf Raumtemperatur abgekühlt, mit Salzsäure sauer gestellt und dreimal in Wasser/Diäthyläther verteilt. Die wässrigen Phasen wurden mit Diäthyläther nachextrahiert. Die organischen Phasen wurden über Magnesiumsulfat getrocknet und

eingeengt. Das zurückbleibende Oel (4,1 g) wurde an mit Silbernitrat beschichtetem Kieselgel mit Diäthyläther/Hexan chromatographisch gereinigt. Es resultierten 3,15 g (77%) p-(3E-Pentenyl)phenol als leicht gelbliche Flüssigkeit.

Beispiel 22

a) Eine Suspension von 11,5 g 3,3-Aethylendioxypropyl-triphenylphosphoniumbromid in 90 ml t-Butylmethyläther wurde bei 0°C mit 3,02 g Kalium-t-butylat versetzt und dann bei Raumtemperatur 40 Minuten gerührt. Danach wurde das Gemisch bei 5°C tropfenweise mit einer Lösung von 2,6 g p-Cyanobenzaldehyd in 30 ml Tetrahydrofuran versetzt und noch 1 Stunde bei Raumtemperatur gerührt. Anschliessend wurde das Reaktionsgemisch mit Wasser versetzt. Die Phasen wurden getrennt und die wässrige Phase wurde zweimal mit Tetrahydrofuran nachextrahiert. Die organischen Phasen wurden zweimal mit Wasser gewaschen, über Magnesiumsulfat getrocknet und eingeengt. Der Rückstand wurde in heissem Aethylacetat gelöst, die Lösung mit Petroläther versetzt und das ausgefallene Triphenylphosphinoxid abgenutscht. Das nach Einengen des Filtrates erhaltene gelbe Oel (4,5 g) wurde an Kieselgel mit Petroläther und Petroläther/Aethylacetat chromatographisch gereinigt. Es resultierten 3,73 g (89,2%) p-(4,4-Aethylendioxy-1-butenyl)benzonitril als leicht gelbliches Oel.

b) Eine Lösung von 3,7 g p-(4,4-Aethylendioxy-1-butenyl)benzonitril in 50 ml Toluol wurde mit 350 mg Palladium/Kohle (5%) versetzt und 2,5 Stunden hydriert (Wasserstoffverbrauch 385 ml). Dann wurde das Reaktionsgemisch filtriert (Nachwaschen mit Diäthyläther) und das Filtrat eingedampft. Es resultierten 308 mg (82%) p-(4,4-Aethylendioxybutyl)benzonitril als farbloses, teilweise kristallisierendes Oel.

c) Ein Gemisch von 2,8 g p-(4,4-Aethylendioxybutyl)benzonitril, 56 ml Tetrahydrofuran und 56 ml 10-proz. Salzsäure wurde 3 Stunden bei Raumtemperatur gerührt und dann über Nacht stehengelassen. Danach wurde das Reaktionsgemisch mit Wasser verdünnt und dreimal mit Diäthyläther extrahiert. Die organischen Phasen wurden zweimal mit Wasser gewaschen, über Magnesiumsulfat getrocknet, filtriert und eingeengt. Es resultierten 2,6 g 4-(p-Cyanophenyl)butyraldehyd, welche ohne zusätzliche Reinigung weiterverwendet wurden.

d) Eine Suspension von 6,5 g Methyl-triphenylphosphoniumbromid in 50 ml t-Butylmethyläther wurde bei −5°C mit 2,1 g Kalium-t-butylat versetzt und dann bei Raumtemperatur 40 Minuten gerührt. Anschliessend wurde das Gemisch bei 0°C mit einer Lösung von 2,6 g 4-(p-Cyanophenyl)butyraldehyd in 30 ml t-Butylmethyläther versetzt und 1 Stunde bei Raumtemperatur gerührt. Danach wurde das Reaktionsgemisch mit Wasser versetzt und dreimal mit Diäthyläther extrahiert.

Die organischen Phasen wurden zweimal mit Wasser gewaschen, über Magnesiumsulfat getrocknet, filtriert und eingeengt. Der kristalline Rückstand wurde in heissem Aethylacetat gelöst, die Lösung mit Petroläther versetzt und das ausgefallene Triphenylphosphin abfiltriert. Das nach Einengen des Filtrates erhaltene Oel wurde an Kieselgel mit Aethylacetat/Petroläther (Vol. 5:95) chromatographisch gereinigt, wobei 1,92 g p-(4-Pentenyl)benzonitril als leicht gelbliche Flüssigkeit isoliert wurden.

e) Ein Gemisch von 1,9 g p-(4-Pentenyl)benzonitril und einer 10-proz. Lösung von Kaliumhydroxid in Diäthylenglykol wurde 2 Stunden bei 180°C gekocht. Dann wurde das Reaktionsgemisch auf Raumtemperatur abgekühlt, mit 23-proz. Salzsäure auf pH 3 gestellt, mit Wasser verdünnt und viermal mit Methyenchlorid extrahiert. Die organischen Phasen wurden zweimal mit Wasser gewaschen, über Magnesiumsulfat getrocknet, filtriert und eingeengt, wobei 2,12 g p-(4-Pentenyl)benzoesäure als braune Kristalle isoliert.

Beispiel 23

a) Eine Lösung von 3-Butenylmagnesiumbromid in Diäthyläther (hergestellt aus 172 mg Magnesium-Späne und 0,604 ml 4-Brom-1-buten in 20 ml Diäthyläther) wurde bei Raumtemperatur tropfenweise mit einer Lösung von 1,0 g p-[trans-4-(4-Pentenyl)cyclohexyl]benzonitril in 10 ml Toluol versetzt und das Gemisch 17 Stunden bei 45°C gerührt. Danach wurde das Reaktionsgemisch bei 0°C vorsichtig mit gesättigter Ammoniumchlorid-Lösung versetzt und dreimal in Diäthyläther/Wasser verteilt. Die organischen Phasen wurden zweimal mit Wasser gewaschen, über Magnesiumsulfat getrocknet, filtriert und eingeengt. Chromatographische Trennung des gelben, kristallinen Rückstandes (1,28 g) an Kieselgel mit Aethylacetat/Petroläther (Vol. 3:97) ergab 1,19 g (97%) p-[trans-4-(4-Pentenyl)cyclohexyl]-4-pentenoylphenon als farbloses Oel.

b) Eine Lösung von 1,19 g p-[trans-4-(4-Pentenyl)cyclohexyl]-4-pentenoylphenon in 10 ml Aethanol und 10 ml Diäthylenglykol wurde unter Argonbegasung mit 0,372 ml Hydrazinhydrat versetzt und dann unter Rühren 2 Stunden zum Rückfluss erhitzt (Badtemperatur 110°C). Anschliessend wurde das Gemisch mit 439 mg festem Kaliumhydroxid versetzt, die Badtemperatur auf 210°C erhöht und der Aethanol abdestilliert. Nach 2 stunden bei 210°C wurde die Reaktion abgebrochen und das Gemisch dreimal in Wasser/Petroläther verteilt. Die organischen Extrakte wurden zweimal mit Wasser gewaschen, über Magnesiumsulfat getrocknet und eingedampft. Chromatographische Trennung des Rückstandes (0,84 g) and Kieselgel mit Hexan als Eluens ergab 0,725 g (64%) 4-(4-Pentenyl-1-[trans-4-(4-pentenyl)cyclohexyl]benzol. Kugelrohrdestillation ergab bei 165°C/0,25 Torr 530 mg Produkt als farbloses Oel mit Smp. −32,6°C.

In analoger Weise wurden folgende Verbindungen hergestellt:

4'-[trans-4-(1E-Pentenyl)cyclohexyl]-4-(4-pentenoyl)biphenyl; Smp. 133,7°C,

4'-[trans-4-(1E-Pentenyl)cyclohexyl]-4-(4-pentenyl)biphenyl; Klp. (S—I) 182,5°C, nicht kristallisierbar.

Beispiel 24

a) Eine Suspension von 91,0 g Pyridiniumchlorochromat in 650 ml Methylenchlorid wurde unter Rühren bei Raumtemperatur innert 5 Minuten tropfenweise mit einer Lösung von 22,6 g 5-Hexen-1-ol in 70 ml Diäthyläther versetzt und noch 2 Stunden gerührt Anschliessend wurde das Gemisch mit 400 ml Diäthyläther versetzt und noch 15 Minuten gerührt. Danach wurde die Reaktionslösung vom ausgeschiedenen schwarzen Harz abdekantiert und filtriert. Fraktionierte Destillation des Filtrates unter Normaldruck ergab bei 110—122°C 11,88 g 5-Hexanal.

b) Eine Suspension von 62,2 g Methoxymethyl-triphenylphosphoniumchlorid in 250 ml Diäthyläther wurde bei 0°C unter Stickstoff mit 21,4 g Kalium-t-butylat versetzt. Die erhaltene orangerote Suspension wurde bei 5—10°C innert 15 Minuten tropfenweise mit einer Lösung von 11,88 g 5-Hexenal in 65 ml Diäthyläther versetzt und noch 3 Stunden bei Raumtemperatur gerührt. Danach wurde das Reaktionsgemisch mit 7,5 g Natriumhydrogencarbonat und 125 ml Wasser versetzt und 10 Minuten gerührt. Die wässrige Phase wurde abgetrennt und mit 30 ml Diäthyläther nachextrahiert. Die organischen Phasen wurden zweimal mit je 30 ml Wasser gewaschen, über Natriumsulfat getrocknet und filtriert. Das Filtrat wurde bei 60°C Badtemperatur unter Normaldruck von Lösungsmittel befreit. Der Destillationsruckstand (gelbe Flüssigkeit) wurde mit 400 ml Pentan geschüttelt, bis der ungelöste Rückstand fest geworden war. Die erhaltene Suspension wurde auf −25°C gekühlt und filtriert. Fraktionierte Destillation des Filtrates unter Normaldruck ergab bei 120—143°C 10,4 g 1-Methoxy-1,6-heptadien.

c) 13,2 ml Orthoameisensäuretrimethylester wurden bei 3°C unter Inertgasatmosphäre mit 0,22 ml Bortrifluorid-diäthylätherat versetzt. Dann wurde das Gemisch unter Rühren innert 5 Minuten tropfensweise mit 2,52 g 1-Methoxy-1,6-heptadien versetzt. Das Reaktionsgemisch wurde noch 3 Stunden im Eisbad stehengelassen, dann mit 0,22 ml Triäthanolamin versetzt und im Rotationsverdampfer bei 50°C eingeengt. Der Rückstand wurde in 30 ml Hexan gelöst und die Lösung mit 5 ml gesättigter Natriumhydrogencarbonat-Lösung und viermal mit je 5 ml Wasser gewaschen, über Natriumsulfat getrocknet und eingeengt. Kugelrohrdestillation des Rückstandes (4,0 g) bei 220°C/9 Torr ergab 3,74 g (4-Pentenyl)malonaldehydtetramethylacetal als farblose Flüssigkeit.

d) Ein Gemisch von 3,74 g (4-Pentenyl)malonaldehydtetramethylacetal, 0,27 ml Wasser und 75 mg p-Toluolsulfonsäure-Monohydrat wurde 2 Stunden bei einer Badtemperatur von 110°C erhitzt. Danach wurde das Gemisch mit 0,12 ml Triäthylamin versetzt, abkühlen gelassen und auf 80 ml Hexan ausgegossen. Das Reaktionsgemisch wurde zweimal mit je 15 ml gesättigter Natriumhydrogencarbonat-Lösung gewaschen, über Natriumsulfat getrocknet und filtriert. Einengen des Filtrates ergab 2,4 g rohes 3-Methoxy-2-(4-pentenyl)acrolein.

Beispiel 25

Eine Lösung von 21,2 g 5-Butyl-2-(p-isopropoxyphenyl)pyrimidin [hergestellt gemäss Synthesevariante 2 in J. prakt. Chem. *317,* 617 (1975)] in 300 ml Methylenchlorid wurde unter Rühren bei 0°C innert 1 Stunde tropfenweise mit einer Lösung von 37 ml Titantetrachlorid in 200 ml Methylenchlorid versetzt. Das Reaktionsgemisch wurde noch 30 Minuten bei 0°C und 3 Stunden bei Raumtemperatur gerührt und dann auf 1 l Eiswasser gegossen. Die wässrige Phase wurde mit 1,2 l Methylenchlorid extrahiert. Die vereinigten organischen Phasen wurden mit Wasser neutral gewaschen, über Natriumsulfat getrocknet, filtriert und eingeengt. Das braune, feste Rohprodukt (15,0 g) wurde, adsorbiert an 75 g Kieselgel, auf eine Säule von Kieselgel in Toluol/Aceton (Vol. 9:1) aufgetragen und mit dem gleichen Lösungsmittelgemisch eluiert. Das erhaltene Produkt (12,8 g) wurde mit Hexan ausgekocht, wobei 12,6 g 5-Butyl-2-(p-hydroxyphenyl)pyrimidin mit Smp. 184,2—184,6°C isoliert wurden.

Beispiel 26

a) Zu einer farblosen Lösung von 36,4 g 3-Butenylbenzol in 300 ml Tetrachlorkohlenstoff tropfte man unter Rühren bei 10—15°C im Verlaufe von 5 Stunden 275 ml einer 1M Lösung von Brom in Tetrachlorkohlenstoff. Das Reaktionsgemisch wurde über Nacht bei Raumtemperatur stehengelassen und dann im Vakuum von Lösungsmittel befreit. Man erhielt 79,9 g (3,4-Dibrombutyl)benzol als gelbliches Oel.

b) Zu einer auf 0°C gekühlten Lösung von 40,0 g (3,4-Dibrombutyl)benzol in 48 ml Acetanhydrid tropfte man im Verlaufe von 50 Minuten bei 3—4°C eine Nitrierlösung (hergestellt durch Zutropfen von 16,5 g 96-proz. Salpetersäure zu einer gerührten Mischung von 8,2 ml Eisessig und 8,2 ml Acetanhydrid bei 5—9°C). die gelbliche Lösung wurde im Verlaufe von 4 Stunden auf Raumtemperatur erwärmen gelassen. Nach Stehenlassen über Nacht wurde das Gemisch auf eine Mischung von 250 g Eis und 100 ml Wasser gegossen und mit 13,5 g Natriumcarbonat ($Na_2CO_3 \cdot 10H_2O$) versetzt. Extraktion des Gemisches mit Diäthyläther, Waschen der ätherischen Lösungen mit Wasser, gesättigter Natriumhydrogencarbonat-Lösung und Wasser, Trocknen mit Natriumsulfat und Einengen im Vakuum ergab 47,3 g braunes Oel enthaltend 39,7% o-Nitroderivat und 58,7% p-Nitroderivat. Chromatographie an Kieselgel in Hexan und Elution mit Hexan-Toluol ergab 14,2 g 1-(3,4-Dibrombutyl)-2-nitro-benzol, 13,7 g Isomergemisch und 18,2 g 1-(3,4-Dibrombutyl)-4-nitrobenzol.

c) Eine Mischung von 52,1 g Natriumjodid, 300 ml Aceton und 28,6 g 1-(3,4-Dibrombutyl)-4-nitrobenzol wurde während 3,5 Stunden unter Rückfluss gerührt. Die braune Suspension wurde nach dem Abkühlen im Vakuum vom Lösungsmittel befreit und der braune Rückstand in Diäthyläther und Wasser aufgenommen.

Das ausgeschiedene Jod wurde durch Zugabe von festem Natriumthiosulfat reduziert. Die farblossen Aetherlösungen wurden nach Waschen mit Wasser und Trocknen mit Natriumsulfat im Vakuum eingeengt. Der Rückstand (14,9 g) wurde im Hochvakuum destilliert, wobei 13,4 g 1-(3-Butenyl)-4-nitrobenzol als praktisch farblose Flüssigkeit erhalten wurden: Sdp. 86—89°C/0,2 Torr.

d) Unter Rühren und Stickstoffbegasung wurde eine Lösung von 11,9 g 1-(3-Butenyl)-4-nitrobenzol in 875 ml Methanol mit 6,5 g Magnesiumspänen versetzt. Nach 4 Stunden Rühren wurde das gelbe, trübe Reaktionsgemisch im Vakuum vom Methanol befreit. Der Rückstand wurde in 220 ml Wasser und 110 ml 17-proz. Salzsäure gelöst und die Lösung erschöpfend mit Methylenchlorid extrahiert. Die organischen Phasen wurden mit Wasser neutral gewaschen, über Natriumsulfat getrocknet und im Vakuum von Lösungsmittel befreit. Der Rückstand (10,4 g) wurde mit 100 ml Wasser versetzt und einer Wasserdampfdestillation unterworfen. Aus dem Destillat konnten durch Ausschütteln mit Methylenchlorid 3,3 g Ausgangsprodukt zurückgewonnen werden. Extraktion des Destillationsrückstandes mit Methylenchlorid ergab 7,0 g braune Flüssigkeit, die in Hexan gelöst an 260 g Kieselgel in Hexan chromatographiert wurden. Hexan/Toluol eluierte 2,074 g eines Gemisches von Ausgangsprodukt und 4,4'-Di-(3-butenyl)-azobenzol, dann 0,353 g Mischfraktion und schliesslich 4,358 g rohe Azoxyverbindung als dunkelgelbe, flüssigkristalline, später kristallisierende Substanz. Umkristallisation aus Diäthyläther/Methanol ergab reines 4,4'-Di-(3-butenyl)azoxybenzol mit Smp. (C—N) 34,0°C, Klp. (N—I) 75,5°C.

**Patentansprüche für die Vertragsstaaten: CH DE FR GB LI**

1. Verbindungen der allgemeinen Formel

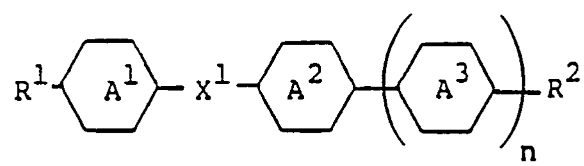

I

worin n für die Zahl 0 oder 1 steht; die Ringe $A^1$, $A^2$ und $A^3$ 1,4-Phenylen, 2-Fluor-1,4-phenylen oder trans-1,4-Cyclohexylen darstellen oder einer dieser Ringe auch einen 2,5-disubstituierten Pyrimidinring oder einen trans-2,5-disubstituierten m-Dioxanring darstellt; $X^1$ eine einfache Kovalenzbindung, —COO—, —OOC—, $—CH_2CH_2—$, $p-C_6H_4—$, $—CH_2CH_2-p-C_6H_4—$, $—CH_2CH_2-p-C_6H_4—CH_2CH_2—$ oder, sofern die Ringe $A^1$ und $A^2$ 1,4-Phenylen darstellen, auch eine Azoxygruppe —N=N(O)— oder —N(O)=N— bezeichnet; $R^2$ 1E-Alkenyl, 2Z-Alkenyl, 3E-Alkenyl, 4-Alkenyl oder Alkenyloxy darstellt, mit der Massgabe, dass das Sauerstoffatom in Alkenyloxy mit einem gesättigten Kohlenstoffatom verknüpft ist; und $R^1$ 1E-Alkenyl, 2Z-Alkenyl, 3E-Alkenyl, 4-Alkenyl oder, sofern $R^2$ Alkenyloxy darstellt, auch Alkyl bedeutet; mit der Massgabe, dass an einen Benzol- oder Pyrimidinring gebundene Reste $R^1$ und $R^2$ 3E-Alkenyl, 4-Alkenyl oder im Falle von $R^2$ auch Alkenyloxy oder im Falle von $R^1$ auch Alkyl bedeuten; und mit der weiteren Massgabe, dass nicht zugleich $R^1$ Alkyl, die Ringe $A^1$ und $A^2$ jeweils 1,4-Phenylen oder trans-1,4-Cyclohexylen, $X^1$ eine einfache Kovalenzbindung, n die Zahl 0 und $R^2$ Alkenyloxy bedeuten.

2. Verbindungen nach Anspruch 1, dadurch gekennzeichnet, dass $R^2$ 1E-Alkenyl, 2Z-Alkenyl, 3E-Alkenyl, 4-Alkenyl, (2E-Alkenyl)oxy, (3-Alkenyl)oxy, (4-Alkenyl)oxy oder (5-Alkenyl)oxy darstellt, und $R^1$ 1E-Alkenyl, 2Z-Alkenyl, 3E-Alkenyl, 4-Alkenyl oder, sofern $R^2$ eine Alkenyloxygruppe darstellt, auch Alkyl bedeutet.

3. Verbindungen nach Anspruch 2, dadurch gekennzeichnet, dass die Ringe $A^1$, $A^2$ und $A^3$ 1,4-Phenylen oder trans-1,4-Cyclohexylen darstellen oder einer dieser Ringe auch einen 2,5-disubstituierten Pyrimidinring oder einen trans-2,5-disubstituierten m-Dioxanring darstellt; $R^2$ 1E-Alkenyl, 2Z-Alkenyl, 3E-Alkenyl, 4-Alkenyl, (2E-Alkenyl)oxy oder (3-Alkenyl)oxy darstellt; und $R^1$ 1E-Alkenyl, 2Z-Alkenyl, 3E-Alkenyl, 4-Alkenyl oder, sofern $R^2$ (2E-Alkenyl)oxy oder (3-Alkenyl)oxy darstellt, auch Alkyl bedeutet.

4. Verbindungen nach einem der Ansprüche 1 bis 3, dadurch gekennzeichnet, dass $R^1$ und $R^2$ geradkettige Reste mit höchstens 12 Kohlenstoffatomen, vorzugsweise höchstens 7 Kohlenstoffatomen bedeuten.

5. Verbindungen nach einem der Ansprüche 1 bis 4, dadurch gekennzeichnet, dass $R^2$ (2E-Alkenyl)oxy bedeutet oder einer der Reste $R^1$ und $R^2$ 1E-Alkenyl oder 3E-Alkenyl bedeutet.

6. Verbindungen nach einem der Ansprüche 1 bis 5, dadurch gekennzeichnet, dass $X^1$ eine einfache Kovalenzbindung, —COO—, —OOC—, $—CH_2CH_2—$ oder $p-C_6H_4—$ bezeichnet.

7. Verbindungen nach einem der Ansprüche 1 bis 5, dadurch gekennzeichnet, dass Ring $A^1$ für trans-1,4-Cyclohexylen steht, und $X^1$ eine einfache Kovalenzbindung, —COO—, $—CH_2CH_2—$, $p-C_6H_4—$, $—CH_2CH_2-p-C_6H_4—$ oder $—CH_2CH_2-p-C_6H_4—CH_2CH_2—$ bezeichnet.

8. Verbindungen nach einem der Ansprüche 1 bis 7, dadurch gekennzeichnet, dass n die Zahl 0 und Ring $A^2$ 1,4-Phenylen, trans-1,4-Cyclohexylen oder einen 2,5-disubstituierten Pyrimidinring bedeutet, oder dass n Zahl 1 und Ring $A^2$ 1,4-Phenylen oder einen 2,5-disubstituierten Pyrimidinring bedeutet.

9. Verbindungen nach einem der Ansprüche 1 bis 8, dadurch gekennzeichnet, dass n für die Zahl 0 steht, Ring $A^1$ trans-1,4-Cyclohexylen darstellt, Ring $A^2$ 1,4-Phenylen darstellt und $X^1$ eine einfache Kovalenzbindung, —COO—, $—CH_2CH_2—$ oder $p-C_6H_4—$ bezeichnet.

10. Verbindungen nach einem der Ansprüche 1 bis 8, dadurch gekennzeichnet, dass ring $A^1$ einen 2,5-disubstituierten Pyrimidinring oder einen trans-2,5-disubstituierten m-Dioxanring darstellt, Ring $A^2$ 1,4-Phenylen oder trans-1,4-Cyclohexylen darstellt und $X^1$ eine einfache Kovalenzbindung bezeichnet.

11. Flüssigkristallines Gemisch mit mindestens 2 Komponenten, dadurch gekennzeichnet, dass mindestens eine Komponente eine Verbindung der allgemeinen Formel

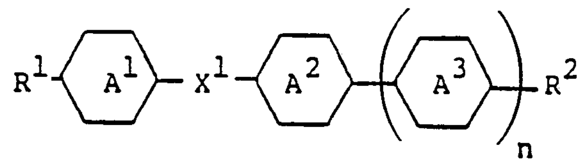

I

worin n für die Zah 0 oder 1 steht; die Ringe $A^1$, $A^2$ und $A^3$ 1,4-Phenylen, 2-Fluor-1,4-phenylen oder trans-1,4-Cyclohexylen darstellen oder einer dieser Ringe auch einen 2,5-disubstituierten Pyrimidinring oder einen trans-2,5-disubstituierten m-Dioxanring darstellt; $X^1$ eine einfache Kovalenzbindung, —COO—, —OOC—, $—CH_2CH_2—$, $p-C_6H_4—$, $—CH_2CH_2-p-C_6H_4—$, $—CH_2CH_2-p-C_6H_4—CH_2CH_2—$ oder, sofern die Ringe $A^1$ und $A^2$ 1,4-Phenylen darstellen, auch eine Azoxygruppe —N=N(O)— oder —N(O)=N— bezeichnet; $R^2$ 1E-Alkenyl, 2Z-Alkenyl, 3E-Alkenyl, 4-Alkenyl oder Alkenyloxy darstellt, mit der Massgabe, dass das Sauerstoffatom in Alkenyloxy mit einem gesättigten Kohlenstoffatom verknüpft ist; und $R^1$ 1E-Alkenyl, 2Z-Alkenyl, 3E-Alkenyl, 4-Alkenyl oder, sofern $R^2$ Alkenyloxy darstellt, auch Alkyl bedeutet; mit der Massgabe, dass an einen Benzol- oder Pyrimidinring gebundene Reste $R^1$ und $R^2$ 3E-Alkenyl, 4-Alkenyl oder im Falle von $R^2$ auch Alkenyloxy oder im Falle von $R^1$ auch Alkyl bedeuten, ist.

12. Flüssigkristallines Gemisch nach Anspruch 11, dadurch gekennzeichnet, dass $R^2$ (2E-Alkenyl)oxy bedeutet oder einer der Reste $R^1$ und $R^2$ 1E-Alkenyl oder 3E-Alkenyl bedeutet.

13. Flüssigkristallines Gemisch nach Anspruch 11 oder 12, dadurch gekennzeichnet, dass n für die Zahl 0 steht, Ring $A^1$ trans-1,4-Cyclohexylen darstellt, Ring $A^2$ 1,4-Phenylen darstellt und $X^1$ eine einfache Kovalenzbindung, —COO—, $—CH_2CH_2—$ oder $p-C_6H_4—$ bezeichnet.

14. Verwendung der Verbindungen der in Anspruch 11 definierten Formel I für elektro-optische Zwecke.

15. Verbindungen der allgemeinen Formel

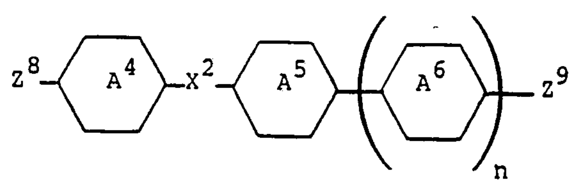

XXXIXa

worin n für die Zahl 0 oder 1 steht; die Ringe $A^4$, $A^5$ und $A^6$ 1,4-Phenylen, 2-Fluor-1,4-phenylen oder trans-1,4-Cyclohexylen darstellen; $X^2$ eine einfache Kovalenzbindung, $—CH_2CH_2—$, $p-C_6H_4—$, $—CH_2CH_2-p-C_6H_4—$ oder $—CH_2CH_2-p-C_6H_4—CH_2CH_2—$ bezeichnet; und einer der Reste $Z^8$ und $Z^9$ 3E-Alkenoyl oder 4-Alkenoyl und der andere 3E-Alkenyl, 4-Alkenyl, 3E-Alkenoyl, 4-Alkenoyl oder, sofern er nicht an einen Benzol- oder Pyrimidinring gebunden ist, auch 1E-Alkenyl oder 2Z-Alkenyl bedeutet.

**Patentansprüche für die Vertragsstaaten: IT SE**

1. Verbindungen der allgemeinen Formel

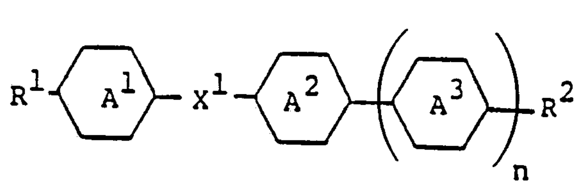

I

worin n für die Zahl 0 oder 1 steht; die Ringe $A^1$, $A^2$ und $A^3$ 1,4-Phenylen, 2-Fluor-1,4-phenylen oder trans-1,4-Cyclohexylen darstellen oder einer dieser Ringe auch einen 2,5-disubstituierten Pyrimidinring oder einen trans-2,5-disubstituierten m-Dioxanring darstellt; $X^1$ eine einfache Kovalenzbindung, —COO—, —OOC—, $—CH_2CH_2—$, $p-C_6H_4—$, $—CH_2CH_2—p-C_6H_4—$, $—CH_2CH_2—p-C_6H_4—CH_2CH_2—$ oder, sofern die Ringe $A^1$ und $A^2$ 1,4-Phenylen darstellen, auch eine Azoxygruppe —N=N(O)— ode —N(O)=N— bezeichnet; $R^2$ 1E-Alkenyl, 2Z-Alkenyl, 3E-Alkenyl, 4-Alkenyl oder Alkenyloxy darstellt, mit der Massgabe, dass das Sauerstoffatom in Alkenyloxy mit einem gesättigten Kohlenstoffatom verknüpft ist; und $R^1$ 1E-Alkenyl, 2Z-Alkenyl, 3E-Alkenyl, 4-Alkenyl oder, sofern $R^2$ Alkenyloxy darstellt, auch Alkyl bedeutet; mit der Massgabe,

dass an einen Benzol- oder Pyrimidinring gebundene Reste $R^1$ und $R^2$ 3E-Alkenyl, 4-Alkenyl oder im Falle von $R^2$ auch Alkenyloxy oder im Falle von $R^1$ auch Alkyl bedeuten.

2. Verbindungen nach Anspruch 1, dadurch gekennzeichnet, dass $R^2$ 1E-Alkenyl, 2Z-Alkenyl, 3E-Alkenyl, 4-Alkenyl, (2E-Alkenyl)oxy, (3-Alkenyl)oxy, (4-Alkenyl)oxy oder (5-Alkenyl)oxy darstellt, und $R^1$ 1E-Alkenyl, 2Z-Alkenyl, 3E-Alkenyl, 4-Alkenyl oder, sofern $R^2$ eine Alkenyloxygruppe darstellt, auch Alkyl bedeutet.

3. Verbindungen nach Anspruch 2, dadurch gekennzeichnet, dass die Ringe $A^1$, $A^2$ und $A^3$ 1,4-Phenylen oder trans-1,4-Cyclohexylen darstellen oder einer dieser Ringe auch einen 2,5-disubstituierten Pyrimidinring oder einen trans-2,5-disubstituierten m-Dioxanring darstellt; $R^2$ 1E-Alkenyl, 2Z-Alkenyl, 3E-Alkenyl, 4-Alkenyl, (2E-Alkenyl)oxy oder (3-Alkenyl)oxy darstellt; und $R^1$ 1E-Alkenyl, 2Z-Alkenyl, 3E-Alkenyl, 4-Alkenyl oder, sofern $R^2$ (2E-Alkenyl)oxy oder (3-Alkenyl)oxy darstellt, auch Alkyl bedeutet.

4. Verbindungen nach einem der Ansprüche 1 bis 3, dadurch gekennzeichnet, dass $R^1$ und $R^2$ geradkettige Reste mit höchstens 12 Kohlenstoffatomen, vorzugsweise höchstens 7 Kohlenstoffatomen bedeuten.

5. Verbindungen nach einem der Ansprüche 1 bis 4, dadurch gekennzeichnet, dass $R^2$ (2E-Alkenyl)oxy bedeutet oder einer der Reste $R^1$ und $R^2$ 1E-Alkenyl oder 3E-Alkenyl bedeutet.

6. Verbindungen nach einem der Ansprüche 1 bis 5, dadurch gekennzeichnet, dass $X^1$ eine einfache Kovalenzbindung, —COO—, —OOC—, $—CH_2CH_2—$ oder $p\text{-}C_6H_4—$ bezeichnet.

7. Verbindungen nach einem der Ansprüche 1 bis 5, dadurch gekennzeichnet, dass Ring $A^1$ für trans-1,4-Cyclohexylen steht, und $X^1$ eine einfache Kovalenzbindung, —COO—, $—CH_2CH_2$, $p\text{-}C_6H_4—$, $—CH_2CH_2\text{-}p\text{-}C_6H_4—$ oder $—CH_2CH_2\text{-}p\text{-}C_6H_4—CH_2CH_2—$ bezeichnet.

8. Verbindungen nach einem der Ansprüche 1 bis 7, dadurch gekennzeichnet, dass n die Zahl 0 und Ring $A^2$ 1,4-Phenylen, trans-1,4-Cyclohexylen oder einen 2,5-disubstituierten Pyrimidinring bedeutet, oder dass n Zahl 1 und Ring $A^2$ 1,4-Phenylen oder einen 2,5-disubstituierten Pyrimidinring bedeutet.

9. Verbindungen nach einem der Ansprüche 1 bis 8, dadurch gekennzeichnet, dass n für die Zahl 0 steht, Ring $A^1$ trans-1,4-Cyclohexylen darstellt, Ring $A^2$ 1,4-Phenylen darstellt und $X^1$ eine einfache Kovalenzbindung, —COO—, $—CH_2CH_2—$ oder $p\text{-}C_6H_4—$ bezeichnet.

10. Verbindungen nach einem der Ansprüche 1 bis 8, dadurch gekennzeichnet, dass Ringe $A^1$ einen 2,5-disubstituierten Pyrimidinring oder einen trans-2,5-disubstituierten m-Dioxanring darstellt, Ring $A^2$ 1,4-Phenylen oder trans-1,4-Cyclohexylen darstellt und $X^1$ eine einfache Kovalenzbindung bezeichnet.

11. Flüssigkristallines Gemisch mit mindestens 2 Komponenten, dadurch gekennzeichnet, dass mindestens eine Komponente eine Verbindung der allgemeinen Formel

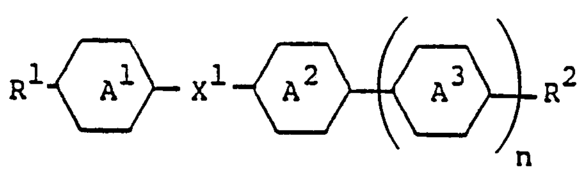

I

worin n für die Zah 0 oder 1 steht; die Ringe $A^1$, $A^2$ und $A^3$ 1,4-Phenylen, 2-Fluor-1,4-phenylen oder trans-1,4-Cyclohexylen darstellen oder einer dieser Ringe auch einen 2,5-disubstituierten Pyrimidinring oder einen trans-2,5-disubstituierten m-Dioxanring darstellt; $X^1$ eine einfache Kovalenzbindung, —COO—, —OOC—, $—CH_2CH_2—$, $p\text{-}C_6H_4$, $—CH_2CH_2\text{-}p\text{-}C_6H_4—$, $—CH_2CH_2\text{-}p\text{-}C_6H_4—CH_2CH_2—$ oder, sofern die Ringe $A^1$ und $A^2$ 1,4-Phenylen darstellen, auch eine Azoxygruppe —N=N(O)— oder —N(O)=N— bezeichnet; $R^2$ 1E-Alkenyl, 2Z-Alkenyl, 3E-Alkenyl, 4-Alkenyl oder Alkenyloxy darstellt, mit der Massgabe, dass das Sauerstoffatom in Alkenyloxy mit einem gesättigten Kohlenstoffatom verknüpft ist; und $R^1$ 1E-Alkenyl, 2Z-Alkenyl, 3E-Alkenyl, 4-Alkenyl oder, sofern $R^2$ Alkenyloxy darstellt, auch Alkyl bedeutet; mit der Massgabe, dass an einen Benzol- oder Pyrimidinring gebundene Reste $R^1$ und $R^2$ 3E-Alkenyl, 4-Alkenyl oder im Falle von $R^2$ auch Alkenyloxy oder im Falle von $R^1$ auch Alkyl bedeuten, ist.

12. Flüssigkristallines Gemisch nach Anspruch 11, dadurch gekennzeichnet, dass $R^2$ (2E-Alkenyl)oxy bedeutet oder einer der Reste $R^1$ und $R^2$ 1E-Alkenyl oder 3E-Alkenyl bedeutet.

13. Flüssigkristallines Gemisch nach Anspruch 11 oder 12, dadurch gekennzeichnet, dass n für die Zahl 0 steht, Ring $A^1$ trans-1,4-Cyclohexylen darstellt, Ring $A^2$ 1,4-Phenylen darstellt und $X^1$ eine einfache Kovalenzbindung, —COO—, $—CH_2CH_2—$ oder $p\text{-}C_6H_4—$ bezeichnet.

14. Verwendung der Verbindungen der in Anspruch 1 definierten Formel I für elektro-optische Zwecke.

15. Verbindungen der allgemeinen Formel

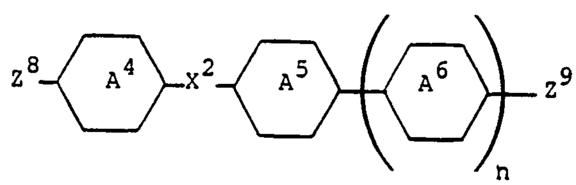

XXXIXa

worin n für die Zahl 0 oder 1 steht; die Ringe $A^4$, $A^5$ und $A^6$ 1,4-Phenylen, 2-Fluor-1,4-phenylen oder trans-1,4-Cyclohexylen darstellen; $X^2$ eine einfache Kovalenzbindung, $—CH_2CH_2—$, $p-C_6H_4—$, $—CH_2CH_2-p-C_6H_4—$ oder $—CH_2CH_2-p-C_6H_4—CH_2CH_2—$ bezeichnet; und einer der Reste $Z^8$ und $Z^9$ 3E-Alkenoyl oder 4-Alkenoyl und der andere 3E-Alkenyl, 4-Alkenyl, 3E-Alkenoyl, 4-Alkenoyl oder, sofern er nicht an einen Benzol- oder Pyrimidinring gebunden ist, auch 1E-Alkenyl oder 2Z-Alkenyl bedeutet.

**Revendications pour les Etats contractants: CH DE FR GB LI**

1. Composés de formule générale

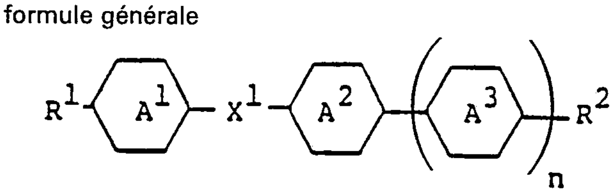

I

où n représente le nombre 0 ou 1; les noyaux $A^1$, $A^2$ et $A^3$ représentent le 1,4-phénylène, le 2-fluoro-1,4-phénylène ou le trans-1,4-cyclohexylène ou l'un de ces noyaux représente également un noyau pyrimidique disubstitué en 2,5 ou un noyau m-dioxane disubstitué en trans-2,5; $X^1$ représente une simple liaison covalente, $—COO—$, $—OOC—$, $—CH_2CH_2—$, $p-C_6H_4—$, $—CH_2CH_2-p-C_6H_4—$, $—CH_2CH_2-p-C_6H_4—CH_2CH_2—$ ou, dans le cas où les noyaux $A^1$ et $A^2$ représentent le 1,4-phénylène, également un groupe azoxy $—N{=}N(O)—$ ou $—N(O){=}N—$; $R^2$ représente un 1E-alcényle, un 2Z-alcényle, un 3E-alcényle, un 4-alcényle ou un alcényloxy, sous réserve que l'atome d'oxygène dans l'alcényloxy soit relié à un atome de carbone saturé; et $R^1$ représente un 1E-alcényle, un 2Z-alcényle, un 3E-alcényle, un 4-alcényle ou, dans le cas où $R^2$ représente un alcényloxy, également un alkyle; sous réserve que les restes $R^1$ et $R^2$ liés au noyau benzénique ou au noyau pyrimidique représentent un 3E-alcényle, un 4-alcényle ou dans le cas de $R^2$ également un alcényloxy ou dans le cas de $R^1$ également un alkyle; et sous cette autre réserve qu'en même temps $R^1$ ne représente pas un alkyle, les noyaux $A^1$ et $A^2$ le 1,4-phénylène ou le trans-1,4-cyclohexylène, $X^1$ une simple liaison covalente, n le nombre 0 et $R^2$ un alcényloxy.

2. Composés selon la revendication 1, caractérisés en ce que $R^2$ représente un 1E-alcényle, un 2Z-alcényle, un 3E-alcényle, un 4-alcényle, un (2E-alcényl)oxy, un (3-alcényl)oxy, un (4-alcényl)oxy ou un (5-alcényl)oxy et $R^1$ représente un 1E-alcényle, un 2Z-alcényle, un 3E-alcényle, un 4-alcényle ou, dans le cas où $R^2$ représente un groupe alcényloxy, également un alkyle.

3. Composés selon la revendication 2, caractérisés en ce que les noyaux $A^1$, $A^2$ et $A^3$ représentent le 1,4-phénylène ou le trans-1,4-cyclohexylène ou l'un de ces noyaux représente également un noyau pyrimidique disubstitué en 2,5 ou un noyau m-dioxane disubstitué en trans-2,5; $R^2$ représente un 1E-alcényle, un 2Z-alcényle, un 3E-alcényle, un 4-alcényle, un (2E-alcényl)oxy ou un (3-alcényl)oxy; et $R^1$ représente un 1E-alcényle, un 2Z-alcényle, un 3E-alcényle, un 4-alcényle ou, dans le cas où $R^2$ représente un (2E-alcényl)oxy ou un (3-alcényl)oxy, également un alkyle.

4. Composés selon l'une des revendications 1 à 3, caractérisés en ce que $R^1$ et $R^2$ représentent des restes à chaîne droite ayant au plus 12 atomes de carbone, de préférence au plus 7 atomes de carbone.

5. Composés selon l'une des revendications 1 à 4, caractérisés en ce que $R^2$ représente un (2E-alcényl)oxy ou l'un des restes $R^1$ et $R^2$ représente un 1E-alcényle ou un 3E-alcényle.

6. Composés selon l'une des revendications 1 à 5, caractérisés en ce que $X^1$ représente une simple liaison covalente, $—COO—$, $—OOC—$, $—CH_2CH_2—$ ou $p-C_6H_4—$.

7. Composés selon l'une des revendications 1 à 5, caractérises en ce que le noyau $A^1$ représente le trans-1,4-cyclohexylène et $X^1$ représente une simple liaison covalente, $—COO—$, $—CH_2CH_2—$, $p-C_6H_4—$, $—CH_2CH_2-p-C_6H_4—$ ou $—CH_2CH_2-p-C_6H_4—CH_2CH_2—$.

8. Composés selon l'une des revendications 1 à 7, caractérisés en ce que n représente le nombre 0 et le noyau $A^2$ représente le 1,4-phénylène, le trans-1,4-cyclohexylène ou un noyau pyrimidique disubstitué en 2,5 ou en ce que n représente le nombre 1 et le noyau $A^2$ représente le 1,4-phénylène ou un noyau pyrimidique disubstitué en 2,5.

9. Composés selon l'une des revendications 1 à 8, caractérisés en ce que n représente le nombre 0, le noyau $A^1$ représente le trans-1,4-cyclohexylène, le noyau $A^2$ représente le 1,4-phénylène et $X^1$ représente une simple liaison covalente, $—COO—$, $—CH_2CH_2—$ ou $p-C_6H_4—$.

10. Composés selon l'une des revendications 1 à 8, caractérisés en ce que le noyau $A^1$ représente un noyau pyrimidique disubstitué en 2,5 ou un noyau m-dioxane disubstitué en trans-2,5, le noyau $A^2$ représente le 1,4-phénylène ou le trans-1,4-cyclohexylène et $X^1$ représente une simple liaison covalente.

11. Mélange à cristaux liquides contenant au moins 2 composants, caractérisé en ce que au moins un composant est un composé de formule générale

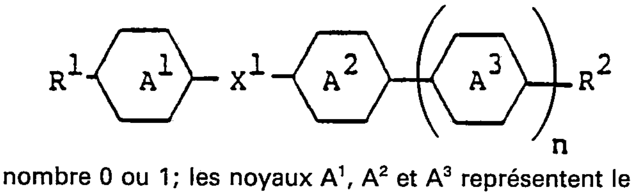

I

où n représente le nombre 0 ou 1; les noyaux $A^1$, $A^2$ et $A^3$ représentent le 1,4-phénylène, le 2-fluoro-1,4-

phénylène ou le trans-1,4-cyclohexylène ou l'un de ces noyaux représente également un noyau pyrimidique disubstitué en 2,5 ou un noyau m-dioxane disubstitué en trans-2,5; $X^1$ représente une simple liaison covalente, —COO—, —OOC—, $—CH_2CH_2—$, p-$C_6H_4$—, $—CH_2CH_2$-p-$C_6H_4$—, $—CH_2CH_2$-p-$C_6H_4—CH_2CH_2—$ ou, dans le cas où les noyaux $A^1$ et $A^2$ représentent le 1,4-phénylène, également un groupe azoxy —N=N(O)— ou —N(O)=N—; $R^2$ représente un 1E-alcényle, un 2Z-alcényle, un 3E-alcényle, un 4-alcényle ou un alcényloxy, sous réserve que l'atome d'oxygène dans l'alcényloxy soit relié à un atome de carbone saturé; et $R^1$ représente un 1E-alcényle, un 2Z-alcényle, un 3E-alcényle, un 4-alcényle ou, dans le cas où $R^2$ représente un alcényloxy, également un alkyle; sous réserve que les restes $R^1$ et $R^2$ liés au noyau benzénique ou au noyau pyrimidique représentent un 3E-alcényle, un 4-alcényle ou dans le cas de $R^2$ également un alcényloxy ou dans le cas de $R^1$ également un alkyle.

12. Mélange à cristaux liquides selon la revendication 11, caractérise en ce que $R^2$ représente un (2E-alcényl)oxy ou l'un des restes $R^1$ et $R^2$ représente un 1E-alcényle ou un 3E-alcényle.

13. Mélange à cristaux liquides selon la revendication 11 ou 12, caractérisé en ce que n représente le nombre 0, le noyau $A^1$ représente le trans-1,4-cyclohexylène, le noyau $A^2$ représente le 1,4-phénylène et $X^1$ représente une simple liaison covalente, —COO—, $—CH_2CH_2—$ ou p-$C_6H_4$—.

14. Utilisation des composés de formule I définie dans la revendication 11 à des fins électro-optiques.

15. Composés de formule générale

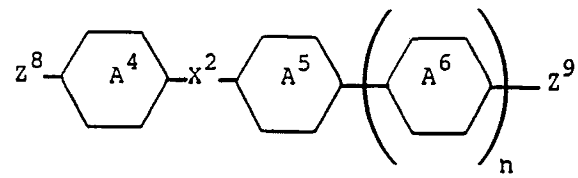

XXXIXa

où n représente le nombre 0 ou 1; les noyaux $A^4$, $A^5$ et $A^6$ représentent le 1,4-phénylène, le 2-fluoro-1,4-phénylène ou le trans-1,4-cyclohexylène; $X^2$ représente une simple liaison covalente, $—CH_2CH_2—$, p-$C_6H_4$—, $—CH_2CH_2$-p-$C_6H_4$— ou $—CH_2CH_2$-p-$C_6H_4—CH_2CH_2—$, et l'un des restes $Z^8$ et $Z^9$ représente un 3E-alcénoyle ou un 4-alcénoyle et l'autre reste représente un 3E-alcényle, un 4-alcényle, un 3E-alcénoyle, un 4-alcénoyle ou, dans le cas où il n'est pas lié à un noyau benzénique ou à un noyau pyrimidique, également un 1E-alcényle ou un 2Z-alcényle.

**Revendications pour les Etats contractants: IT SE**

1. Composés de formule générale

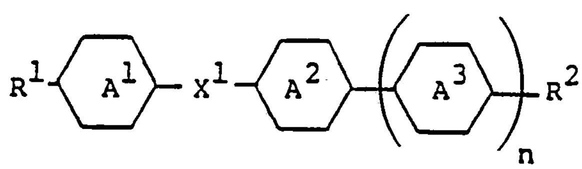

I

où n représente le nombre 0 ou 1; les noyaux $A^1$, $A^2$ et $A^3$ représentent le 1,4-phénylène, le 2-fluoro-1,4-phénylène ou le trans-1,4-cyclohexylène ou l'un de ces noyaux représente également un noyau pyrimidique disubstitué en 2,5 ou un noyau m-dioxane disubstitué en trans-2,5; $X^1$ représente une simple liaison covalente, —COO—, —OOC—, $—CH_2CH_2—$, p-$C_6H_4$—, $—CH_2CH_2$-p-$C_6H_4$, $—CH_2CH_2$-p-$C_6H_4—CH_2CH_2—$ ou, dans le cas où les noyaux $A^1$ et $A^2$ représentent le 1,4-phénylène, également un groupe azoxy —N=N(O)— ou —N(O)=N—; $R^2$ représente un 1E-alcényle, un 2Z-alcényle, un 3E-alcényle, un 4-alcényle ou un alcényloxy, sous réserve que l'atome d'oxygène dans l'alcényloxy soit relié à un atome de carbone saturé; et $R^1$ représente un 1E-alcényle, un 2Z-alcényle, un 3E-alcényle, un 4-alcényle ou, dans le cas où $R^2$ représente un alcényloxy, également un alkyle; sous réserve que les restes $R^1$ et $R^2$ liés au noyau benzénique ou au noyau pyrimidique représentent un 3E-alcényle, un 4-alcényle ou dans le cas de $R^2$ également un alcényloxy ou dans le cas de $R^1$ également un alkyle.

2. Composés selon la revendication 1, caractérisés en ce que $R^2$ représente un 1E-alcényle, un 2Z-alcényle, un 3E-alcényle, un 4-alcényle, un (2E-alcényl)oxy, un (3-alcényl)oxy, un (4-alcényl)oxy ou un (5-alcényl)oxy et $R^1$ représente un 1E-alcényle, un 2Z-alcényle, un 3E-alcényle, un 4-alcényle ou, dans le cas où $R^2$ représente un groupe alcényloxy, également un alkyle.

3. Composés selon la revendication 2, caractérisés en ce que les noyaux $A^1$, $A^2$ et $A^3$ représentent le 1,4-phénylène ou le trans-1,4-cyclohexylène ou l'un de ces noyaux représente également un noyau pyrimidique disubstitué en 2,5 ou un noyau m-dioxane disubstitué en trans-2,5; $R^2$ représente un 1E-alcényle, un 2Z-alcényle, un 3E-alcényle, un 4-alcényle, un (2E-alcényl)oxy ou un (3-alcényl)oxy; et $R^1$ représente un 1E-alcényle, un 2Z-alcényle, un 3E-alcényle, un 4-alcényle ou, dans le cas où $R^2$ représente un (2E-alcényl)oxy ou un (3-alcényl)oxy, également un alkyle.

4. Composés selon l'une des revendications 1 à 3, caractérisés en ce que $R^1$ et $R^2$ représentent des restes à chaîne droite ayant au plus 12 atomes de carbone, de préférence au plus 7 atomes de carbone.

5. Composés selon l'une des revendications 1 à 4, caractérisés en ce que $R^2$ représente un (2E-alcényl)oxy ou l'un des restes $R^1$ et $R^2$ représente un 1E-alcényle ou un 3E-alcényle.

6. Composés selon l'une des revendications 1 à 5, caractérisés en ce que $X^1$ représente une simple liaison covalente, —COO—, —OOC—, $—CH_2CH_2—$ ou p-$C_6H_4$—.

7. Composés selon l'une des revendications 1 à 5, caractérises en ce que le noyau $A^1$ représente le trans-1,4-cyclohexylène et $X^1$ représente une simple liaison covalente, —COO—, $CH_2CH_2$—, p-$C_6H_4$—, $—CH_2CH_2$-p-$C_6H_4$— ou $—CH_2CH_2$-p-$C_6H_4$—$CH_2CH_2$—.

8. Composés selon l'une des revendications 1 à 7, caractérisés en ce que n représente le nombre 0 et le noyau $A^2$ représente le 1,4-phénylène, le trans-1,4-cyclohexylène ou un noyau pyrimidique disubstitué en 2,5 ou en ce que n représente le nombre 1 et le noyau $A^2$ représente le 1,4-phénylène ou un noyau pyrimidique disubstitué en 2,5.

9. Composés selon l'une des revendications 1 à 8, caractérisés en ce que n représente le nombre 0, le noyau $A^1$ représente le trans-1,4-cyclohexylène, le noyau $A^2$ représente le 1,4-phénylène et $X^1$ represente une simple liaison covalente, —COO—, $—CH_2CH_2—$ ou p-$C_6H_4$—.

10. Composés selon l'une des revendications 1 à 8, caractérisés en ce que le noyau $A^1$ représente un noyau pyrimidique disubstitué en 2,5 ou un noyau m-dioxane disubstitué en trans-2,5, le noyau $A^2$ représente le 1,4-phénylène ou le trans-1,4-cyclohexylène et $X^1$ représente une simple liaison covalente.

11. Mélange à cristaux liquides contenant au moins 2 composants, caractérisé en ce que au moins un composant est un composé de formule générale

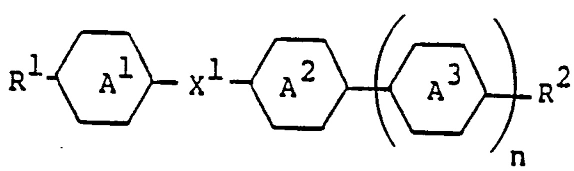

I

où n représente le nombre 0 ou 1; les noyaux $A^1$, $A^2$ et $A^3$ représentent le 1,4-phénylène, le 2-fluoro-1,4-phénylène ou le trans-1,4-cyclohexylène ou l'un de ces noyaux représente également un noyau pyrimidique disubstitué en 2,5 ou un noyau m-dioxane disubstitué en trans-2,5; $X^1$ représente une simple liaison covalente, —COO—, —OOC—, $—CH_2CH_2—$, p-$C_6H_4$—, $—CH_2CH_2$-p-$C_6H_4$—, $—CH_2CH_2$-p-$C_6H_4$—$CH_2CH_2$— ou, dans le cas où les noyaux $A^1$ et $A^2$ représentent le 1,4-phénylène, également un groupe azoxy —N=N(O)— ou —N(O)=N—; $R^2$ représente un 1E-alcényle, un 2Z-alcényle, un 3E-alcényle, un 4-alcényle ou un alcényloxy, sous réserve que l'atome d'oxygène dans l'alcényloxy soit relié à un atome de carbone saturé; et $R^1$ représente un 1E-alcényle, un 2Z-alcényle, un 3E-alcényle, un 4-alcényle ou, dans le cas où $R^2$ représente un alcényloxy, également un alkyle; sous réserve que les restes $R^1$ et $R^2$ liés au noyau benzénique ou au noyau pyrimidique représentent un 3E-alcényle, un 4-alcényle ou dans le cas de $R^2$ également un alcényloxy ou dans le cas de $R^1$ également un alkyle.

12. Mélange à cristaux liquides selon la revendication 11, caractérisé en ce que $R^2$ représente un (2E-alcényl)oxy ou l'un des restes $R^1$ et $R^2$ représente un 1E-alcényle ou un 3E-alcényle.

13. Mélange à cristaux liquides selon la revendication 11 ou 12, caractérisé en ce que n représente le nombre 0, le noyau $A^1$ représente le trans-1,4-cyclohexylène, le noyau $A^2$ représente le 1,4-phénylène et $X^1$ représente une simple liaison covalente, —COO—, $—CH_2CH_2—$ ou p-$C_6H_4$—.

14. Utilisation des composés de formule I définie dans la revendication 11 à des fins électro-optiques.

15. Composés de formule générale

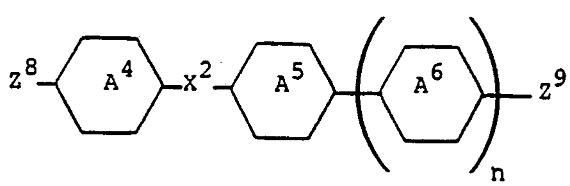

XXXIXa

où n représente le nombre 0 ou 1; les noyaux $A^4$, $A^5$ et $A^6$ représentent le 1,4-phénylène, le 2-fluoro-1,4-phénylène ou le trans-1,4-cyclohexylène; $X^2$ représente une simple liaison covalente, $—CH_2CH_2—$, p-$C_6H_4$—, $—CH_2CH_2$-p-$C_6H_4$— ou $—CH_2CH_2$-p-$C_6H_4$—$CH_2CH_2$—, et l'un des restes $Z^8$ et $Z^9$ représente un 3E-alcénoyle ou un 4-alcénoyle et l'autre reste représente un 3E-alcényle, un 4-alcényle, un 3E-alcénoyle, un 4-alcénoyle ou, dans le cas où il n'est pas lié à un noyau benzénique ou à un noyau pyrimidique, également un 1E-alcényle ou un 2Z-alcényle.

**Claims for the Contracting States: CH DE FR GB LI**

1. Compounds of the general formula

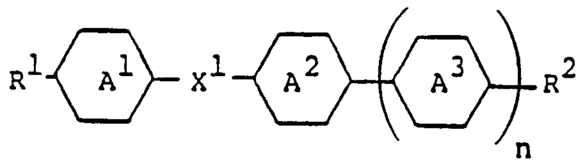

I

wherein n stands for the number 0 or 1; the rings $A^1$, $A^2$ and $A^3$ represent 1,4-phenylene, 2-fluoro-1,4-phenylene or trans-1,4-cyclohexylene or one of these rings also represents a 2,5-disubstituted pyrimidine ring or a trans-2,5-disubstituted m-dioxane ring; $X^1$ represents a single covalent bond, —COO—, —OOC—, —$CH_2CH_2$—, p-$C_6H_4$—, —$CH_2CH_2$-p-$C_6H_4$—, —$CH_2CH_2$-p-$C_6H_4$—$CH_2$—$CH_2$— or, insofar as the rings $A^1$ and $A^2$ represent 1,4-phenylene, also an azoxy group —N=N(O)— or —N(O)=N—; $R^2$ represents 1E-alkenyl, 2Z-alkenyl, 3E-alkenyl, 4-alkenyl or alkenyloxy, with the proviso that the oxygen atom in alkenyloxy is linked with a saturated carbon atom; and $R^1$ signifies 1E-alkenyl, 2Z-alkenyl, 3E-alkenyl, 4-alkenyl or, insofar as $R^2$ represents alkenyloxy, also alkyl; with the proviso that residues $R^1$ and $R^2$ attached to a benzene or pyrimidine ring signify 3E-alkenyl, 4-alkenyl or in the case of $R^2$ also alkenyloxy or in the case of $R^1$ also alkyl; and with the further proviso that simultaneously $R^1$ does not signify alkyl, the rings $A^1$ and $A^2$ do not each signify 1,4-phenylene or trans-1,4-cyclohexylene, $X^1$ does not signify a single covalent bond, n does not signify the number 0 and $R^2$ does not signify alkenyloxy.

2. Compounds according to claim 1, characterized in that $R^2$ represents 1E-alkenyl, 2Z-alkenyl, 3E-alkenyl, 4-alkenyl (2E-alkenyl)oxy, (3-alkenyl)oxy, (4-alkenyl)oxy or (5-alkenyl)oxy and $R^1$ signifies 1E-alkenyl, 2Z-alkenyl, 3E-alkenyl, 4-alkenyl or, insofar as $R^2$ represents an alkenyloxy group, also alkyl.

3. Compounds according to claim 2, characterized in that the rings $A^1$, $A^2$ and $A^3$ represent 1,4-phenylene or trans-1,4-cyclohexylene or one of these rings also represents a 2,5-disubstituted pyrimidine ring or a trans-2,5-disubstituted m-dioxane ring; $R^2$ represents 1E-alkenyl, 2Z-alkenyl, 3E-alkenyl, 4-alkenyl, (2E-alkenyl)oxy or (3-alkenyl)oxy; $R^1$ signifies 1E-alkenyl, 2Z-alkenyl, 3E-alkenyl, 4-alkenyl or, insofar as $R^2$ represents (2E-alkenyl)oxy or (3-alkenyl)oxy, also alkyl.

4. Compounds according to any one of claims 1 to 3, characterized in that $R^1$ and $R^2$ signify straight-chain residues with a maximum of 12 carbon atoms, preferably a maximum of 7 carbon atoms.

5. Compounds according to any one of claims 1 to 4, characterized in that $R^2$ signifies (2E-alkenyl)oxy or one of the residues $R^1$ and $R^2$ signifies 1E-alkenyl or 3E-alkenyl.

6. Compounds according to any one of claims 1 to 5, characterized in that $X^1$ denotes a single covalent bond, —COO—, —OOC—, —$CH_2CH_2$— or p-$C_6H_4$—.

7. Compounds according to any one of claims 1 to 5, characterized in that ring A stands for trans-1,4-cyclohexylene and $X^1$ denotes a single covalent bond, —COO—, —$CH_2CH_2$—, p-$C_6H_4$—, —$CH_2CH_2$-p-$C_6H_4$— or —$CH_2CH_2$-p-$C_6H$—$CH_2CH_2$—.

8. Compounds according to any one of claims 1 to 7, characterized in that n signifies the number 0 and ring $A^2$ signifies 1,4-phenylene, trans-1,4-cyclohexylene or a 2,5-disubstituted pyrimidine ring or in that n signifies the number 1 and ring $A^2$ signifies 1,4-phenylene or a 2,5-disubstituted pyrimidine ring.

9. Compounds according to any one of claims 1 to 8, characterized in that n stands for the number 0, ring $A^1$ represents trans-1,4-cyclohexylene, ring $A^2$ represents 1,4-phenylene and $X^1$ denotes a single covalent bond, —COO—, —$CH_2CH_2$— or p-$C_6H_4$.

10. Compounds according to any one of claims 1 to 8, characterized in that ring $A^1$ represents a 2,5-disubstituted pyrimidine ring or a trans-2,5-disubstituted m-dioxane ring, ring $A^2$ represents 1,4-phenylene or trans-1,4-cyclohexylene and $X^1$ denotes a single covalent bond.

11. A liquid crystalline mixture with at least 2 components, characterized in that at least one component is a compound of the general formula

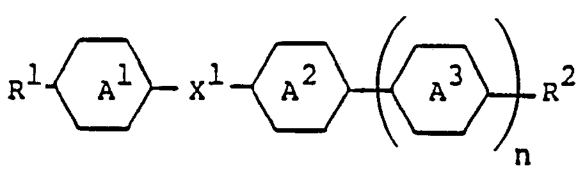

I

wherein n stands for the number 0 or 1; the rings $A^1$, $A^2$ and $A^3$ represent 1,4-phenylene, 2-fluoro-1,4-phenylene or trans-1,4-cyclohexylene or one of these rings also represents a 2,5-disubstituted pyrimidine ring or a trans-2,5-disubstituted m-dioxane ring; $X^1$ represents a single covalent bond, —COO—, —OOC—, —$CH_2CH_2$—, p-$C_6H_4$—, —$CH_2CH_2$-p-$C_6H_4$—, —$CH_2CH_2$-p-$C_6H_4$—$CH_2$—$CH_2$— or, insofar as the rings $A^1$ and $A^2$ represent 1,4-phenylene, also an azoxy group —N=N(O)— or —N(O)=N—; $R^2$ represents 1E-alkenyl, 2Z-alkenyl, 3E-alkenyl, 4-alkenyl or alkenyloxy, with the proviso that the oxygen atom in alkenyloxy is linked with a saturated carbon atom; and $R^1$ signifies 1E-alkenyl, 2Z-alkenyl, 3E-alkenyl, 4-alkenyl or, insofar as $R^2$

represents alkenyloxy, also alkyl; with the proviso that residues $R^1$ and $R^2$ attached to a benzene or pyrimidine ring signify 3E-alkenyl, 4-alkenyl or in the case of $R^2$ also alkenyloxy or in the case of $R^1$ also alkyl.

12. A liquid crystalline mixture according to claim 11, characterized in that $R^2$ signifies (2E-alkenyl)oxy or one of the residues $R^1$ and $R^2$ signifies 1E-alkenyl or 3E-alkenyl.

13. A liquid crystalline mixture according to claim 11 or 12, characterized in that n stands for the number 0, ring $A^1$ represents trans-1,4-cyclohexylene, ring $A^2$ represents 1,4-phenylene and $X^1$ denotes a single covalent bond, —COO—, $—CH_2CH_2—$ or $p-C_6H_4—$.

14. The use of the compounds of formula I defined in claim 11 for electro-optical purposes.

15. Compounds of the general formula

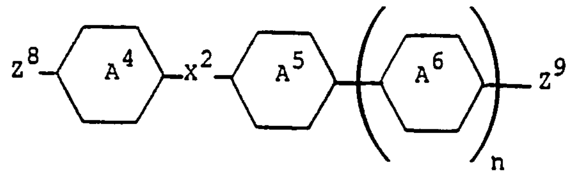

XXXIXa

wherein n stands for the number 0 or 1; the rings $A^4$, $A^5$ and $A^6$ represent 1,4-phenylene, 2-fluoro-1,4-phenylene or trans-1,4-cyclohexylene; $X^2$ denotes a single covalent bond, $—CH_2CH_2—$, $p-C_6H_4—$, $—CH_2CH_2-p-C_6H_4—$ or $—CH_2CH_2-p-C_6H_4—CH_2CH_2—$; and one of the residues $Z^8$ and $Z^9$ signifies 3E-alkenoyl or 4-alkenoyl and the other signifies 3E-alkenyl, 4-alkenyl, 3E-alkenoyl, 4-alkenoyl or, insofar as it is not attached to a benzene or pyrimidine ring, also 1E-alkenyl or 2Z-alkenyl.

**Claims for the Contracting States: IT SE**

1. Compounds of the general formula

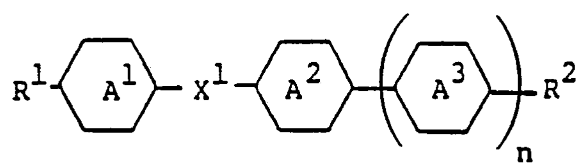

I

wherein n stands for the number 0 or 1; the rings $A^1$, $A^2$ and $A^3$ represent 1,4-phenylene, 2-fluoro-1,4-phenylene or trans-1,4-cyclohexylene or one of these rings also represents a 2,5-disubstituted pyrimidine ring or a trans-2,5-disubstituted m-dioxane ring; $X^1$ represents a single covalent bond, —COO—, —OOC—, $—CH_2CH_2—$, $p-C_6H_4—$, $—CH_2CH_2-p-C_6H_4—$, $—CH_2CH_2-p-C_6H_4—CH_2—CH_2—$ or, insofar as the rings $A^1$ and $A^2$ represent 1,4-phenylene, also an azoxy group —N=N(O)— or —N(O)=N—; $R^2$ represents 1E-alkenyl, 2Z-alkenyl, 3E-alkenyl, 4-alkenyl or alkenyloxy, with the proviso that the oxygen atom in alkenyloxy is linked with a saturated carbon atom; and $R^1$ signifies 1E-alkenyl, 2Z-alkenyl, 3E-alkenyl, 4-alkenyl or, insofar as $R^2$ represents alkenyloxy, also alkyl; with the proviso that residues $R^1$ and $R^2$ attached to a benzene or pyrimidine ring signify 3E-alkenyl, 4-alkenyl or in the case of $R^2$ also alkenyloxy or in the case of $R^1$ also alkyl.

2. Compounds according to claim 1, characterized in that $R^2$ represents 1E-alkenyl, 2Z-alkenyl, 3E-alkenyl, 4-alkenyl (2E-alkenyl)oxy, (3-alkenyl)oxy, (4-alkenyl)oxy or (5-alkenyl)oxy and $R^1$ signifies 1E-alkenyl, 2Z-alkenyl, 3E-alkenyl, 4-alkenyl or, insofar as $R^2$ represents an alkenyloxy group, also alkyl.

3. Compounds according to claim 2, characterized in that the rings $A^1$, $A^2$ and $A^3$ represent 1,4-phenylene or trans-1,4-cyclohexylene or one of these rings also represents a 2,5-disubstituted pyrimidine ring or a trans-2,5-disubstituted m-dioxane ring; $R^2$ represents 1E-alkenyl, 2Z-alkenyl, 3E-alkenyl, 4-alkenyl, (2E-alkenyl)oxy or (3-alkenyl)oxy; $R^1$ signifies 1E-alkenyl, 2Z-alkenyl, 3E-alkenyl, 4-alkenyl or, insofar as $R^2$ represents (2E-alkenyl)oxy or (3-alkenyl)oxy, also alkyl.

4. Compounds according to any one of claims 1 to 3, characterized in that $R^1$ and $R^2$ signify straight-chain residues with a maximum of 12 carbon atoms, preferably a maximum of 7 carbon atoms.

5. Compounds according to any one of claims 1 to 4, characterized in that $R^2$ signifies (2E-alkenyl)oxy or one of the residues $R^1$ and $R^2$ signifies 1E-alkenyl or 3E-alkenyl.

6. Compounds according to any one of claims 1 to 5, characterized in that $X^1$ denotes a single covalent bond, —COO—, —OOC—, $—CH_2CH_2—$ or $p-C_6H_4—$.

7. Compounds according to any one of claims 1 to 5, characterized in that ring A stands for trans-1,4-cyclohexylene and $X^1$ denotes a single covalent bond, —COO—, $—CH_2CH_2—$, $p-C_6H_4—$, $—CH_2CH_2-p-C_6H_4—$ or $—CH_2CH_2-p-C_6H_4—CH_2CH_2—$.

8. Compounds according to any one of claims 1 to 7, characterized in that n signifies the number 0 and ring $A^2$ signifies 1,4-phenylene, trans-1,4-cyclohexylene or a 2,5-disubstituted pyrimidine ring or in that n signifies the number 1 and ring $A^2$ signifies 1,4-phenylene or a 2,5-disubstituted pyrimidine ring.

9. Compounds according to any one of claims 1 to 8, characterized in that n stands for the number 0, ring $A^1$ represents trans-1,4-cyclohexylene, ring $A^2$ represents 1,4-phenylene and $X^1$ denotes a single covalent bond, —COO—, —$CH_2CH_2$— or p-$C_6H_4$.

10. Compounds according to any one of claims 1 to 8, characterized in that ring $A^1$ represents a 2,5-disubstituted pyrimidine ring or a trans-2,5-disubstituted m-dioxane ring, ring $A^2$ represents 1,4-phenylene or trans-1,4-cyclohexylene and $X^1$ denotes a single covalent bond.

11. A liquid crystalline mixture with at least 2 components, characterized in that at least one component is a compound of the general formula

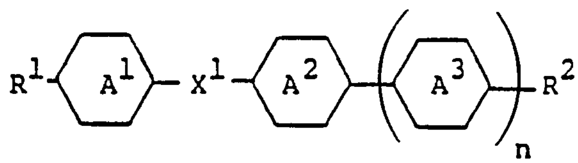

I

wherein n stands for the number 0 or 1; the rings $A^1$, $A^2$ and $A^3$ represent 1,4-phenylene, 2-fluoro-1,4-phenylene or trans-1,4-cyclohexylene or one of these rings also represents a 2,5-disubstituted pyrimidine ring or a trans-2,5-disubstituted m-dioxane ring; $X^1$ represents a single covalent bond, —COO—, —OOC—, —$CH_2CH_2$—, p-$C_6H_4$—, —$CH_2CH_2$-p-$C_6H_4$—, —$CH_2CH_2$-p-$C_6H_4$—$CH_2$—$CH_2$— or, insofar as the rings $A^1$ and $A^2$ represent 1,4-phenylene, also an azoxy group —N=N(O)— or —N(O)=N—; $R^2$ represents 1E-alkenyl, 2Z-alkenyl, 3E-alkenyl, 4-alkenyl or alkenyloxy, with the proviso that the oxygen atom in alkenyloxy is linked with a saturated carbon atom; and $R^1$ signifies 1E-alkenyl, 2Z-alkenyl, 3E-alkenyl, 4-alkenyl or, insofar as $R^2$ represents alkenyloxy, also alkyl; with the proviso that residues $R^1$ and $R^2$ attached to a benzene or pyrimidine ring signify 3E-alkenyl, 4-alkenyl or in the case of $R^2$ also alkenyloxy or in the case of $R^1$ also alkyl.

12. A liquid crystalline mixture according to claim 11, characterized in that $R^2$ signifies (2E-alkenyl)oxy or one of the residues $R^1$ and $R^2$ signifies 1E-alkenyl or 3E-alkenyl.

13. A liquid crystalline mixture according to claim 11 or 12, characterized in that n stands for the number 0, ring $A^1$ represents trans-1,4-cyclohexylene, ring $A^2$ represents 1,4-phenylene and $X^1$ denotes a single covalent bond, —COO—, —$CH_2CH_2$— or p-$C_6H_4$—.

14. The use of the compounds of formula I defined in claim 11 for electro-optical purposes.

15. Compounds of the general formula

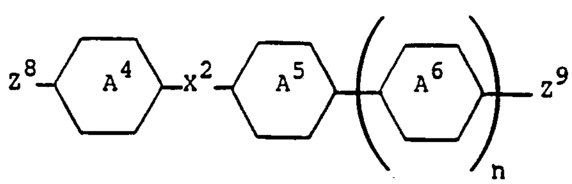

XXXIXa

wherein n stands for the number 0 or 1; the rings $A^4$, $A^5$ and $A^6$ represent 1,4-phenylene, 2-fluoro-1,4-phenylene or trans-1,4-cyclohexylene; $X^2$ denotes a single covalent bond, —$CH_2CH_2$—, p-$C_6H_4$—, —$CH_2CH_2$-p-$C_6H_4$— or —$CH_2CH_2$-p-$C_6H_4$—$CH_2CH_2$—; and one of the residues $Z^8$ and $Z^9$ signifies 3E-alkenoyl or 4-alkenoyl and the other signifies 3E-alkenyl, 4-alkenyl, 3E-alkenoyl, 4-alkenoyl or, insofar as it is not attached to a benzene or pyrimidine ring, also 1E-alkenyl or 2Z-alkenyl.